# EUROPEAN PATENT APPLICATION

(11) **EP 3 406 719 A1**
(43) Date of publication of application: **28.11.2018**
(21) Application number: 17172826.4
(22) Date of filing: 24.05.2017
(51) Int. Cl.: C12N 15/113, A61K 31/7088

(54) **CALCINEURIN B HOMOLOGOUS PROTEIN 1 INHIBITORS AND THERAPEUTIC AND NON-THERAPEUTIC USES THEREOF**

(71) Applicant: Universität zu Köln, 50923 Köln (DE)
(72) Inventor: Wirth, Brunhilde, 53225 Bonn (DE); Janzen, Eva, 50931 Köln (DE); Mendoza-Ferreira, Natalia, 50667 Köln (DE); Hosseinibarkooie, Seyyedmohsen, 50737 Köln (DE)
(74) Representative: Lahrtz, Fritz

(57) **Abstract**

The invention relates to an inhibitor of Calcineurin B Homologous Protein 1 (CHP1) for use in a method for the treatment or prevention of a patient suffering from or being at risk of developing a disorder associated with a pathological calcium homeostasis, impaired vesicular trafficking and/or disturbed neuronal and/or neuromuscular synaptic function.

## Description

The present invention relates to an inhibitor of Calcineurin B Homologous Protein 1 (CHP1) for use in a method for the treatment or prevention of a patient suffering from or being at risk of developing a disorder associated with a pathological calcium homeostasis, impaired vesicular trafficking and/or disturbed neuronal and/or neuromuscular synaptic function.

Today, numerous disorders associated with a pathological calcium homeostasis impaired vesicular trafficking and/or disturbed neuronal and/or neuromuscular synaptic function are known of which many, in particular those that are neuronal disorders, are up to day not sufficiently curable.

Exemplarily, so far, no adequate and satisfactory therapy for spinal muscular atrophies (SMA), amyotrophic lateral sclerosis (ALS), hereditary motor neuropathies (HMN), Parkinson's disease, Frontotemporal Dementia, Alzheimer's disease, Morbus Huntington, and polyglutamin expansion disorders has been found. Although these disorders have a widespread incidence throughout the population, today, only few symptoms may be cured or even merely retarded, but there is still a lack of a comprehensive treatment and prevention of such disorders.

Autosomal recessive spinal muscular atrophy (SMA), for instance, has an incidence of approximately 1:6000 and is, therefore, after cystic fibrosis (mucoviscidosis), the second most often occurring autosomal recessive disorder in human. Approximately one in 35 individuals bears a genetic disposition for developing SMA, which is frequently caused by the loss of the *survival motor neuron 1* (*SMN1*) gene which typically leads to a malfunction of alpha-motoneurons in the spinal cord which may result in severe atrophy of the voluntary musculature and, in roughly half of cases, causes death in early childhood.

Autosomal recessive spinal muscular atrophy (SMA) is caused by homozygous mutations or deletions of the *survival motor neuron 1* (*SMN1*) gene (Lefebvre et al., 1995) resulting in reduced SMN protein levels. Besides *SMN2,* whose copy number inversely correlates with SMA severity (Feldkotter et al., 2002), Plastin 3 (PLS3) and Neurocalcin delta (NCALD) have been identified as human genetic modifiers of SMA (Oprea et al., 2008; Riessland et al., 2017). Overexpression of the actin-binding and -bundling protein PLS3, as well as downregulation/inhibition of the neuronal calcium sensor protein NCALD have been found to protect individuals carrying the same homozygous deletion of *SMN1* and identical numbers *of SMN2* as their affected siblings. The beneficial/protective effect of PLS3 overexpression and NCALD inhibition on the SMA phenotype has been confirmed in various in *vitro and in vivo* models, including mice, zebrafish, flies and worms (Dimitriadi et al., 2010; Hosseinibarkooie et al., 2016; Kaifer et al., 2017; Oprea et al., 2008; Riessland et al., 2017). In more detail, PLS3 overexpression or NCALD suppression have been shown to rescue axon length, improve neuromuscular junctions (NMJ), increase motor neuron size, increase levels of acetylcholine receptors and improve neurotransmission in mice (Ackermann et al., 2013); Hosseinibarkooie et al., 2016; Riessland et al., 2017). Most importantly, homozygous overexpression of PLS3 improved the survival in a low dose SMN-ASO (Spinraza or Nusinersen) induced intermediate SMA mouse model from 26 days to >250 days in 60% of animals and to >400 day in 30% of the animals (Hosseinibarkooie et al., 2016). Both human modifier PLS3 and NCALD unravelled impaired endocytosis as a main cellular pathway disturbed in SMA, which could be rescued by both modifiers.

In the view of the above, there are still unmet needs for either a therapeutic agent suitable for treating a patient suffering from a disorder associated with a pathological calcium homeostasis as well as for preventing a patient being at risk thereof.

Surprisingly, it has been found that inhibitors of Calcineurin B Homologous Protein 1 (CHP1) are suitable for treating and preventing such disorders in a patient *in vivo* by restoring impaired endocytosis. As shown in the examples, CHP1 is strongly interacting with PLS3 and is a novel SMA modifier, which restores processes impaired in SMA including endocytosis and axonal outgrowth effects.

Calcineurin B Homologous Protein 1 is also named Calcineurin-like EF-hand protein 1 and is abbreviated as CHP1.

In a first aspect, the present invention relates to an inhibitor of CHP1 for use in a method for the treatment or prevention of a patient suffering from or being at risk of developing a disorder associated with a pathological calcium homeostasis, impaired vesicular trafficking and/or disturbed neuronal and/or neuromuscular function.

As used in the context of the present invention, the term "inhibitor" may be understood in the broadest sense as any molecular structure that is, in an adequate environment, able to reduce the expression and/or the functionality of CHP1. As used herein, the terms "inhibitor of Calcineurin B Homologous Protein 1" and "Calcineurin B Homologous Protein 1 inhibitor" and "inhibitor of CHP1" and "CHP1 inhibitor" may be understood interchangeably. As the CHP1 is present in cells, an adequate targeting environment may preferably be an intracellular environment, most preferably a cytoplasmic, membrane, and/or karyoplasmic environment. The molecular structure serving as an inhibitor may be a single molecule or may be a complex of molecules. Preferably, it is a single molecule or a dimer of two complementary oligomers. An oligomer in the sense of the present invention may be an oligonucleotide or a polypeptide, most preferably an oligonucleotide. The molecular structure may have a molecular weight of more than 500 Da or may be a small molecule of less than 500 Da. Alternatively, the molecular structure may also have a molecular weight of more than 500 Da, more than 1000 Da or even more than 2000 Da.

Most preferably, the inhibitor is an oligonucleotide. As used in the context of the present invention, the term oligonucleotide may be understood in the broadest sense as any nucleotide strand mainly composed of a consecutive strand of deoxyribonucleic acid (DNA) nucleotides and/or ribonucleic acid (RNA) nucleotides, wherein the strand is at least four nucleotide moieties (bases) in length and typically not longer than 100 bases. Preferably, the oligonucleotide is at least ten bases, more preferably at least 15, even more preferably at least 18, in particular of from approximately 18 to approximately 24 bases in length.

Alternatively, the inhibitor is a polypeptide of at least ten amino acids moieties, preferably of at least 15 amino acids moieties, more preferably of at least 20 amino acids moieties, even more preferably of at least 50 amino acids moieties, even, more preferably of at least 100 amino acids moieties in length.

Alternatively, the inhibitor, in particular when it is a polypeptide or a small molecule inhibitor, may bind to the CHP1 polypeptide and may block its function by non-covalently or covalently binding that may be competitively, non-competitively or uncompetitively binding to a binding site *(e.g.,* the calcium binding site, a second messenger binding site *(e.g.,* the cyclic guanidine monophosphate (cGMP) binding site), and/or a cofactor binding site) of CHP1 or by amending the CHP1 polypeptide's three-dimensional structure sterically. A small molecule inhibitor may be exemplarily FTY720 or a similar molecule.

Alternatively, the inhibitor may also be an antibody or an antibody fragment *(e.g.,* a Fab fragment, a single chain antibody binding domain, a diabody or a triabody) directed against CHP1. This may bind to the CHP1 non-covalently at a binding site (binding pocket, *e.g.,* the calcium binding site or a cofactor binding site) of CHP1 or by amending the CHP1 polypeptide's three-dimensional structure sterically.

Alternatively, the inhibitor, may bind non-covalently or covalently to genomic DNA encoding for CHP1, to regulatory CHP1 *elements in cis (e.g.* promotor, enhancer, silencer), to pre-mRNA comprising CHP1-encoding regions, to mature mRNA encoding for CHP1, to one or more transcription factor(s) triggering CHP1 expression and/or to one or more translation factor(s) triggering CHP1 expression and thereby reduces CHP1 expression.

Optionally, in case the inhibitor may also be genetic material encoding for an oligonucleotide in the sense of the present invention or for a polypeptide inhibiting CHP1 expression or the biological activity of the CHP1 polypeptide. Then, the genetic material may optionally encode for an antisense or silencing oligonucleotide. As used in the context of the present invention, the term "genetic material" may be understood in the broadest sense as any carrier suitable for conveying genetic information into cells known in the art such as, *e.g.,* DNA, RNA or any analogue thereof. Genetic material may be or include a linear consecutive strand mainly composed of nucleotide moieties and/or analogues thereof. Alternatively, it may also be a circular molecule *(e.g.,* a plasmid). Optionally, such genetic material may be embedded into any vector known in the art such as, e.g., a plasmid, an episome, a virus, a viroid or a bacterial cell. In result, the patient and the cells, respectively, administered with the inhibitor then may produce the compounds inhibiting CHP1 expression and/or functionality themselves.

"Calcineurin B Homologous Protein 1" and its abbreviation "CHP1", respectively, as used herein is a neuronal calcium-binding polypeptide that belongs to the neuronal calcium sensor (NCS) family of proteins, containing Ca²⁺ binding EF-Hand motifs as well as comprising a Ca²⁺-myristoyl switch (Di Sole et al., 2012). CHP1 is a ubiquitously expressed protein with particularly high expression in neuronal tissues including brain and spinal cord. Most importantly, CHP1 is an endogenous calcineurin (CaN) inhibitor (Lin et al., 1999). CaN is a major regulator of essential proteins required for synaptic transmission and neuronal endocytosis (Baumgartel and Mansuy, 2012). Moreover, CHP1 is involved in various cellular processes including constitutive membrane traffic (Barroso et al., 1996) (Di Sole et al., 2012), microtubule-membrane interaction (Timm et al., 1999), microtubule bundle formation (Andrade et al., 2004), regulation of gene transcription (Jimenez-Vidal et al., 2010) and cell death (Kuwahara et al., 2003).

As used herein, the CHP1 may be understood in the broadest sense as CHP1 originating from any species, preferably mammalian CHP1, most preferably human CHP1. The CHP1 synonyms: p24, p22, CHP, Sid470p, SLC9A1BP may be understood with the name CHP1 interchangeably.

As used herein, human CHP1 preferably comprises a polypeptide having an amino acid sequence of at least 98 % homology to SEQ ID NO: 19:

MGSRASTLLRDEELEEIKKETGFSHSQITRLYSRFTSLDKGENGTLSREDFQRIPELA
INPLGDRIINAFFPEGEDQVNFRGFMRTLAHFRPIEDNEKSKDVNGPEPLNSRSNKL
HFAFRLYDLDKDEKISRDELLQVLRMMVGVNISDEQLGSIADRTIQEADQDGDSAI
SFTEFVKVLEKVDVEQKMSIRFLH

More preferably, human CHP1 comprises a polypeptide of SEQ ID NO: 22. Even more preferably, human CHP1 is a polypeptide having an amino acid sequence of at least 98 % homology to SEQ ID NO: 22. Most preferably, human CHP1 is a polypeptide of SEQ ID NO: 22.

A cDNA encoding CHP1 is given in SEQ ID NO:21, while a genomic sequence is given in SEQ ID NO:20.

As used throughout the present invention, the term "homology" may be understood in the broadest sense as sequence homology. As used in the art, the terms "sequence homology" and "sequence identity" may be understood interchangeably. The percentage of sequence homology may typically be determined by sequence alignment. Methods for such sequence alignment for the purpose of sequence comparison are well known by those skilled in the art. Preferably, homology as used herein is determined by means of the open access National Center for Biotechnology Information (NCBI) Basic Local Alignment Search Tool (BLAST) available from several sources, including the National Center for Biotechnology Information (NCBI, Bethesda, MD). In the context of amino acid sequences, the algorithm *blastp* may preferably be used. In the context of nucleotide sequences, the algorithm *blastn* may preferably be used.

As used throughout the present invention, the term "polypeptide" may be understood interchangeably with the terms "peptide" and "protein" in the broadest sense as any molecule that is mainly composed of amino acid moieties linked by amide bonds with another and comprises at least four consecutive amino acid moieties. Herein, the terms "amide bond" and "peptidic bond" may be understood interchangeably. Likewise, also the terms "moiety", "radical" and "residue" may be understood interchangeably.

As can be seen from the amino acid sequence of SEQ ID NO: 22, most preferably, CHP1 essentially consists of 195 consecutive amino acid moieties.

However, in particular in an individual suffering from a disorder associated with a pathological calcium homeostasis, impaired vesicular trafficking and/or disturbed neuronal and/or neuromuscular synaptic function, CHP1 may also optionally be modified such as by truncation and or by elongation. Therefore, the CHP1 polypeptide to be targeted may also comprise between 150 and 230 consecutive amino acids, more preferably between 160 and 220 consecutive amino acids, even more preferably between 170 and 210 consecutive amino acids, more preferably between 180 and 200 consecutive amino acids, even more preferably between 185 and 197 consecutive amino acids, even more preferably between 187 and 197 consecutive amino acids, even more preferably between 190 and 196 consecutive amino acids, even more preferably between 191 and 195 consecutive amino acids, even more preferably between 192 and 195 consecutive amino acids. Further, an individual suffering from a disorder associated with a pathological calcium homeostasis may also bear dimers, trimers or even complexes of higher order of CHP1 that are also embraced by the definition of the CHP1 of the present invention.

Preferably, any polypeptide of the present invention essentially consists of a linear chain of consecutive amino acid moieties. Preferably, the majority of amino acid moieties are natural amino acid moieties linked via amide bonds with another. The amino acid backbone of the polypeptide may optionally be modified (e.g., by methylation, alkylation, oxidation, cyclization, dehydration). In particular the polypeptide may or may not be subjected to one or more posttranslational modification(s) and/or be conjugated with one or more non-amino acid moiety/moieties. The termini of the polypeptide may, optionally, be capped by any means known in the art, such as, *e.g.,* amidation, acetylation, methylation, acylation. Posttranslational modifications are well-known in the art and may be but may not be limited to lipidation, phosphorylation, sulfatation, glycosylation, myristoilation, truncation, oxidation, reduction, decarboxylation, acetylation, amidation, deamidation, disulfide bond formation, amino acid addition, cofactor addition *(e.g.,* biotinylation, heme addition, eicosanoid addition, steroid addition) and complexation of metal ions, non-metal ions, peptides or small molecules and addition of iron-sulphide clusters. Moreover, optionally, co-factors, in particular cyclic guanidinium monophosphate (cGMP), but optionally also such as, *e.g.,* ATP, ADP, NAD⁺, NADH+H⁺, NADP⁺, NADPH+H⁺, metal ions, anions, lipids, etc. may be bound to the polypeptide, irrespective on the biological influence of these co-factors.

As used in the context of the present invention, the term "patient" may be understood in the broadest sense as any living being, which is preferably any animal, more preferably a mammal including human, in particular a human being.

The term "suffering from" as used herein may be understood in the broadest sense in a way that the patient has developed a disorder associated with a pathological calcium homeostasis, impaired vesicular trafficking and/or disturbed neuronal and/or neuromuscular synaptic function, i.e., that the disorder is present in the patient. The patient suffering from a disorder not necessarily but optionally bears medicinal symptoms such as, *e.g.,* muscle weakness and atrophy, pain and/or one or more other perceptional complaint(s). The patient may also be asymptomatic.

The term "being at risk of developing" means that the patient has a certain risk of having a disorder associated with a pathological calcium homeostasis, impaired vesicular trafficking and/or disturbed neuronal and/or neuromuscular synaptic function in the future. In this context, preferably, the patient has a higher risk compared to the average risk of developing a disorder associated with a pathological calcium homeostasis, impaired vesicular trafficking and/or disturbed neuronal and/or neuromuscular synaptic function present in the entire population. More preferably, the risk is at least 1.1-fold increased, even more preferably the risk is at least 1.5-fold increased, even more preferably the risk is at least 2-fold increased, even more preferably the risk is at least 5-fold increased.

As used herein, the term "disorder" may be understood in the broadest sense as any condition that differs from the healthy condition. Therefore, the terms "disorder" and "pathological condition" may be understood interchangeably. Preferably, a disorder is a "disease", thus a disorder accompanied by medicinal symptoms. In this context, the terms a "disease", "illness" and "ailment" may be understood interchangeably.

As used in the context of the present invention, a "disorder associated with a pathological calcium homeostasis" may be understood in the broadest sense as any pathological condition caused by or accompanied by a pathological calcium homeostasis.

In the context of the present invention, in a disorder with disturbed neuronal and/or neuromuscular synaptic function, the synaptic transmission may be disturbed at the neuronal and/or neuromuscular end plate. In disorders associated with a pathological calcium homeostasis the synaptic transmission may be generally disturbed.

In the context of the present invention, a disorder associated with impaired vesicular trafficking is a disorder where the vesicular trafficking within a cell is disturbed. Preferably, in such a disorder, endocytosis is impaired.

For example spinal muscular atrophy (SMA) as well as other motor neuron (MN) or neurodegenerative diseases such as Parkinson disease, amyotrophic lateral sclerosis (ALS), and hereditary spastic paraplegias are characterized by endocytosis and Ca²⁺ misregulation. In SMA a reduced Ca²⁺ influx as well as reduced endocytosis was shown to contribute to the disease pathology. Moreover, genes encoding for proteins that function in endosomal membrane trafficking were identified as new disease genes for Parkinson disease, amyotrophic lateral sclerosis, and hereditary spastic paraplegias. (Hosseinibarkooie et al., 2016; Riessland et al., 2017; Schreij et al., 2016).

Preferably, a disorder in the context of the present invention is a neuronal disorder, in particular a neuronal disorder selected from the group consisting of spinal muscular atrophies (SMA), amyotrophic lateral sclerosis (ALS), hereditary motor neuropathies (HMN) Parkinson's disease, Frontotemporal Dementia, Alzheimer's disease, Morbus Huntington, and polyglutamin expansion diseases.

As used herein, the inhibitor may bear any mode of action. It may reduce the local amount of CHP1 in a cell or may reduce the CHP1's biological activity. Preferably, the intracellular concentration of CHP1 is reduced.

Therefore, in a preferred embodiment, the inhibitor reduces the expression rate of CHP1, preferably wherein said inhibitor knocks down the CHP1 expression, in particular wherein knocking down is reducing the transcription rate of the CHP1 gene, reducing the translation rate of the CHP1 messenger ribonucleic acid (mRNA), reducing the transcript level of CHP1 and/or reducing the CHP1 function by pharmacological inhibitors.

In this context, knocking down the expression of CHP1 may preferably be performed by one or more antisense oligonucleotide(s) (ASOs) and/or one or more analogue(s) thereof.

As used throughout the present invention, the term "knocking down the expression" may be understood in the broadest sense as reducing the production rate of a certain polypeptide, in particular by means of reducing the transcription rate, the rate of correct splicing and/or the translation rate regarding said polypeptide.

Knocking down the translation rate of the CHP1 mRNA may preferably be performed by one or more interfering oligonucleotide(s).

Accordingly, in a preferred embodiment, the inhibitor is an oligonucleotide or an oligonucleotide analogue, in particular an oligonucleotide or an oligonucleotide analogue selected from the group consisting of:
(a) an antisense oligonucleotide, in particular an antisense deoxyribonucleic acid (asDNA), an antisense ribonucleic acid (asRNA);
(b) an antisense oligonucleotide analogue, in particular an antisense 2'-O-methoxyethyl (2'MOE) oligonucleotide, an antisense morpholino, Pip6a-morpholino phosphorodiamidate oligomer (PMO, an antisense peptide nucleic acid (PNA), an antisense glycol nucleic acid (GNA), an antisense locked nucleic acid (LNA) or an antisense threose nucleic acid (TNA);
(c) an interfering oligonucleotide, more preferably small interfering ribonucleic acid (siRNA), short hairpin ribonucleic acid (shRNA) or micro ribonucleic acid (microRNA), in particular siRNA of from 18 to 24 bases in length;
(d) an oligonucleotide modifying the splicing of pre-mRNA, in particular wherein said oligonucleotide is single stranded deoxyribonucleic acid (ssDNA) or single stranded ribonucleic acid (ssRNA);
(e) an oligonucleotide analogue modifying the splicing of pre-mRNA, in particular wherein said oligonucleotide is a 2'MOE, morpholino, PNA, GNA, LNA or TNA; and
(f) an oligonucleotide encoding for one or more of the aforementioned (a)-(e), optionally wherein said oligonucleotide is embedded in a vector or virus in particular a self-complementary adeno-associated viruses (scAAV).

As used in this context of the present invention, the term "antisense oligonucleotide" may be understood in the broadest sense as generally understood in the art. Therefore, an antisense oligonucleotide may be any single-stranded oligonucleotide complementary to the CHP1 mRNA and may, therefore also be designated as "CHP1 mRNA-interfering complementary oligonucleotides". It will be understood that an oligonucleotide according to the present invention may also comprise one or more modifications such as, e.g., one or more sulfur chemistry modification(s)/sulfatation *(e.g.,* phosphorothioates), methylation, alkylation, oxidation, lipidation, phosphorylation, glycosylation, oxidation, reduction, deamidation and/or partial intramolecular cyclization. Particularly preferably, an oligonucleotide according to the present invention comprises one or more nucleotide analogues shown in detail below.

Further, additionally or alternatively, the oligonucleotide may optionally comprise one or more non-nucleotide moiety/moieties and/or one or more non-natural nucleotide moiety/moieties. In particular, the termini of the oligonucleotide may, optionally, be capped by any means known in the art, such as, e.g., by sulfatation, amidation, acetylation, methylation, acylation, by one or more non-nucleotide moiety/moieties and/or by one or more non-natural nucleotide moiety/moieties. Optionally, the oligonucleotide may also be conjugated to any one of biotin, heme, eicosanoid(s), steroid(s), peptide(s) and/or small molecule(s). Preferably, such modified forms of oligonucleotide are those more stable against degradation in comparison with unmodified oligonucleotides.

An antisense oligonucleotide according to the present invention may be introduced into a cell to inhibit translation of CHP1 by base pairing to the mRNA encoding it and physically/sterically obstructing the translation machinery regarding CHP1. Most typically, an antisense oligonucleotide according to the present invention may bind to the CHP1 polypeptide-encoding region. However, an antisense oligonucleotide may also be complementary to an untranslated region (UTR) of the CHP1 mRNA, in particular such located at the 3' end or 5'UTR, in particular overlapping with the start codon (ATG) region. Typically, but not necessarily, such region will be in a range of not more than 40 base pairs (bp), more preferably not more than 30 bp, even more preferably not more than 20 bp from the CHP1 polypeptide-encoding region. Particularly preferably, an antisense oligonucleotide in the sense of the present invention is antisense RNA (asRNA). In this context, it may be noted that the 3'UTR of CHP1 is comparably long and comprises several regulatory elements, which might be inhibited.

An antisense oligonucleotide analogue according to the present invention acts in a way comparable with the action of an antisense oligonucleotide as laid out above. The only difference is that an antisense oligonucleotide analogue may typically be more stable against metabolic degradation. Therefore, the bonds between the moieties of the oligomers (monomers), thus, the nucleotide moiety analogues, of the antisense oligonucleotide analogue will typically be cleaved slower than the bonds between the corresponding nucleotide moieties, of the corresponding antisense oligonucleotide. Further, the rate of backbone and/or base modifications *(e.g.,* acetylation, glycosylation) may preferably be lower in the antisense oligonucleotide analogues.

An antisense 2'-O-methoxyethyl (2'MOE) oligonucleotide may be any oligonucleotide analogue comprising at least one 2'-O-methoxyethyl nucleotide analogues, preferably an oligonucleotide analogue wherein at least 10 % of the nucleotide moieties are 2'-O-methoxyethyl nucleotide analogues, more preferably an oligonucleotide analogue wherein at least 20 % of the nucleotide moieties are 2'-O-methoxyethyl nucleotide analogues, even more preferably an oligonucleotide analogue wherein at least 50 % of the nucleotide moieties are 2'-O-methoxyethyl nucleotide analogues, even more preferably an oligonucleotide analogue wherein at least 80 % of the nucleotide moieties are 2'-O-methoxyethyl nucleotide analogues, even more preferably an oligonucleotide analogue wherein at least 90 % of the nucleotide moieties are 2'-O-methoxyethyl nucleotide analogues, in particular even more preferably an oligonucleotide analogue wherein essentially all nucleotide moieties are 2'-O-methoxyethyl nucleotide analogues. This analogue nears an RNA-like structure.

A morpholino may also be designated as "phosphorodiamidate morpholino oligonucleotide" or "PMO" and may typically interact with a comparably small region of the complementary pre-mRNA or mRNA of from approximately 15 to approximately 30 bases in length. In a morpholino, the bases are bound to morpholine rings instead of ribose moieties in RNA and deoxyribose moieties in DNA, respectively. Accordingly, in a morpholino, the moieties are linked through phosphorodiamidate groups instead of phosphates.

As used throughout the present invention, the terms "peptide nucleic acid" and "PNA" may be understood in the broadest sense as any oligonucleotide analogue comprising repeating N-(2-aminoethyl)-glycine moieties linked by peptide bonds. Various purine and pyrimidine bases may be linked to the backbone by a methylene bridge (-CH₂-) and a carbonyl group (-(C=O)-).

As used throughout the present invention, the terms "glycol nucleic acid" and "GNA" may be understood in the broadest sense as any oligonucleotide analogue comprising 2,3-dihydroxypropylnucleoside analogues and repeating glycol moieties linked by phosphodiester bonds. Typically, in GNA, the Watson-Crick base pairing is comparably stable leading to comparably high melting temperatures of GNAs.

As used throughout the present invention, the terms "locked nucleic acid" and "LNA" may be understood in the broadest sense as any oligonucleotide analogue wherein the ribose moiety is modified with an extra bridge connecting the 2' oxygen and 4' carbon. LNA may also be designated as "inaccessible RNA". This bridge "locks" the ribose in the 3'-endo (North) conformation, which is often found in the A-form duplexes. Typically, the locked ribose conformation enhances base stacking and backbone pre-organization leading to an increased melting temperature.

As used throughout the present invention, the terms "threose nucleic acid" and "TNA" may be understood in the broadest sense as any oligonucleotide analogue comprising a backbone comprising repeating threose sugars linked together by phosphodiester bonds. TNA may form the helical geometry similar to A-form RNA.

In the above inhibitors, nucleotide moiety analogues may be conjugated to DNA and/or RNA moieties in a single molecule then comprising one or more nucleotide moiety/moieties and one or more DNA and/or RNA moiety/moieties whenever desired. Furthermore, additionally or alternatively, such molecule or the above oligonucleotide analogues may be hybridized with one or more DNA and/or RNA oligonucleotide(s) whenever desired.

As used in this context of the present invention, the term "interfering oligonucleotide" may be understood in the broadest sense as generally understood in the art. Accordingly, most typically, the interfering oligonucleotide may be a double-stranded oligonucleotide molecule of from approximately 20 bp to approximately 25 bp in length. Particularly preferably, an interfering oligonucleotide in the sense of the present invention is interfering RNA (iRNA), in particular small interfering RNA (siRNA) suitable for the well-known technology of RNA interference (RNAi), also known as "post-transcriptional gene silencing" (PTGS), specifically interfering with the expression of CHP1 encoded by a gene having a complementary nucleotide sequence. Such RNA may also be short shRNA and micro RNA. As used herein, the terms "small interfering RNA", "short interfering RNA" and "silencing RNA" may be understood interchangeably in the broadest sense as generally understood in the art. Accordingly, most typically, the siRNA may be a double-stranded RNA (dsRNA) molecule of from approximately 20 bp to approximately 25 bp in length.

As used herein, the term "precursor messenger RNA" (pre-mRNA) may be understood in the broadest sense as any immature single strand of messenger ribonucleic acid (mRNA). Typically, pre-mRNA is synthesized from a template of genomic DNA by transcription and comprises the bulk of heterogeneous nuclear RNA (hnRNA). Once pre-mRNA has been completely processed, it is typically designated as "mature messenger RNA" (mature mRNA).

The person skilled in the art will immediately know that pre-mRNA may be processed further by splicing. Such splicing processes are well-known in detail by any person skilled in the art. Therefore, "splicing" in the context of the present invention may be understood in the broadest sense as a process of modifying nascent pre-mRNA that may take place after or concurrently with its generation by transcription of the genomic DNA. By splicing, introns may be removed whereas the exons may preferably remain in the mature mRNA. In many cases this may be needed before the mRNA can be used to produce a correct polypeptide strand by mean of translation of the mRNA. For many eukaryotic introns, splicing is performed in a series of reactions typically catalyzed by the spliceosome, a complex of small nuclear ribonucleoproteins (snRNPs), but the person skilled in the art will also know self-splicing introns wherein splicing may typically be performed directly in the nucleus. Any splicing process may, in principle, be modified by oligonucleotides according to the present invention.

The oligonucleotide encoding for one or more of the aforementioned (a)-(e) may be any genetic material as known in the art and exemplified above.

The person skilled in the art will notice that, optionally,
(i) two or more antisense oligonucleotides;
(ii) more than one interfering oligonucleotide(s);
(iii) more than one interfering oligonucleotide analogue(s);
(iv) more than one oligonucleotides modifying pre-mRNA splicing; or
(v) more than one oligonucleotide analogues modifying pre-mRNA,
may be combined with another.

Moreover, the person skilled in the art will also notice that, optionally, one or more antisense oligonucleotide(s) may be combined with:
(i) one or more antisense oligonucleotide analogue(s);
(ii) one or more antisense oligonucleotide(s); and/or
(iii) one or more oligonucleotide(s) modifying pre-mRNA splicing.

Moreover, the person skilled in the art will also notice that, optionally, one or more antisense oligonucleotide(s) may be combined with:
(i) one or more oligonucleotide(s) modifying pre-mRNA splicing; and/or
(ii) one or more oligonucleotide analogue(s) modifying pre-mRNA splicing.

Moreover, the person skilled in the art will also notice that, optionally, one or more interfering oligonucleotide(s) may be combined with one or more oligonucleotide analogue(s) modifying pre-mRNA splicing;

Moreover, also one or more antisense oligonucleotide(s) and one or more antisense oligonucleotide analogue(s) may be combined with:
(i) one or more oligonucleotide(s) modifying pre-mRNA-splicing; or
(ii) one or more oligonucleotide analogue(s) modifying pre-mRNA-splicing.

Moreover, also one or more interfering oligonucleotide(s), one or more antisense oligonucleotide(s) and one or more oligonucleotide(s) modifying pre-mRNA splicing may be combined with another. Moreover, also one or more interfering oligonucleotide(s), one or more antisense oligonucleotide analogues(s) and one or more oligonucleotide(s) modifying pre-mRNA splicing may be combined with another. Moreover, also one or more interfering oligonucleotide(s), one or more antisense oligonucleotide(s) and one or more oligonucleotide analogues(s) modifying pre-mRNA splicing may be combined with another. Moreover, also one or more interfering oligonucleotide(s), one or more antisense oligonucleotide analogues(s) and one or more oligonucleotide analogues(s) modifying pre-mRNA splicing may be combined with another.

Furthermore, also four or even all of the above listed oligonucleotide and oligonucleotide analogue groups (a) to (f) may be combined with another.

The oligonucleotides in the sense of the present invention may be administered to cells and/or patients by any means known in the art. The person skilled in the art will know how many methods suitable for administering such molecules to cells and patients. Further, some of applicable methods are exemplified in the example section below.

Exemplarily, an oligonucleotide or an analogue thereof may be administered to cells by means of electroporation (e.g., single pulse or multi-pulse electroporation (e.g., nucleofection), one or more amphiphilic lipid(s), one or more cell-penetrating peptide(s) (e.g., the chariot peptide, a polyarginine (e.g., R7, R8, R9, R10, R11 or R12), the HIV tat peptide, a lactoferrin-derived peptide, or an antimicrobial peptide, a nucleic targeting sequence), one or more liposome(s), one or more micelle(s), one or more episome(s), one or more polymersome(s), one or more microbead(s), one or more nanobead(s), one or more amphiphilic polymer(s), one or more positively charged polymer(s) (e.g., polyethylene imine (PEI)), one or more virus(es) (e.g., self-complementary adeno-associated viruses (scAAV), an altered herpes simplex virus (HSV)), one or more viroid(s), and/or gene gun technology (e.g., by using gold beads).

Exemplarily, an oligonucleotide or an analogue thereof may be administered to a patient by means of one or more amphiphilic lipid(s), one or more cell-penetrating peptide(s), one or more liposome(s), one or more micelle(s), one or more polymersome(s), one or more microbead(s), one or more nanobead(s), one or more amphiphilic polymer(s), one or more positively charged polymer(s), one or more virus(es) and/or one or more viroid(s)

The person skilled in the art will know how to administer the oligonucleotides and analogues thereof. Administration to a patient may be systemically and/or locally. Preferably, the patients are injected intrathecally in order to directly target the brain and the spine and optionally concomitantly systemically (e.g., in the blood) in order to target the other organs.

Preferably, administration to a patient may include injecting the oligonucleotide(s) and/or analogue(s) thereof or the nasally uptake of these in order to circumvent the first pass effect.

Such oligonucleotide may be any one suitable for the purpose of the present invention, i.e., serving as an inhibitor of CHP1.

In a preferred embodiment, the inhibitor is an oligonucleotide having a sequence homology of at least 80 % to any of SEQ ID NOs: 1-8, preferably of at least 90 % to any of SEQ ID NOs: 1-8, more preferably of at least 95 % to any of SEQ ID NOs: 1-8, in particular wherein said oligonucleotide has a sequence of any of SEQ ID NOs: 1-8.

As used herein, the nucleotides of SEQ ID NOs: 1-8 have nucleic acid sequences of:

| | |
|---|---|
| SEQ ID NO:1: | TTCTTGCTTTCTAGTATTTAA |
| SEQ ID NO:2: | CACGTTATTGCGGGACGAAGA |
| SEQ ID NO:3: | CAATAAATCAATTGTTGTTAA |
| SEQ ID NO:4: | AGAGCTCGAGGAGATCAAGAA |
| SEQ ID NO:5: | ACGGAAGTGCCTAATATCCCA |
| SEQ ID NO:6: | TTGGCAAATGTTAATGACGGA |
| SEQ ID NO:7: | TTGGTTGACTTTCTGACTCAT |
| SEQ ID NO:8: | CAGCAGTATGCTTACAGGTTT |

As mentioned above, each of these sequences may be an oligonucleotide or a corresponding oligonucleotide analogue. Given are the DNA sequences. It will however be noted that also the corresponding RNA sequences are encompassed by the present invention. Then, the base "T" may be replaced by uracil ("U"). Highly preferably, the above sequences are:

| | |
|---|---|
| SEQ ID NO:1: | murine Mm Chp_1 |
| SEQ ID NO:2: | murine Mm Chp_2 |
| SEQ ID NO:3: | murine Mm Chp_3 |
| SEQ ID NO:4: | murine Mm Chp_4 |
| SEQ ID NO:5: | human Hs CHP_5 |
| SEQ ID NO:6: | human Hs CHP_6 |
| SEQ ID NO:7: | human Hs CHP_7 |
| SEQ ID NO:8: | human Hs CHP_8 |

Alternatively, the inhibitor may also be an oligonucleotide analogue corresponding to an oligonucleotide having a sequence homology of at least 80 % to any of SEQ ID NOs: 1-8, preferably of at least 90 % to any of SEQ ID NOs: 1-8, more preferably of at least 95 % to any of SEQ ID NOs: 1-8, in particular wherein said oligonucleotide has a sequence of any of SEQ ID NOs: 1-8.

Furthermore, the use of the inhibitors of CHP1 is exemplarily shown in the example section below.

The inhibitor of CHP1, in particular when it is an oligonucleotide, may be an unbound molecule or molecular complex or may be covalently and/or non-covalently bound to a high-molecular weight molecular structure. Such bonding may optionally stabilize the inhibitor, in particular when it is an oligonucleotide, against degradation when administered to a patient and/or to cells.

Therefore, in a preferred embodiment, the inhibitor is
(a) covalently and/or non-covalently bound to at least one cell-penetrating peptide and/or at least one membrane disrupting peptide;
(b) included in or covalently and/or non-covalently bound to a liposome;
(c) included in or covalently and/or non-covalently bound to a micelle;
(d) included in or covalently and/or non-covalently bound to a polymersome;
(e) included in an episome;
(f) covalently and/or non-covalently bound to or included in a microbead and/or nanobead; and/or
(g) covalently and/or non-covalently bound to a non-toxic polymer, preferably, wherein said polymer is a water soluble polymer, in particular wherein said polymer is polyethylene glycol (PEG), polyethylene imine (PEI), polylactic acid (PLA), polyglycolic acid (PGA), poly(lactic-co-glycolic acid) (PLGA), hydroxypropyl methacrylate copolymer (HPMA) or a copolymer or block polymer of two or more thereof.

In case of a covalent bond, the terms "bound to", "conjugated to", "conjugated with" may be understood interchangeably. In case of a non-covalent bond, the terms "bound to", "complexed by", "complexed with", "attached to" and "associated with" may be understood interchangeably. A cell-penetrating peptide (CPP) may be understood interchangeably with a protein transduction domain (PTD) as any polypeptide that may facilitate cellular uptake such as, e.g., the chariot peptide, a polyarginine (e.g., hexaarginine, heptaarginine, octaarginine, nonaarginine, decaarginine, etc.), the HIV tat peptide, a lactoferrin-derived peptide, or an antimicrobial peptide, a nucleic targeting sequence) that is typically between seven and 25 amino acids in length. A membrane disrupting peptide is most preferably an amphiphilic peptide such as an amphiphilic antimicrobial peptide.

Most preferably, the inhibitor is non-covalently bound to one or more of the above molecular structures that bear a positive net charge such as, e.g. polyarginine (s), positively charged liposomes and/or PEI.

As mentioned above, a disorder associated with a pathological calcium homeostasis may be any pathological condition caused by or accompanied by a pathological calcium homeostasis.

Experimentally, it could be demonstrated that downregulation of CHP1 counteracts spinal muscular atrophy (SMA) (see experimental section below). Accordingly, an inhibitor of CHP1 may be used for treating and/or preventing SMA in a patient.

Further, it could be experimentally demonstrated that Calcineurin B Homologous Protein 1 (CHP1) has a significant impact on regulation of endocytosis acting as a Ca²⁺-dependent regulator of endocytosis (see experimental section below). Surprisingly, it was found that upon reducing CHP1 activity (and/or increasing plastin 3 (PLS3) activity), endocytosis may be restored in various species, including mammals. Therefore, inhibiting CHP1 may be used to restore endocytosis. In this context, it may be noted that interestingly a number of neuronal disorders (in particular motoneuron diseases) are associated with impaired endocytosis, F-actin dynamics and/or Ca²⁺- homeostasis. Such diseases may be treated in the context of the present invention.

Therefore, an inhibitor of CHP1 may be used for treating or preventing a number of disorders associated with a pathological calcium homeostasis, impaired vesicular trafficking and/or disturbed neuronal and/or neuromuscular synaptic function, in particular neuronal disorders (e.g., motoneuron diseases, diseases with impaired synaptogenesis), in particular when such disorder is associated with impaired endocytosis, F-actin dynamics and/or Ca²⁺- homeostasis.

Accordingly, in a preferred embodiment, the disorder is a neuronal disorder, preferably a motoneuron disease, in particular a disorder selected from the group consisting of spinal muscular atrophy (SMA), amyotrophic lateral sclerosis (ALS), hereditary motor neuron disease (HMN) or a neurodegenerative disorders particularly Parkinson's disease, Frontotemporal Dementia, Alzheimer's disease, Morbus Huntington, and polyglutamic acid disease.

As used herein, the terms "neuronal disorder" and "neurological disorder" may be understood interchangeably in the broadest sense as any pathological condition of the body's nervous system.

As used herein, the terms "motoneuron disease" and "motoneuron lesion" may be understood interchangeably in the broadest sense as any lesion which affects nerve fibers traveling from the anterior horn of the spinal cord to the relevant muscle(s), i.e., to the motor neuron. Preferably, a motoneuron disease is characterized by flaccid paralysis, i.e., paralysis accompanied by muscle atrophy. Symptoms may or may not include, e.g., muscle paresis or paralysis, fibrillations, fasciculations, hypotonia, atonia, areflexia and/or hyporeflexia.

The patient may be administered with the inhibitor of the present invention alone or may be administered with a combination of one or more inhibitor(s) according to the present invention and one or more further compound(s).

In a preferred embodiment, the patient is further administered with a compound selected from the group consisting of HDAC inhibitors.

As used throughout the present invention, the terms "HDAC inhibitor", "histone deacetylase inhibitor" and "HDIs" may be understood interchangeably in the broadest sense as a compound interfering with the function of histone deacetylase. In this context, the compound may also be valproic acid (VPA), phenylbutyrate (PB), TSA, SAHA, LBH589, JNJ-26481585

The person skilled in the art will notice that the patient may also be administered with one or more one or more inhibitor(s) according to the present invention and two, three or even more of the above compounds and/or also other compounds suitable for treating or preventing a disorder associated with a pathological calcium homeostasis, impaired vesicular trafficking and/or disturbed neuronal and/or neuromuscular synaptic function. Examples for such combinations are shown in the example section below.

Moreover, alternatively or additionally, the patient may be further administered with one or more agent(s) increasing the activity of survival motor neuron (SMN).

Accordingly, in a preferred embodiment, the patient is further administered with an agent increasing survival motor neuron (SMN) activity, preferably wherein said agent is selected from the group consisting of:
(a) an agent increasing the expression rate of SMN, more preferably wherein said agent is an antisense oligonucleotide or an oligonucleotide analogue blocking a silencer or enhancing an enhancer of exon 7 inclusion of pre-mRNA of survival motor neuron 2 (SMN2), in particular wherein said agent is an oligonucleotide having a sequence homology of at least 80 % to any of SEQ ID NOs: 9-16, preferably of at least 90 % to any of SEQ ID NOs: 9-16, more preferably of at least 95 % to any of SEQ ID NOs: 9-16, more in particular wherein the oligonucleotide has a sequence of any of SEQ ID NOs: 9-16;
(b) an agent capable of inhibiting non coding RNAs which are inhibitors of SMN2,
(c) an agent increasing the rate of functional SMN, in particular wherein said agent is an oligonucleotide or an oligonucleotide analogue modifying pre-mRNA splicing;
(d) an agent comprising genetic material encoding for functional SMN, optionally wherein said genetic material is embedded in a vector;
(e) an agent stabilizing the SMN; or inhibiting the proteasomal degradation of SMN;
(f) an agent that is increasing the activity of the SMN;
(g) replacement by gene therapy expressing SMN1 in an self-complementary adenovirus vector,
(h) an agent increasing PLS3 activity, and
(i) an chemical molecule which increases the inclusion of exon 7 by enhancing U1-pre-mRNA association such as compounds which have been identified by Roche (pyrido-pyrimidinone series such as SMN-C3, or SMN-C4 also know n as RG7800 or RG7916) or Novartis (NVS-SM1, 5-(1H-pyrazol-4-yl)-2-[6-[(2,2,6,6-tetramethyl-4-piperidyl)oxy]-pyridazin-3-yl]phenol (LMI070))) (Naryshkin et al., 2014; Palacino et al., 2015; Pinard et al., 2017).

As used throughout the present invention, the term "increasing the SMN activity" may be understood in the broadest sense as boosting of the function of SMN. This may be achieved by increasing the amount/concentration of functional SMN and/or by activating SMN already present in a cell and/or in a patient.

The term "rate of functional SMN" as used herein refers to the fraction of SMN that is fully functional, i.e., has at least 10 %, preferably at least 20 %, more preferably at least 30 %, even more preferably at least 40 %, even more preferably at least 50 %, even more preferably at least 60%, even more preferably at least 70 %, even more preferably at least 80 %, even more preferably at least 90 % and most preferably approximately 100 % or even more compared to SMN1.

As used herein, SMN may be survival of motor neuron 1 (SMN1) and/or survival of motor neuron 2 (SMN2). When herein referred to "SMN activity" in general, this may be understood as the overall activity of SMN1 and SMN2 together. Typically, the gene encoding SMN1 is considerably more expressed than a corresponding SMN2 gene.

As used throughout the present invention, the term "increasing the expression rate" may be understood in the broadest sense as boosting the production of a certain polypeptide, in particular by means of increasing the transcription rate, the rate of correct splicing and/or the translation rate regarding said polypeptide.

In this context, the terms "antisense oligonucleotide", "antisense oligonucleotide analogue", "interfering oligonucleotide", "oligonucleotide modifying pre-mRNA splicing" and "oligonucleotide analogue modifying pre-mRNA splicing" may be understood in the broadest sense as generally understood in the art and as laid out in the context of the respective SMN- and CHP1-related oligonucleotides and analogues thereof defined above. The person skilled in the art will know how to administer such molecules to cells and patients. This is further exemplified in the context of SMN- and CHP1-related oligonucleotides above.

An oligonucleotide modifying SMN2 pre-mRNA splicing or analogue thereof may cause the generation of an mRNA strand originating from the SMN2 gene encoding for a polypeptide that bears at least 60 %, more preferably at least 70 %, even more preferably at least 80 %, even more preferably at least 90 %, in particular approximately 100 % or even more biological activity compared to SMN1. Most preferably said oligonucleotide modifying SMN2 pre-mRNA splicing or analogue thereof facilitating exon 7 inclusion into the mRNA. As used throughout the invention, a silencer of SMN2 expression may be an intronic or exonic silencer and an enhancer may be an exonic or intronic enhancer.

Preferably, the oligonucleotide or analogue thereof may also be directed against a silencer in intron 7. Alternatively, the oligonucleotide or analogue thereof may also be directed against a silencer in intron 6 or is amplifying the exonic splice enhancer. When amplifying the exonic splice enhancer, the correct splicing of exon 7 may be regulated by several *cis* regulatory intronic an exonic enhancers and silencers. Further, several *trans* splicing factors binding to the *cis* elements may influence the splicing pattern (e.g., TRA2B, SRSF1, RBMX, HNRNPA1, TARDBP, SRSF9).

Accordingly, in a highly-preferred embodiment, the patient is administered with an inhibitor of CHP1 and with an agent increasing SMN activity. Particularly preferably, the patient is administered with an oligonucleotide or an oligonucleotide analogue knocking down CHP1 and with an oligonucleotide or an oligonucleotide analogue knocking down a silencer region of the SMN2 gene (double knockdown of CHP1 and SMN2 silencer) or alternative an oligonucleotide or an oligonucleotide analogue enhancing a splice enhancer of exon 7 of the SMN2 gene. The inhibitor of CHP1 and the agent increasing SMN activity may be administered concomitantly or subsequently, preferably essentially concomitantly, i.e., within a time frame of not more than 24 h, preferably not more than 10 h, even more preferably not more than 5 h, in particular not more than 2 h.

Indeed, the presence of SMN molecules in the patient and/or cell of interest may improve patient's health, such as, e.g., the health of a patient suffering from SMA. This may be achieved by increasing the expression of an SMN2 gene, by correcting the SMN2 pre-mRNA splicing, by increasing the SMN stability or by means of gene therapy (e.g., via a viral vector such as, e.g., scAAV-SMN1).

As used herein, SEQ ID NOs: 9-19 refer to nucleic acid sequences of:

| | |
|---|---|
| SEQ ID NO: 9: | ATTCACTTTCATAATGCTGG |
| SEQ ID NO: 10: | TCACTTTCATAATGCTGG |
| SEQ ID NO: 11: | TTTCATAATGCTGGC |
| SEQ ID NO: 12: | ATTCACTTTCATAATGCTGG |
| SEQ ID NO: 13: | GTAAGATTCACTTTCATAATGCTGG |
| SEQ ID NO: 14: | AGATTCACTTTCATAATGCTGG |
| SEQ ID NO: 15: | AAGAAGGAAAGTGCTCACATA |
| SEQ ID NO: 16: | CGACATCTTCTGCACCATTGGC |
| SEQ ID NO: 17: | CAGAGTCCTCGGTAGAACTT |
| SEQ ID NO:18: | GAAATCTAATTTTTCAGTTT |
| SEQ ID NO:19: | CATAGUGGAACAGAT |

As mentioned above, each of these sequences may be an oligonucleotide or a corresponding oligonucleotide analogue. Given are the DNA sequences. It will however be noted that also the corresponding RNA sequences are encompassed by the present invention. Then, the base "T" may be replaced by uracil ("U"). Highly preferably, the above sequences are:

| | |
|---|---|
| SEQ ID NO: 9: | SMN2 human ISS blocking morpholino |
| SEQ ID NO: 10: | SMN2 human ISS blocking ASO 10-27 2'-O-methoxyethyl-modified oligonucleotide with phosphorothioate backbone |
| SEQ ID NO: 11: | SMN2 human ISS blocking ASO 09-23 2'-O-methoxyethyl-modified oligonucleotide with phosphorothioate backbone |
| SEQ ID NO: 12: | SMN2 human ISS blocking phosporodiaminidate morpholino oligonucleotide (PMO) SMN2E7 (-10-29) |
| SEQ ID NO: 13: | SMN2 human ISS blocking phosporodiaminidate morpholino oligonucleotide (PMO) SMN2E7 (-10-34) |
| SEQ ID NO: 14: | SMN2 human ISS blocking phosporodiaminidate morpholino oligonucleotide (PMO) SMN2E7 (-10-31) |
| SEQ ID NO: 15: | SMN silencer, preferably, SMN-siRNA |
| SEQ ID NO: 16: | morpholino knocking down SMN |
| SEQ ID NO: 17: | Non-coding SMN antisense AS1 (d'Ydewalle et al., 2017) |
| SEQ ID NO:18: | Non-coding SMN antisense AS2 (d'Ydewalle et al., 2017) |
| SEQ ID NO:19: | Non-coding SMN antisense IncRNA (Woo et al 2017) |

Alternatively, the agent increasing SMN activity may also be an oligonucleotide analogue corresponding to an oligonucleotide having a sequence homology of at least 80 % to any of SEQ ID NOs: 9-16, preferably of at least 90 % to any of SEQ ID NOs: 9-16, more preferably of at least 95 % to any of SEQ ID NOs: 9-16, in particular wherein said oligonucleotide has a sequence of any of SEQ ID NOs: 9-16.

The person skilled in the art will notice that the agent increasing SMN activity may also be covalently and/or non-covalently bound to one or more cell-penetrating peptide(s), membrane disrupting peptide(s), liposome(s), micelle(s) and/or non-toxic polymer(s) as described in the context of the inhibitor of CHP1 above. Alternatively, the agent increasing SMN activity may also be a vector comprising a gene encoding for SMN (e.g., a viral vector such as, e.g., scAAV-SMN) or may be a vector (e.g., a viral vector) encoding for shRNA(s) or microRNA(s). The agent increasing SMN activity may optional also be administered in combination with one or more other compound(s) as described above. Examples for such combinations are shown in the example section below.

The patient may be administered once or more often. Optionally, the patient may be administered once per day, twice per day or three times a day. Alternatively, the patient may be administered every second day, twice a week or even less frequently.

The inhibitor(s) of CHP1 and optionally also the agent(s) increasing SMN2 activity and/or the further compound(s) may be administered to the patient by any means known in the art such as, e.g., by injection or by oral, nasal or transdermal administration. Administration may be local administration (e.g., intrathecally, intracerebroventricularly (*icv*)*,* topically or intravitreally) or systemic administration (e.g., intravenously (*i.v.*), intraarterially (*i.a.*), intraperitoneally (*i.p.*), intramusculary (*i.m.*), subcutaneously (*s.c.*)). In particular when the inhibitor is an oligonucleotide or an analogue thereof, it is preferably administered to the patient by injection or nasally in order to circumvent the first past effect and the accompanied degradation. Preferably, administration is performed by injection, more preferably systemic injection, in particular intravenous injection.

The term "genetic material encoding for functional SMN" may be any genetic material that may convey genetic information known in the art such as, e.g., DNA, RNA or any analogue thereof. It may be a linear strand or may be circular (e.g., a plasmid). Optionally, it may be embedded into any vector known in the art such as, e.g., a plasmid, an episome, a virus, a viroid or a bacterial cell. Preferably, the genetic material may further comprise at least one promoter region and optionally one or more enhancer region(s).

In the context of the present invention, the inhibitors of CHP1 according to the present invention cannot only be used in a patient *in vivo,* but also in other animal models *in vivo,* cell or tissue culture *in vitro.*

In a further aspect, the present invention also relates to a pharmaceutical composition comprising an inhibitor according to the present invention and a pharmaceutically acceptable carrier.

It will be noticed that the pharmaceutical composition may optionally further comprise one or more further compound(s) such as, e.g., an HDAC inhibitor and/or an agent increasing SMN activity.

As used herein, the term "pharmaceutically acceptable carrier" may refer to any substance that may support the pharmacological acceptance of the inhibitor. The pharmaceutical composition may be administered orally or may be injected. It may be pharmaceutically formulated in a dry form (e.g., as a powder, a tablet, a pill, a capsule, a chewable capsule, etc.) or a liquid (e.g., a spray, a syrup, a juice, a gel, a liquid, a paste, an injection solution, an aerosol, an enema, etc.) A pharmaceutically acceptable carrier may be a solvent with no or low toxicity such as, e.g., an aqueous buffer, saline, water, dimethyl sulfoxide (DMSO), ethanol, vegetable oil, paraffin oil or combinations thereof. Furthermore, the pharmaceutically acceptable carrier may contain one or more detergent(s), one or more foaming agent(s) (e.g., sodium lauryl sulfate (SLS), sodium doceyl sulfate (SDS)), one or more coloring agent(s) (e.g., TiO₂, food coloring), one or more vitamin(s), one or more salt(s) (e.g., sodium, potassium, calcium, zinc salts), one or more humectant(s) (e.g., sorbitol, glycerol, mannitol, propylenglycol, polydextrose), one or more enzyme(s), one or more preserving agent(s) (e.g., benzoic acid, methylparabene), one or more texturing agent(s) (e.g., carboxymethyl cellulose (CMC), polyethylene glycol (PEG), sorbitol), one or more emulsifier(s), one or more bulking agent(s), one or more glacing agent(s), one or more separating agent(s), one or more antioxidant(s), one or more herbal and plant extract(s), one or more stabilizing agent(s), one or more polymer(s) (e.g., hydroxypropyl methacrylamide (HPMA), polyethylene imine (PEI), carboxymethyl cellulose (CMC), polyethylene glycol (PEG)), one or more uptake mediator(s) (e.g., polyethylene imine (PEI), dimethyl sulfoxide (DMSO), a cell-penetrating peptide (CPP), a protein transduction domain (PTD), an antimicrobial peptide, etc.) one or more antibody/antibodies, one or more sweetener(s) (e.g., sucrose, acesulfam K, saccharin Na, stevia), one or more counterstain dye(s) (e.g., fluorescein, fluorescein derivatives, Cy dyes, an Alexa Fluor dyes, S dyes, rhodamine, quantum dots, etc.), one or more gustatory substance(s) and/or one or more fragrance(s).

Accordingly, in a further aspect, the present invention refers to the use of an inhibitor of CHP1 for inducing axon proliferation *in vitro.*

A still further related aspect of the present invention refers to the use of an inhibitor of CHP1 to restore impaired endocytosis in synaptic terminals contributing to development, maturation and maintenance of synapses and neuromuscular junctions (NMJs) and, thus, also for synaptic particular neuromuscular junction (NMJ) development, maturation and maintenance *in vitro.*

As used throughout the present invention, the terms *"in vitro"* and *"ex vivo"* may be understood interchangeably in the broadest sense as any use or method not considered as therapeutic or diagnostic use or method as defined by patent practice in the corresponding jurisdiction. Therefore, *"in vitro"* preferably means that there is no substantial health risk for a patient involved when carrying out the invention.

The axon proliferation may preferably be accompanied by a differentiation and/or maturation of precursor cells, in particular of neuronal stem cells (NSCs). Then, the use of an inhibitor of CHP1 may include contacting it with precursor cells, in particular neuronal stem cells (NSCs). As used herein, the differentiation and/or maturation of precursor cells may preferably be the differentiation into motoneuronal cells. Most preferably, the use of an inhibitor of CHP1 includes contacting said inhibitor with neuronal stem cells (NSCs) which, under influence of said inhibitor, differentiate and maturate into motoneuronal cells.

In the context of the use of the inhibitor of CHP1 according to the present invention *in vitro,* all embodiments and specifications laid out above also apply.

Therefore, in a preferred embodiment, the inhibitor of CHP1 is defined as in the context of the inhibitor above.

The use according to the present invention may also include the optional co-incubation of cells with the inhibitor of CHP1 and with an HDAC inhibitor and/or with agent increasing SMN activity. This co-incubation may be concomitant incubation and/or subsequent incubation. This is further exemplified in the example section.

The use of an inhibitor of CHP1 for inducing axon proliferation *in vitro* in the sense of the present invention may also be used for producing matured or partly matured motoneurons. As used herein, the terms "motoneuron" and "motor neuron" may be understood interchangeably. Such motoneurons may be used in many different applications such as for treating a pathological condition associated with disordered and/or injured spinal cord and/or nerve cord(s) in a patient suffering therefrom. Exemplarily, said pathological condition is spinal muscular atrophy. In the context of such pathological condition, neuronal stem cells (NSCs) may be obtained from a patient, differentiated into motoneurons *in vitro* and administered back into the same patient (autologous grafting) or another patient (heterologous grafting), preferably the same patient.

The use of an inhibitor of CHP1 may be used for counteracting disturbed Ca²⁺ homeostasis at synaptic terminals and/or growth cone, which may thereby improve development, maturation and maintenance of synapses and in particular neuromuscular junction (NMJs) in patients with motor neuron diseases (SMA, ALS, HMN) or neurodegenerative disorders (Parkinson's disease, Alzheimer's disease, Morbus Huntington or polyglutamine disorders).

Optionally, the obtained motoneuronal cells may be used in a method for treating or preventing a disorder associated with a pathological calcium homeostasis, impaired vesicular trafficking and/or disturbed neuronal and/or neuromuscular function, preferably wherein said disorder is a neuronal disorder, more preferably a motoneuron disease, in particular a disorder selected from the group consisting of spinal muscular atrophies (SMA), amyotrophic lateral sclerosis (ALS), hereditary motor neuron (HMNN diseases or neurodegenerative disorders in particular Parkinson's disease, Alzheimer's disease, Morbus Huntington, and polyglutamic acid disease.

Furthermore, also *in vitro,* the inhibitor of CHP1 may be combined with one or more further compounds, in particular those defined above, and/or with one or more agent(s) increasing SMN activity, in particular such as defined above. Examples for such combinations are shown in the example section below.

A still further aspect of the present invention relates to a method for the differentiation and/or maturation of neuronal stem cells (NSCs) in *vitro* comprising the following steps:
i) providing neuronal stem cells (NSCs);
ii) contacting said cells with an inhibitor of CHP1; and
iii) cultivating the cells under conditions allowing their differentiation and/or maturation.

In the context of this method according to the present invention, all embodiments and specifications laid out above also apply.

As used herein, the terms "neuronal stem cell", "NSC", "neuronal precursor cell", "NPC" and "induced pluripotent stem cells", "iPSC") may be understood interchangeably in the broadest sense as any self-renewing, multipotent cells that are able to generate the main phenotypes of the nervous system such as, e.g., neurons, astrocytes, and oligodendrocytes. Typically, NSCs are characterized by their capability to differentiate into multiple cell types via exogenous stimuli from their environment.

The person skilled in the art will notice that NCSs as used by the present invention are preferably not embryonic stem cells, in particular not human embryonic stem cells, but rather adult stem cells, in particular pluripotent adult stem cells or induced pluripotent stem cell (in particular such derived from a somatic cell, particular fibroblasts, keratinocytes).

As described in detail above, the NSCs may be contacted with the inhibitor by any means, such as, e.g., by means of co-incubation (i.e., admixing of the inhibitor into the culture medium), electroporation (e.g., single pulse or multi-pulse electroporation (e.g., nucleofection), one or more amphiphilic lipid(s), one or more cell-penetrating peptide(s) (e.g., the chariot peptide, a polyarginine (e.g., R7, R8, R9, R10, R11 or R12), the HIV tat peptide, a lactoferrin-derived peptide, or an antimicrobial peptide, a nucleic targeting sequence), one or more liposome(s), one or more micelle(s), one or more polymersome(s), one or more microbead(s), one or more nanobead(s), one or more amphiphilic polymer(s), one or more positively charged polymer(s) (e.g., polyethylene imine (PEI)), one or more virus(es) (e.g., an altered herpes simplex virus (HSV)), one or more viroid(s), and/or gene gun technology (e.g., by using gold beads). Examples are further provided in the example section below.

The conditions for cultivating the cells allowing their differentiation and/or maturation may be any conditions suitable for this purpose. The person skilled in the art will know such conditions well. Typically, the cells may be cultivated at a temperature range of from approximately 20°C to approximately 45°C, preferably at a temperature range of from approximately 25°C to approximately 44°C, more preferably at a temperature range of from approximately 28°C to approximately 43°C, even more preferably at a temperature range of from approximately 30°C to approximately 42°C, even more preferably at a temperature range of from approximately 32°C to approximately 41°C, even more preferably at a temperature range of from approximately 34°C to approximately 40°C, even more preferably at a temperature range of from approximately 34°C to approximately 39°C, even more preferably at a temperature range of from approximately 36°C to approximately 38°C, in particular at a temperature of approximately 37°C. Typically, the cells may be cultivated in an aqueous buffer bearing a pH in the range of from approximately pH 6 to approximately pH 8, preferably in the range of from approximately pH 6.5 to approximately pH 7.8, more preferably in the range of from approximately pH 6.8 to approximately pH 7.6, even more preferably in the range of from approximately pH 7.0 to approximately pH 7.5, even more preferably in the range of from approximately pH 7.2 to approximately pH 7.5, in particular at a pH of approximately pH 7.4. Optionally, the pH may be adjusted by means of using an open buffer system such as a buffer system including gassing with carbon dioxide. The aqueous buffer may further comprise nutrients sufficient for cellular health such as, e.g., one or more carbohydrate source(s) (e.g., sugars), vitamins, amino acids, essential minerals and/or growth factors. The aqueous buffer may preferably be changed after certain time intervals in order to remove metabolic products and to add nutrients sufficient for cellular health. It will be noticed that any compounds toxic to the cells may preferably be avoided. Examples are further provided in the example section below.

Therefore, in a preferred embodiment, the inhibitor of CHP1 is defined as in the context of the inhibitor and the *in vitro* use above.

Furthermore, also in the method performed *in vitro,* the inhibitor of CHP1 may be combined with one or more further compounds, in particular those defined above, and/or with one or more agent(s) increasing SMN activity, in particular such as defined above.

Therefore, in a preferred embodiment, the method further comprises the step of administering to the cells at least one selected from the following:
(I) an agent increasing survival motor neuron (SMN) activity, preferably wherein said agent is selected from the group consisting of:
   (a) an agent increasing the expression rate of SMN, more preferably wherein said agent is an antisense oligonucleotide or an oligonucleotide analogue blocking a silencer or enhancing an enhancer of exon 7 inclusion of pre-mRNA of survival motor neuron 2 (SMN2), in particular wherein said agent is an oligonucleotide having a sequence homology of at least 80 % to any of SEQ ID NOs: 9-16, preferably of at least 90 % to any of SEQ ID NOs: 9-16, more preferably of at least 95 % to any of SEQ ID NOs: 9-16, more in particular wherein the oligonucleotide has a sequence of any of SEQ ID NOs: 9-16;
   (b) an agent capable of inhibiting non coding RNAs which are inhibitors of SMN2,
   (c) an agent increasing the rate of functional SMN, in particular wherein said agent is an oligonucleotide or an oligonucleotide analogue modifying pre-mRNA splicing;
   (d) an agent comprising genetic material encoding for functional SMN, optionally wherein said genetic material is embedded in a vector;
   (e) an agent stabilizing the SMN; or inhibiting the proteasomal degradation of SMN;
   (f) an agent that is increasing the activity of the SMN;
   (g) replacement by gene therapy expressing SMN1 in an self complementary adenovirus vector,
   (h) an agent increasing PLS3 activity, suppressing NCALD activity, and
   (i) an chemical molecule which increases the inclusion of exon 7 by enhancing U1-pre-mRNA association such as compounds which have been identified by Roche (pyrido-pyrimidinone series such as SMN-C3, or SMN-C4 also know n as RG7800 or RG7916) or Novartis (NVS-SM1, 5-(1H-pyrazol-4-yl)-2-[6-[(2,2,6,6-tetramethyl-4-piperidyl)oxy]-pyridazin-3-yl]phenol (LMI070))) (Naryshkin et al., 2014; Palacino et al., 2015; Pinard et al., 2017)
   and
(II) an HDAC inhibitor.

Examples for such combinations are shown in the example section below.

The administration of an inhibitor of CHP1, in particular an oligonucleotide or oligonucleotide analogue knocking down Chop1, into the cells *in vitro* may, under suitable conditions, trigger the maturation of NSCs, i.e., trigger the differentiation from NSCs into motoneurons and improve development, maturation and maintenance of synapses and neuromuscular junctions (NMJs). In this context, the compound may also be an HDACi (VPA, PB, TSA, SAHA, LBH589, JNJ-26481585)

Therefore, the method according to the present invention laid out in detail above may also be used for producing matured or partly matured motoneurons. Such motoneurons may be used in many different applications such as for treating a pathological condition associated with disordered and/or injured spinal cord and/or nerve cord(s) in a patient suffering therefrom. Exemplarily, said pathological condition is spinal muscular atrophy.

Accordingly, a further aspect of the present invention relates to a matured cell obtainable from the method according to the present invention for use in a method for the treatment of a pathological condition associated with disordered and/or injured spinal cord and/or nerve cord(s) in a patient suffering therefrom, in particular wherein said pathological condition is spinal muscular atrophy.

As described above, most preferably, such matured cell is a motoneuron or a precursor thereof.

In the context of this aspect, the matured cells obtainable from the NSCs matured by means of the above method *in vitro* may be administered back into the same patient the NSCs had been obtained from (autologous grafting) or into another patient (heterologous grafting), preferably the same patient.

The pathological condition may also be any other kind of spinal marrow lesion or motoneuron-associated disease.

The matured cell obtainable from the method according to the present invention may be administered to the patient by any means known in the art for such purpose. Preferably the cell is injected. Administration may be local administration (e.g., intrathecally or intravitreally) or systemic administration (e.g., intravenously (*i.v.*), intraarterially (*i.a.*), intraperitoneally *(i.p.),* intramusculary (*i.m*.), subcutaneously *(s.c.)).*

Further, a matured cell obtainable from the method according to the present invention may be used in a method for treating or preventing a disorder associated with a pathological calcium homeostasis, preferably wherein said disorder is a neuronal disorder, more preferably a motoneuron disease, in particular a disorder selected from the group consisting of spinal muscular atrophy (SMA), amyotrophic lateral sclerosis (ALS), hereditary motor neuron diseases (HMN) or neurodegenerative disorders in particular Parkinson's disease, Alzheimer's disease, Morbus Huntington, and polyglutamic acid disease.

In a yet further aspect, the present invention relates to a method for the treatment or prevention of a patient suffering from or being at risk of developing a disorder associated with a pathological calcium homeostasis, impaired vesicular trafficking and/or disturbed neuronal and/or neuromuscular function, wherein the patient is administered with an inhibitor of Calcineurin B Homologous Protein 1 (CHP1) in an amount sufficient for treating or preventing said disorder.

The embodiments disclosed above for the corresponding use of the invention also apply to this method of the invention.

A still further aspect of the present invention relates to a method for treating a pathological condition associated with disordered and/or injured spinal cord and/or nerve cord(s) in a patient suffering therefrom, wherein said patient is administered with matured cells obtainable from the method of the present invention in an amount sufficient for treating said pathological condition, or wherein said patient is administered the CHP1 inhibitor as mentioned above, in particular wherein said pathological condition is a motoneuron disease.

The embodiments disclosed above for the corresponding use of the invention also apply to this method of the invention.

The following examples as well as the accompanying figures are intended to provide illustrative embodiments of the present invention described and claimed herein. These example is not intended to provide any limitation on the scope of the invented subject-matter.

### Figure Legends

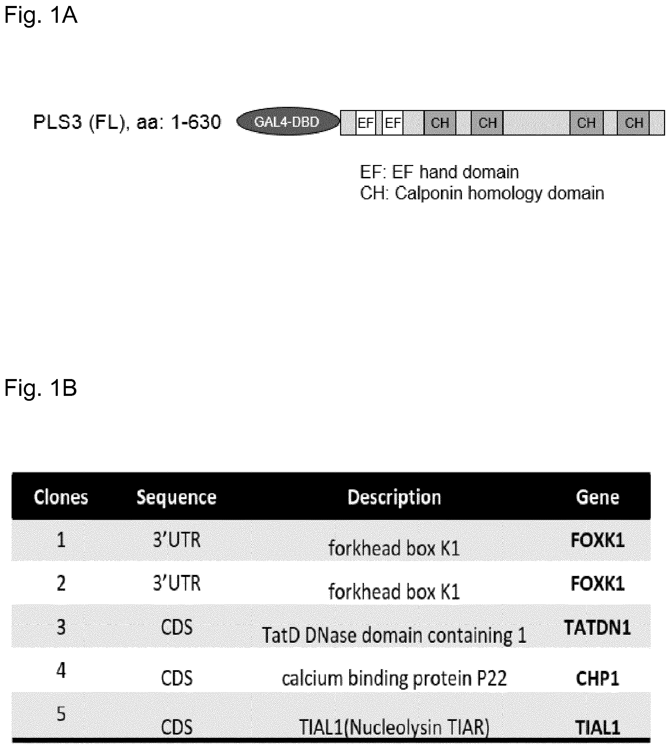

**Figure 1****:** Identification of CHP1 as PLS3 binding partners by yeast two-hybrid screening.
   A.) PLS3 full-length (FL) was cloned into the PBTG9 vector with a GAL4 DNA binding domain (GAL4-DBD).
   B.) Sequencing results of 5 different clones after yeast two-hybrid screen. TATDN1 is considered to be a false positive result, since it is present in many yeast two-hybrid screens. Sequencing result of the *TIAL1* indicate that this fragment included an exon and part of an intronic region. Sequencing result and alignment with CHP1 demonstrate that the C-terminal part of CHP1 could be considered as the potential interacting domain with PLS3.

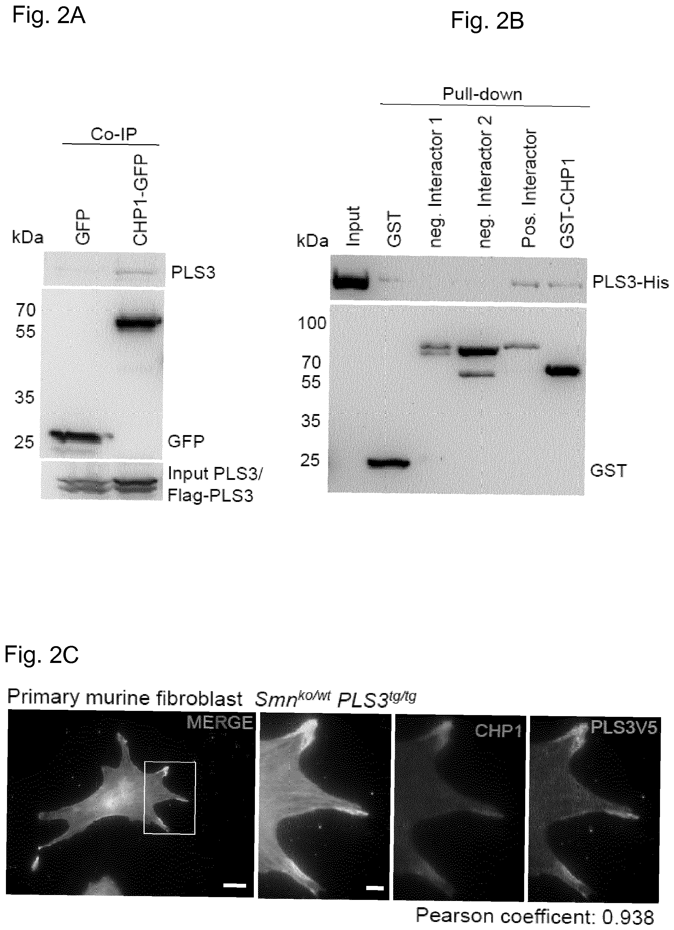

**Figure 2****:** PLS3 and CHP1 are direct interacting partners.
   A.) Western blot analysis upon Co-IP experiments show that CHP1 and PLS3 are interacting. HEK293T cells were transiently co-transfected with CHP1-GFP and FLAG-His-PLS3. Co-Immunoprecipitation with GFP affinity microbeads revealed that CHP1 and PLS3 are co-precipitating.
   B.) Pull-down assay of purified GST-CHP1 and PLS3-His shows that CHP1 and PLS3 are directly interacting. Input: purified PLS3-His protein.
   C.) Co-localization analysis of CHP1 and PLS3V5 in *Smn*^{*ko*/}*^{wt}; PLS3V5*^{*tg*/*tg*} transgenic MEFs. CHP1 and PLS3V5 co-localize in filopodia. *α*-CHP1 antibody (blue), *α-*V5 antibody (green) and Phalloidin to stain F-actin filaments (purple). Left figure (scale: 20 µm), magnifications of insets of the left figure (scale: 5 µm).

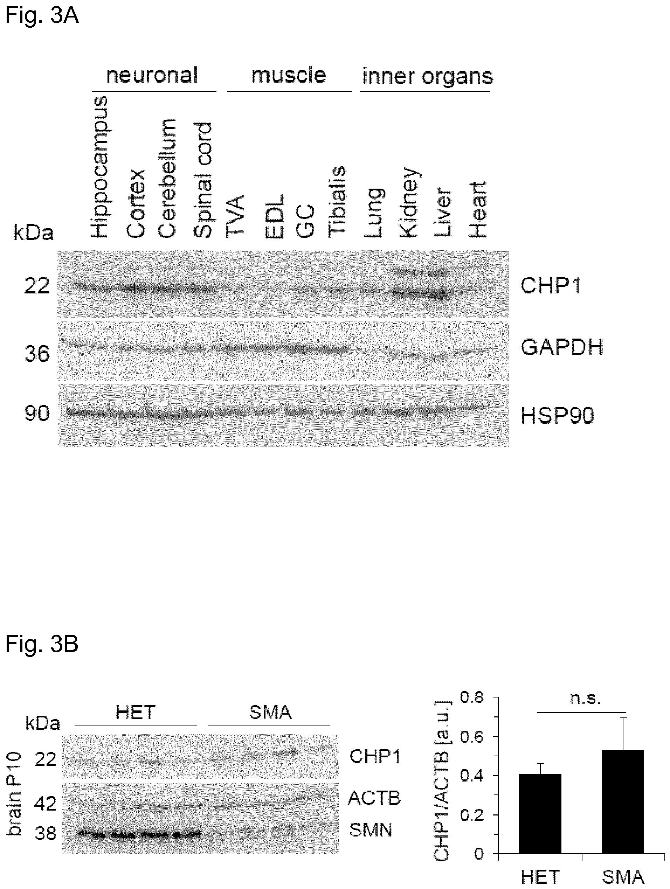

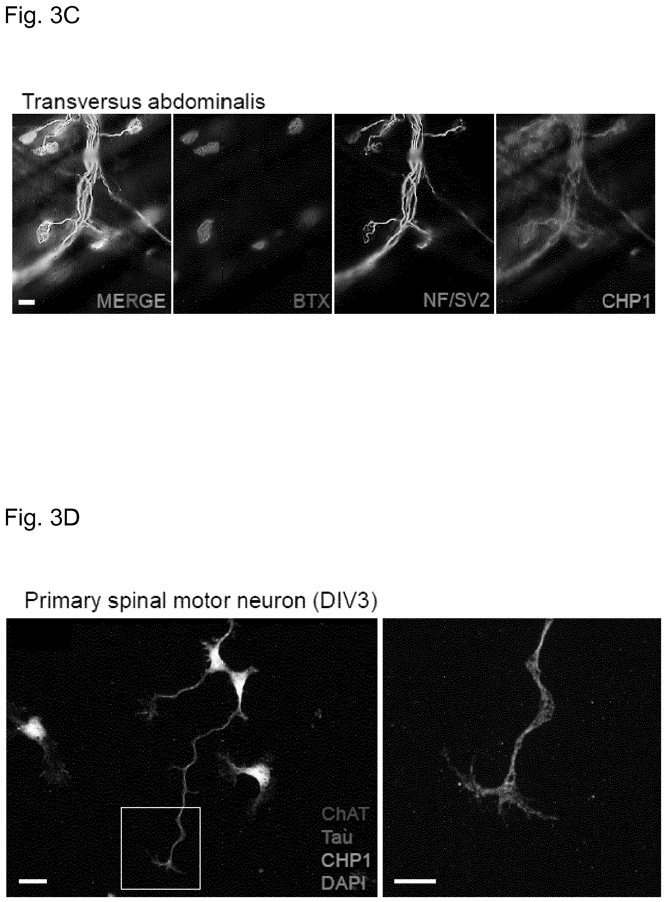

**Figure 3****:** CHP1 is ubiquitously expressed.
   A.) Western blot analysis of different organs shows that CHP1 is ubiquitously expressed and especially high in neuronal tissues.
   B.) Western blot and quantification of CHP1, SMN and ACTB (loading control) in brain of P10 HET (*Smn*^{*ko*/}*^{wt}; SMN2*^{*tg*/*0*}) and SMA (*Smn*^{*ko*/}*^{ko}; SMN2*^{*tg*/*0*}) mice. n.s. not significant. Error bars represent SEM.
   C.) Immunostaining of CHP1 in TVA muscle shows CHP1 expression at pre- and post-synapse. *α*-CHP1 (green), α-bungarotoxin (BTX) (purple), α-neurofilament (NF) and α-synaptic vesicle (SV2) (blue) (scale: 20 µm).
   D.) CHP1 immunostaining of DIV3 MN isolated from WT E13.5 embryos reveals that CHP1 is broadly expressed in motor neurons (MNs) (scale: 20 µm). Magnification: high expression of CHP1 in the growth cone (scale: 10 µm).

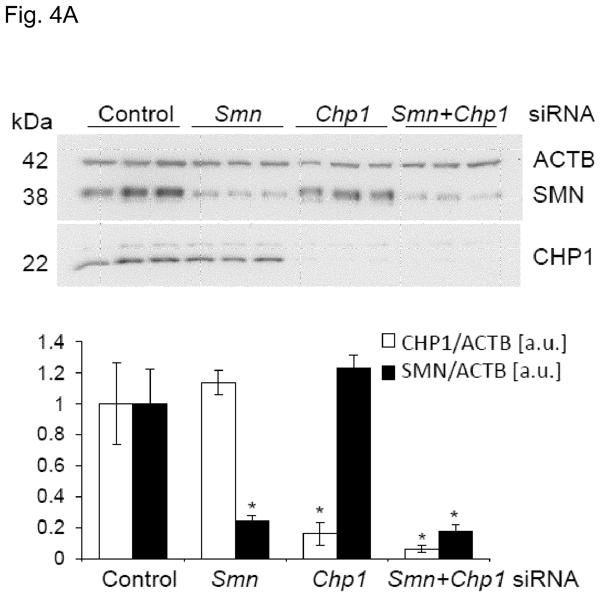

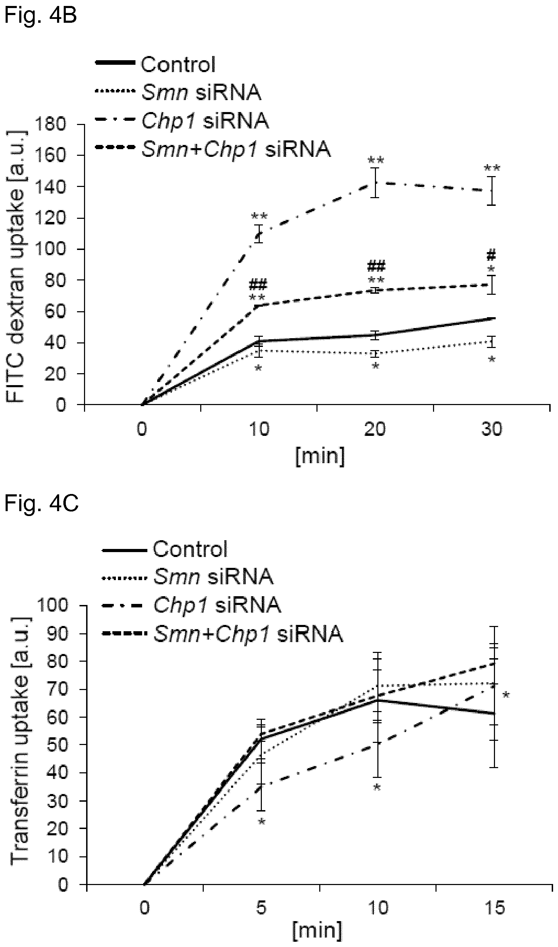

**Figure 4****:** CHP1 reduction rescues impaired FITC-dextran endocytosis in *Smn*-depleted motor neuron like (NSC34) cells.
   A.) Western blot and quantification of siRNA-mediated knockdown of *Chp1* and *Smn* in NSC34 cells. 48 h post transfection, Chp1 and Smn are significantly reduced (ACTB: loading control). *p ≤ 0.05, two-tailed Student's t test. Error bars indicate SEM.
   B.) Knockdown of CHP1 highly improves FITC-dextran uptake and restores impaired FITC-dextran uptake in Smn deficient cells. NSC34 cells were starved 48 h post transfection and incubated for 10, 20 and 30 min with FITC- dextran. FITC-dextran uptake was measured by FACS.
   C.) Clathrin-mediated endocytosis is not changed by *Smn* knockdown. Clathrin mediated endocytosis was analysed by measuring Transferrin uptake. NSC34 cells depleted for CHP1 and SMN were incubated for 5, 10 and 15 min with Transferrin and measured by FACS. Three independent experiments were performed for FACS analysis with 20,000 cells measured per experiment. * indicates tested against control treated cells and # tested against *Smn*-depleted cells, *p ≤ 0.05; ** p ≤ 0.01, *** p ≤ 0.001, two-tailed Student's t test. Error bars represent SEM.

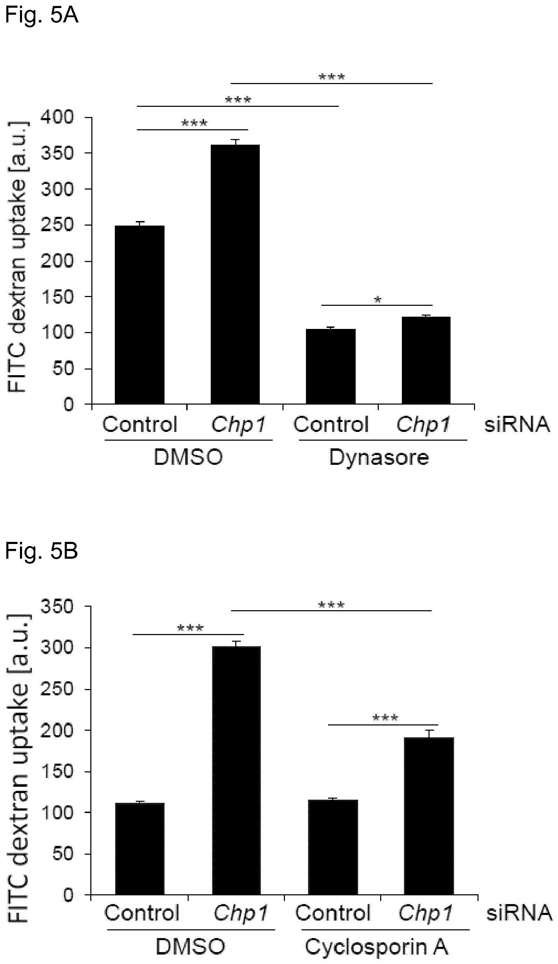

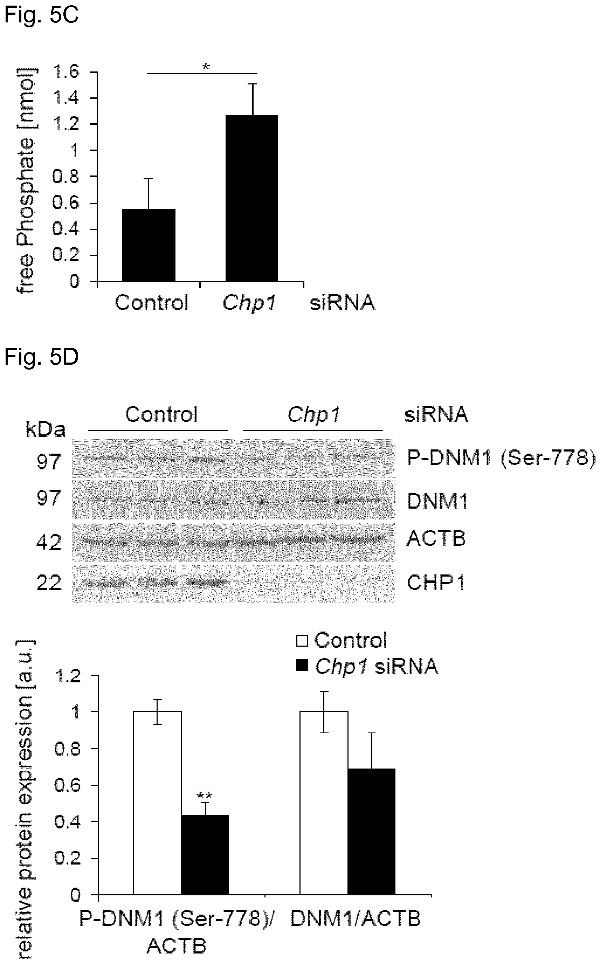

**Figure 5****:** Chp1 knockdown increases CaN phosphatase activity and thereby regulates endocytosis.
   A.) *Chp1* KD-mediated increase in FITC-dextran uptake is dynamin dependent. Inhibition of dynamin, by Dynasore treatment, abolished the increasing effect of *Chp1* KD on FITC-dextran endocytosis. NSC34 cells were treated for 20 min with 80 µM Dynasore. Endocytosis was measured 20 min post FITC-dextran incubation by FACS. N=3, *p ≤ 0.05; ** p ≤ 0.01, *** p ≤ 0.001, two-tailed Student's t test. Error bars represent SEM.
   B.) Inhibition of CaN phosphatase with Cyclosporine A, reduced the FITC-dextran uptake upon *Chp1* KD. NSC34 cells were treated for 20 min with 10 µM Cyclosporine A and FITC-dextran uptake was measured 20 min post incubation by FACS. Three independent experiments were performed for FACS analysis with 20,000 cells measured per experiment. *p ≤ 0.05; ** p ≤ 0.01, *** p ≤ 0.001, two-tailed Student's t test. Error bars represent SEM.
   C.) *Chp1* KD increases CaN phosphatase activity. 48 h post siRNA transfection, cellular CaN phosphatase activity was investigated by measuring the phosphorylation of the RII phosphopeptide, a highly specific substrate of CaN (n = 6). *p ≤ 0.05. Error bars represent SEM.
   D.) Western blot and quantification shows decreased dynamin 1 (DNM1) phosphorylation in NSC34 cells depleted of CHP1. Phosphorylation of DNM1 was investigated by measuring the phosphorylation of Ser-778. Total DNM1 expression
   is not changed. ACTB was used for loading control. **p ≤ 0.01, two-tailed Student's t test. Error bars represent SEM.

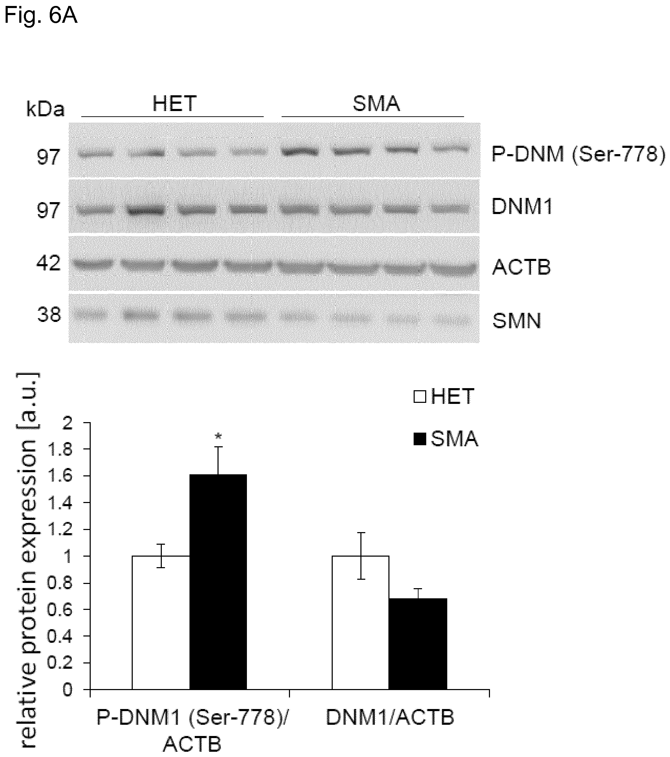

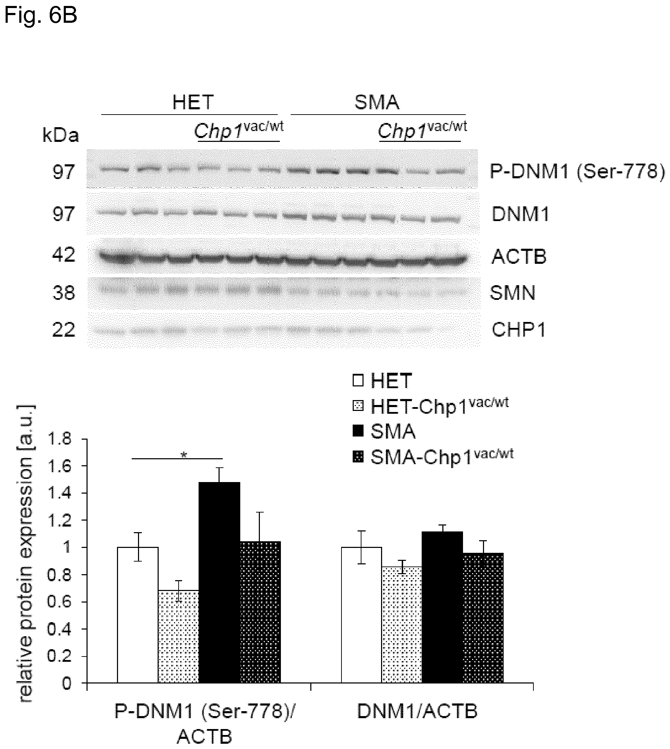

**Figure 6****:** Increased phospho-DNM1 in SMA MN is reduced by reduction of CHP1 in *SMA-Chp1*^{*vac*/*wt*}MN*.*
   A.) Western blot and quantification shows increased dynamin 1 (DNM1) phosphorylation in SMA MN at DIV7. Phosphorylation of DNM1 was investigated by measuring the phosphorylation of Ser-778. Total DNM1 expression is not changed. ACTB was used for loading control. *p ≤ 0.05, two-tailed Student's t test. Error bars represent SEM.
   B.) Western blot and quantification shows decreased dynamin 1 (dim1) phosphorylation in HET- *Chp1*^{*vac*/*wt*} MN at DIV7. Increased phospho-DNM1 in SMA is reduced in SMA-*Chp1*^{*vac*/*wt*} MN. Total DNM1 expression is not changed. ACTB was used for loading control. *p ≤ 0.05, two-tailed Student's t test. Error bars represent SEM.

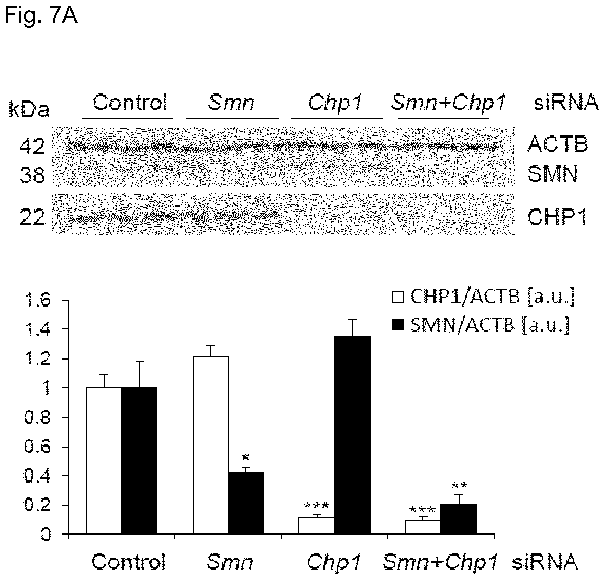

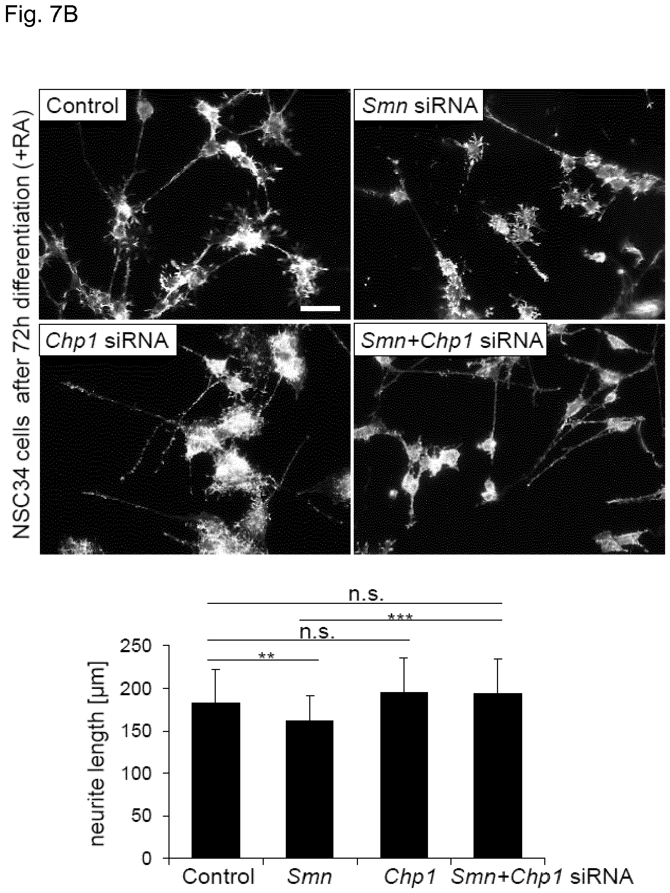

**Figure 7****:** CHP1 reduction rescues neurite outgrowth in *Smn*-depleted NSC34 cells.
   A.) Western blot and quantification of siRNA-mediated knockdown of *Chp1* and *Smn* in NS34 cells. 72 h post transfection and retinoic acid (RA) induced differentiation, CHP1 and SMN are significantly reduced (ACTB: loading control). *p ≤ 0.05; ** p ≤ 0.01, *** p ≤ 0.001, two-tailed Student's t test. Error bars represent SEM.
   B.) The reduced neurite length in *Smn*-depleted NSC34 cells was increased to wildtype length by *Chp1* KD. After siRNA transfection, NSC34 cell differentiation was stimulated for 72h with 1 µM retinoic acid treatment and neurite length was quantified with ZEN software (Zeiss) (N = 3, n=110). F-actin was stained with phalloidin (scale 50 µm). *p ≤ 0.05; ** p ≤ 0.01, *** p ≤ 0.001, two-tailed Student's t test, n.s. = not significant. Error bars represent SEM.

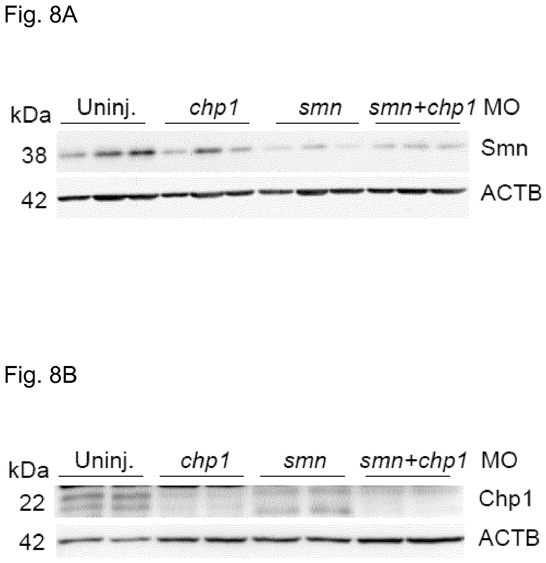

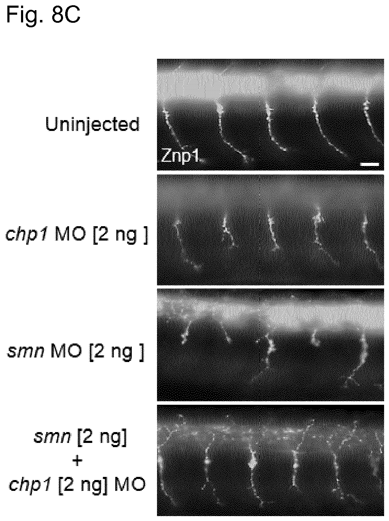

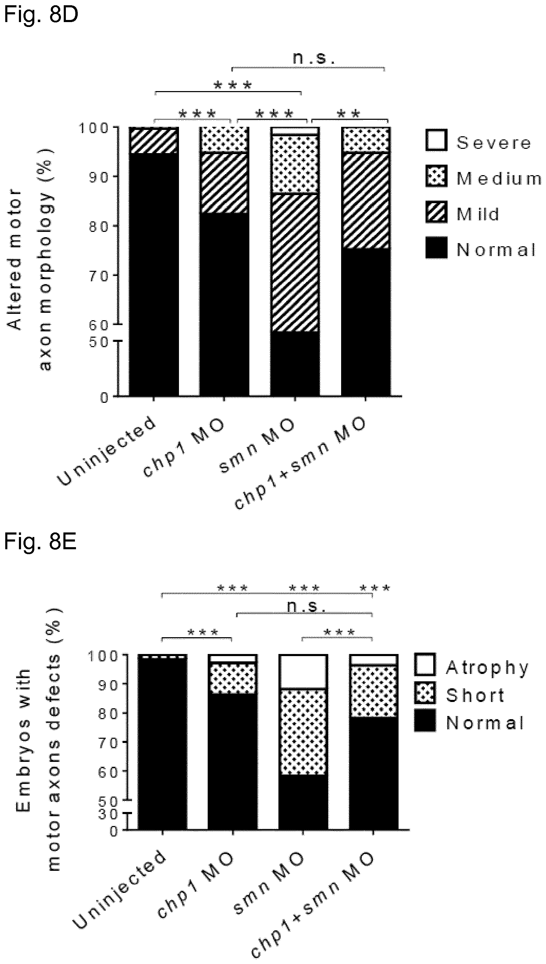

**Figure 8****:** *chp1* knockdown ameliorates the motor neuron phenotype in SMN depleted fish
   A.) and B.) Western blots show the morpholino-mediated knockdown of *smn* (A) and *chp1* (B). Zebrafish embryos were injected at 1-to-4 cell stage with 2 ng *chp1* and *smn* MO and further analysed 34 hpf. ACTB was used for loading control.
   C.) Lateral views of whole-mount embryos labelled with znp1 antibody. Five caudal primary motor axons posterior to the yolk sac were selected, for every condition, as representative pictures (scale bar: 25 µm). Motor axons of *smn* depleted fish show severe truncations (absent or shortened axons) and branching phenotypes (mild, medium and severe), which are ameliorated upon co-injection with the *chp1* MO.
   D.) Quantitative analysis of CaP MN demonstrates that co-injection of *smn* and *chp1* MOs ameliorates increased terminal branching. Results are presented in percentages from n>150 analysed axons, representing three independent experiments. ** p≤ 0.01, *** p≤ 0.001, Chi-square test.
   E.) Quantitative analysis of CaP MN demonstrates that co-injection of *smn* and *chp1* MO ameliorates axonal truncations (absent or shortened axons). Results are presented in percentages from n>150 analysed axons, representing three independent experiments. *** p≤ 0.001, Chi-square test.

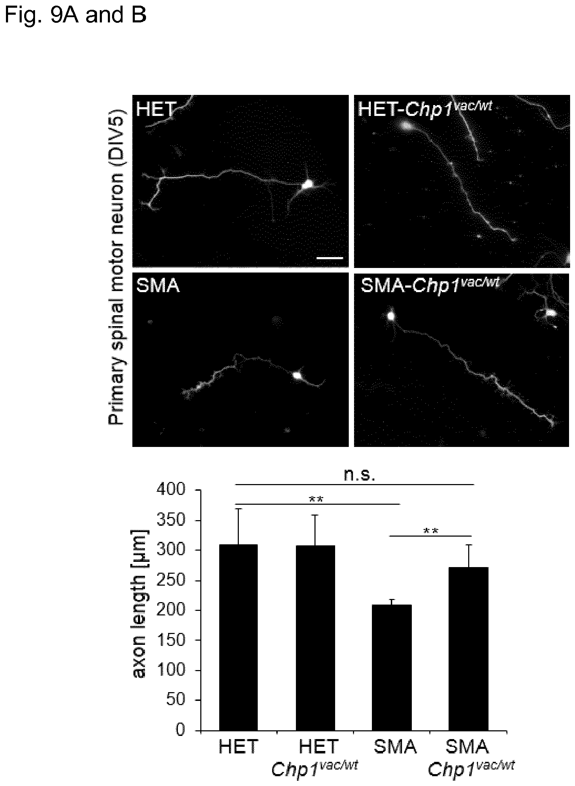

**Figure 9****:** CHP1 reduction rescues axonal outgrowth in SMA mice.
   A.) Representative merge image of DIV5 MNs isolated from E12.5-13.5 embryos. Stained with Tau (green, for axon), HB9 (red, for MN) and Hoechst (blue, for DNA) (scale bar: 50 µm).
   B.) Quantification of MN length with ZEN software (Zeiss) (N = 3-7, n = 20-50 axons per embryo). CHP1 reduction (*Chp1*^{*vac*/*wt*}) has no impact on axonal outgrowth on HET background. In contrast, MNs derived from SMA mice show significantly shorter axon length, which however was restored to HET levels by CHP1 reduction (SMA-*Chp1*^{*vac*/*wt*}). *p ≤ 0.05; ** p ≤ 0.01, *** p ≤ 0.001, two-tailed Student's t test, n.s. = not significant. Error bars represent SEM.

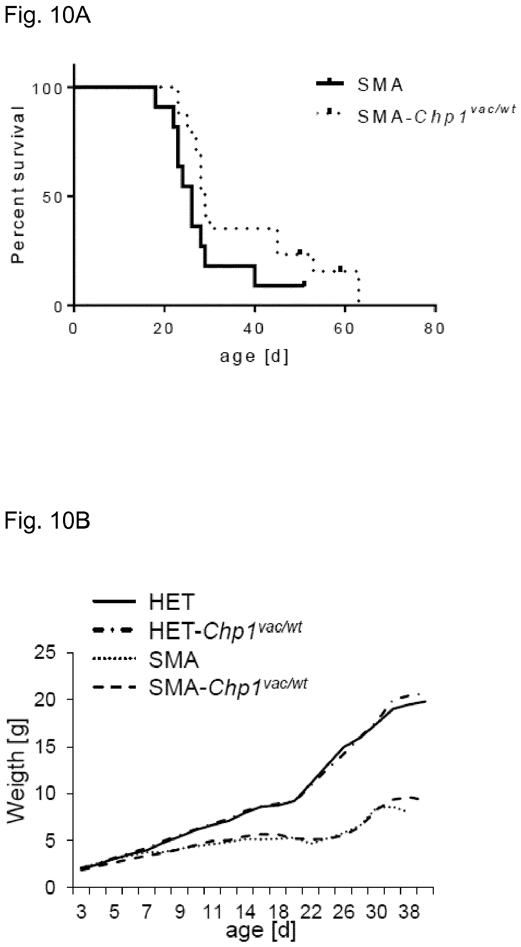

**Figure 10****:** CHP1 reduction improves the survival in an intermediate SMA mouse model.
   A.) Kaplan-Meier curve shows the survival of SMA and SMA-*Chp1*^{*vac*/*wt*} mice injected at P2 and P3 with low dose SMN-ASOs (30 µg). SMA mice live 28 ± 9 days and SMA-*Chp1*^{*vac*/*wt*} live 36 ± 13 days (SMA: n = 11, SMA-*Chp1*^{*vac*/*wt*}: n = 17). p= 0.0735, log-rank (Mantel-Cox) test.
   B.) Weight progression of SMA and SMA-*Chp1*^{*vac*/*wt*} mice in comparison to HET and *HET-Chp1*^{*vac*/*wt*}mice. No significant difference in weight progression was observed between SMA and *SMA*-*Chp1*^{*vac*/*wt*}mice.

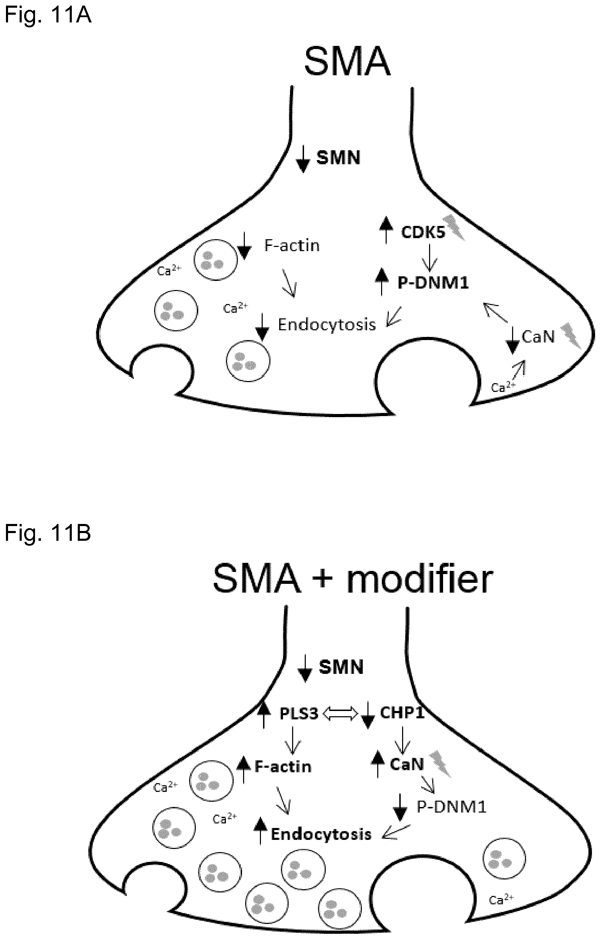

**Figure 11****:** Working model: CHP1 downregulation improves endocytosis and axonal outgrowth by increasing CaN phosphatase activity.
   A.) In SMA, decreased F-actin levels impair endocytosis. Furthermore, decreased Ca²⁺ influx, leading to decreased CaN activation, as well as increased CDK5 activity lead to increased P-DNM1, blocking its interaction with syndapin and thereby inhibiting activity dependent bulk endocytosis (ADBE).
   B.) In SMA with modifiers including PLS3 overexpression and CHP1 reduction the impaired endocytosis is rescued. PLS3 overexpression increases F-actin levels, thereby increasing endocytosis. CHP1 suppression increases CaN activity, leading to increased DNM1 dephosphorylation, allowing DNM1-syndapin interaction, which finally triggers ADBE. Thereby, increased CaN activity compensates for reduced Ca²⁺ influx and increased CDK5 activity in SMA.

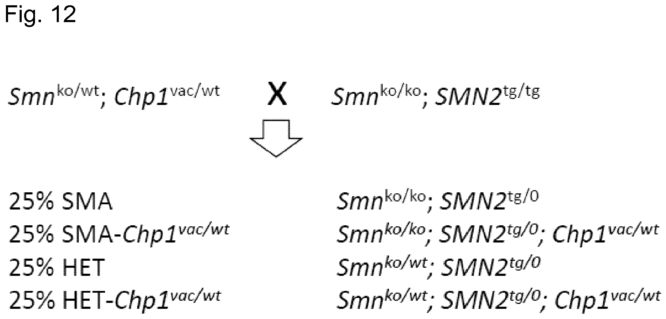

**Figure 12****.)** Breeding scheme to produce SMA, SMA-*Chp1*^{*vac*/*wt*} and HET and HET-*Chp1*^{*vac*/*wt*} mice. All offspring were injected with 30 µg SMN-ASO at P2 and P3.

### Example 1

### MATERIAL AND METHODS

### Yeast-two-hybrid screen

To identify PLS3 interacting partners a yeast-two-hybrid screen was carried out. Full length human PLS3 cDNA (NM 005032.6; 1-630aa) was cloned into the PBTG9 vector using *EcoR1* and *BamH1* restriction sites (Figure 1A). Sequences of the vectors were confirmed and the interaction of full length PLS3 and its deletion constructs were investigated using a brain prey library (adult brain cDNA, Clontech Company; the screen was performed by Dr. Frank Schwarz, German Cancer Research Center, Heidelberg).

### Co-immunoprecipitation (Co-IP) and pull down assay

For Co-IP experiments, human CHP1 cDNA (NM 007236.4) was inserted into pcDNA3.1/CT-GFP TOPO vector using manufacturer's protocol (Invitrogen). Human PLS3 cDNA (NM 005032.6) was cloned into a Flag/His pcDNA6 vector. The pcDNA3.1/CT-GFP-TOPO vector (Invitrogen) was used as a negative control. HEK293T cells were transiently co-transfected with Lipofectamine 2000 (Invitrogen) according to manufacturer's protocol and harvested 48h post transfection. HEK293T cells were lysed for 20 min on ice in IP lysis buffer (100 mM NaCl, 50 mM Tris, 5 mM EGTA, 1% CHAPS, pH 7.5, containing protease inhibitors (Complete Mini, Roche)), sonicated for 10 min and centrifuged for 20 min at 13.000 g at 4°C. Lysates were incubated for 2 h at 4°C with GFP MicroBeads (Miltenyi Biotec). Next, the cell lysates were added to the µ-columns (Miltenyi Biotec), washed and eluted with laemmli buffer.

For Pull down assays, hCHP1 was cloned in a GST tagged pGEX-4T-3 and hPLS3 in a His tagged PET30BZ vector using the restriction sites *MluI* and *NotI.* The production of the recombinant proteins was induced in previously transformed *E.coli* (strain BL-21 (DE3)) with 1 mM isopropyl-b-D-thiogalactosid (IPTG) for 4 h at 25°C. The tagged protein were purified as described (Crowe et al., 1995; Smith and Johnson, 1988). Protein concentration was measured via Bradford protein assy. GST-CHP1 was bound to the Glutathione HiCap matrix (QIAGEN) and incubated for 2 h at 4°C with PLS3-His in pull down buffer (100 mM NaCl, 50 mM Tris, 5 mM EGTA, pH 7.5). Finally, after resin washing (wash buffer: 250 mM NaCl, 50 mM Tris, 5 mM EGTA, pH 7.5) the resin-bounded proteins were eluted in laemmli buffer. Eluates were analysed by Western blot.

### Western Blot analysis

Cells were lysed on ice in RIPA buffer (Sigma) containing protease inhibitors. Tissue samples were homogenized in RIPA buffer containing protease inhibitors using the Precellys24 device (Peqlab). For immunoblotting the following primary antibodies were used: anti-SMN (610647, BD Biosciences), anti-CHP1 (PA5-29876, ThermoFisher), anti-PLS3 (1238, Eurogentec (Oprea et al., 2008)), anti-GFP (produced in hybridoma cell line), anti-GST (34860, QIAGEN), anti-DYN1 (ab3456, Abcam), anti-DYN1-SER778 (NB300-210 Novus Biologicals), anti-ACTB (A5316, Sigma), anti-GAPDH (G9295, Sigma) and anti-HSP90 (4877, Cell Signalling). Signal was detected with HRP conjugated-secondary antibodies (anti-mouse (115035000 Dianova), anti-rabbit (7074, Cell Signalling) and anti-sheep (ab97130, Abcam)) and Chemiluminescence reagent (ThermoFisher) according to manufacturer's protocol.

### Immunohistochemistry

For immunofluorescent staining cells were washed with PBS, fixed in 4% PFA/4% sucrose (Sigma), permeabilized in PBS-T PBS/0.2%Tween20 (AppliChem) and blocked in blocking solution (PBS-T/5%BSA (Sigma)/5% FCS (Biochrom)). Cells were incubated overnight at 4°C with the primary antibodies in blocking solution. Following primary antibodies were used: anti-HB9 (AB2145209, Hybridoma Bank), anti-Tau (sc-390476, Santa Cruz), anti-CHAT (AB144P, Millipore) and anti-V5-HRP (R96125, Invitrogen). Next, Cells were washed and incubated with secondary antibodies and/or phalloidin-AlexaFluor568 (Invitrogen). Secondary antibodies were labelled with AlexaFluor350, AlexaFluor488, AlexaFluor647, or AlexaFluor568 (Invitrogen). The nucleus was stained with Hoechst staining solution (Invitrogen). After washing, the coverslips were mounted with Mowiol (Sigma). Cells were imaged using a fluorescence microscope (equipped with an ApoTome (AxioImager M2, Zeiss).

For NMJ staining the TVA was fixed for 20 min in 4% PFA and washed with PBS. From that step on the staining was proceeded as described above. The following primary antibodies were used anti-SV2 (AB2315387, Hybridoma Bank) and anti-NF-M (2H3, Hybridoma Bank). Bungarotoxin-AlexaFluor555 (Invitrogen) was used for endplate labelling.

### siRNA-mediated RNA knockdown (KD)

For all siRNA experiments NSC34 (CLU140) cells were transfected with Dharmafect1 (ThermoFisher) according to manufacturer's protocol. All siRNAs were purchased from QIAGEN. siRNAs sequences: mmu-*Smn*: 5'-AAGAAGGAAAGTGCTCACATA-3'; mmu-*Chp1* 5'- TTCTTGCTTTCTAGTATTTAA -3'. AllStars Negative Control siRNA were used as controls. 48-72h post transfection, cells were harvested for protein isolation, FACS sorting or imaging. All experiments were performed in triplicates. Differentiation was induced 6 h post transfection with 1 µM retinoic acid.

### Calcineurin cellular activity assay

Cellular CaN phosphatase activity was measured using a complete colorimetric assay (Enzo). The assay was performed according to manufacturer's protocol.

### Endocytosis Assay

NSC34 cells transfected with the respective siRNAs were starved for 4 h by serum depletion. Cells were incubated with 5mg/ml fluorescein isothiocyanate (FITC)-dextran (46945, Sigma) or 5 µg/ml Transferrin (T13342, Invitrogen) for respective time periods at 37°C. Subsequently cells were trypsinized (Trypsin, Sigma) on ice and washed with PBS containing 1% BSA. Fluorescence intensity was measured with FACS Calibur (BD Biosciences) and analyzed with Cyflogic software (CyFlo Ltd.). Dead cells were excluded by propidium iodide staining (10 mg/mL, AppliChem).

In order to inhibit proteins involved in endocytosis, cells were treated with specific inhibitors. Prior to FITC-dextran incubation cells were treated for 30 min with 80 µM Dynasore (Abcam) or 10 µM Cyclosporin A (Sigma) and subsequently incubated for 20 min with FITC-dextran and the respective inhibitor.

### Zebrafish experiments

Experiments for the analysis of Caudal Primary Motor Neurons (CaP MN) were performed with the transgenic line *tg(mnx1-GFP)^{ml2TG}* (Synonym TL/EK-*hb9-GFP* (Flanagan-Steet et al., 2005)). All procedures were approved by the local animal protection committee LANUV NRW (reference number 84-02.04.2012.A251).

### Zebrafish injection and motor axon analysis

*Smn* and *chp1* morpholinos were designed and purchased from GeneTools, LLC. The sequences are: 5'-CGACATCTTCTGCACCATTGGC-'3 and 5'-CTGGAGCCCATG-ACTGCTGAAGATC-'3, respectively.

For visualization of CaP MN, embryos derived from TL/EK wild-type and TL/EK-hb9-GFP lines crossings were used. Embryos at 1-to-4-cell-stage were injected with 2 ng of *chp1* or *smn* MO separately or in combination. After injection, embryos were reared in petri dishes and kept in a 28°C incubator until further processing. For immunostaining of MN, -34 hpf zebrafish were dechorionized, fixed in 4%, permeabilized with 10µg/ml of proteinase K and blocked in in PBS-T (+0.1% Tween20), 1% DMSO (Sigma), 2% BSA and 5% FCS. To visualize motor axons, fish were incubated with anti-znp1 (Hybridoma Bank) followed by secondary α-AlexaFluor488 (Invitrogen). For imaging, fish were embedded in low-melting agarose slides, and the first 10 motor axons after the yolk sac were considered for quantification. Based on overall appearance MN were classified as: normal, short (truncated axonal projection) or atrophy (absent axonal projection). Based on terminal branching, axons were classified as normal, mild (branching ventral from midline), medium (2-3 or more branches at ventral or midline) or Severe (>3 branches ventral or dorsal from midline).

### Western blot analysis of zebrafish

Protein lysates from zebrafish were prepared following the protocol described in Riessland et al, 2017. After ~32hpf, -20 fish larvae were manually dechorionized, gently spun down and lysed in RIPA buffer containing protease inhibitors. Supernatants were collected and processed for WB according to standard protocols. Following antibodies were used: anti-CHP1 (PA5-29876, ThermoFisher), anti-ACTB (zebrafish reactivity, Anaspec - Eurogentec group) and anti-SMN (610647, BD Biosciences). Samples were processed for WB following standard procedures.

### Mouse experiments

All mouse experiments were approved by LANUV NRW (reference number 84-02.04.2014.A242). The Taiwanese SMA mice (FVB.Cg-Tg(SMN2)2Hung Smn1tm1Hung/J, Stock Number 005058) were purchased from Jackson Laboratory. The *vacillator* mice (B6.Cg-CHP1vac) were provided by Susan L. Ackerman, Howard Hughes Medical Institute, The Jackson Laboratory, Bar Harbor, Maine 04609. All mouse lines were backcrossed for at least seven generations to obtain a congenic C57BL/6N background. The severe SMA (*Smn*^{*ko*/}*^{ko}; SMN2*^{*tg*/*0*}) mice and the corresponding heterozygous *Smn* (HET [*Smn*^{*ko*/}*^{wt}; SMN*^{*2tg*/*0*}]) mice were produced as previously described (Hsieh-Li et al., 2000; Riessland et al., 2010).

The breeding colony was maintained as previously described (Riessland et al., 2010), by crossing *Smn*^{*ko*/}*^{ko}; SMN2*^{*tg*/*tg*} mice and *Smn*^{*ko*/*wt*} mice with WT mice. Heterozygous *Chp1*^{*vac*/*wt*} mice were crossed with *Smn*^{*ko*/*wt*} mice to obtain *Smn*^{*ko*/}*^{wt}; Chp1*^{*vac*/*wt*} mice. To obtain all required genotypes within one breeding, we used the breeding scheme depicted in Figure 12. Genotyping for *Smn, SMN2* and *Chp1* was performed as described (Ackermann et al., 2013; Liu et al., 2013).

An intermediate SMA mouse model was generated by suboptimal subcutaneous injection of severe SMA (*Smn*^{*ko*/}*^{ko}; SMN2*^{*tg*/*0*}) mice as previously described (Hosseinibarkooie et al., 2016). Subcutaneous injections of 30 µg SMN-ASO (IONIS Pharmaceuticals) at P2 and P3, respectively, were carried out using a MICROLITER syringe (Hamilton). All pups of each litter were double blindly injected.

### Primary motor neuron (MN) culture

Primary MN were cultured as described (Riessland et al., 2017). Spinal cords were dissected from E12.5-13.5 mouse embryos. Neurons were singularized with trypsin (Worthington) and DNase (Sigma), sieved and plated on poly-D-lysine/laminin (Sigma, Invitrogen) coated coverslips. MN were cultured in MN culture medium composed of Neurobasal medium with B27 supplement, 2 mM L-glutamine and PenStrep (10 U/ml), Amphotericin B (250 µg/ml) (Invitrogen), substituted with 2% Horse serum and 50 ng/mL BDNF, 50 ng/mL GCNF, and 50 ng/mL CNTF (Peprotech) at 37°C in a humidified incubator with 5% CO₂.

### Primary murine embryonic fibroblast (MEF) culture

Primary murine fibroblasts were prepared from E12.5-13.5 animals as previously described (Ackermann et al., 2013). Fibroblasts were isolated from *Smn*^{*ko*/}*^{wt}; PLS3V5*^{*tg*/*tg*} embryos.

### RESULTS

### CHP1 and PLS3 are directly interacting

To identify PLS3 interaction partners, we used the yeast two-hybrid system, using PLS3 full-length as a bait (Figure 1A). Thereby, we identified Calcineurin B Homologous Protein 1 (CHP1) as a potential interaction partner of PLS3 (Figure 1B). Sequencing results and subsequent alignment indicate that the C-terminal part of CHP1 is interacting with the PLS3 full-length bait. The other candidates including forkhead box K1 (FoxK1), TatD DNase domain containing 1 (TATDN1) and TIAL1 (Nucleolysin TIAR) were considered to be artefacts, since they contained untranslated 3'UTR sequences, were unspecifically abundant in several yeast two-hybrid screens or contained in addition to exonic also intronic regions, respectively. Thus, CHP1 was the only promising interacting partner of PLS3 found by yeast-two-hybrid screen. To further confirm this interaction, we conducted detailed interaction studies of PLS3 and CHP1. HEK293T cells were co-transfected with CHP1-GFP and PLS3-His overexpressing plasmids and co-immunoprecipitation studies were performed. Thereby, we proved that CHP1 and PLS3 are co-precipitating (Figure 2A). Since the interaction between CHP1 and PLS3 could also be indirect, mediated by a common interaction partner, we performed direct interaction studies. Pull-down assays revealed that GST-CHP1 and PLS3-His are directly interacting (Figure 2B). Moreover, immunohistological analysis of primary murine embryonic fibroblasts (MEFs) isolated from PLS3V5 overexpressing E13.5 embryos showed that CHP1 and PLS3 strongly co-localize in the filopodia (Figure 2C). In congruence, the calculated correlation coefficient indicated a very high linear correlation between PLS3 and CHP1 (Costes-adjusted Pearson coefficient 0.94). Together, these results confirm that CHP1 and PLS3 interact both *in vitro* and *in vivo.*

### CHP1 is particular highly expressed in neuronal tissue

Next, we analysed the expression of CHP1 protein in various tissues derived from wildtype P10 mice. CHP1 protein was expressed in all tested tissues (Figure 3A), in accordance with previous studies (reviewed in (Di Sole et al., 2012)). CHP1 was found to be particularly abundant in neuronal tissue, namely: hippocampus, cortex, cerebellum and spinal cord. Since SMA is a neurodegenerative disease, we are highly interested in the interface between neuronal and muscular systems. Therefore, we performed additional expression studies of neuronal brain tissue. Indeed, we observed a high expression of CHP1 in brains of SMA *(Smn*^{*ko*/}*^{ko}; SMN2*^{*tg*/*0*}) mice and asymptomatic heterozygous litter mates, referred to as HET *(Smn*^{*ko*/}*^{ko}; SMN2*^{*tg*/*0*}) mice (Figure 3B). In addition, we observed a consistent tendency towards increased CHP1 expression in neuronal tissue of SMA mice, nevertheless statistical significance was not reached. In order to investigate the expression of CHP1 in neuromuscular tissue, we performed immunhistological analysis of the *transverses abdominalis* (TVA) muscle, one of the most affected muscles in SMA (Murray et al., 2008). A strong expression of CHP1 along the axon of the neuron, as well as in the pre-synapse (Figure 3C) was found. Finally, the expression pattern of CHP1 in primary motor neurons (MN) was analysed (Figure 3D). CHP1 staining showed a broad expression throughout the MN soma. Moreover, CHP1 was highly abundant in the axon and the growth cone of MNs (Figure 3D, magnification).

Thus, we confirmed the ubiquitous expression of CHP1, as well as showed that CHP1 is not only abundantly expressed in neuronal tissues but also at SMA relevant sites including the presynaptic site at NMJs as well as MN soma and growth cone.

### CHP1 downregulation rescues impaired endocytosis in SMA

In recent studies, we reported that SMA motor neuron-like (NSC34) cells not only show a reduced calcium influx, but also an impaired endocytosis (Hosseinibarkooie et al., 2016; Riessland et al., 2017). Interestingly, KD of sac6 the SMA modifier PLS3 orthologue in yeast, causes massive impairment of endocytosis (Kubler and Riezman, 1993). Consistent with that, PLS3 overexpression in MEFs derived from SMA embryos rescues the impaired endocytosis (Hosseinibarkooie et al., 2016). Since CHP1 interacts with CaN, an important Ca²⁺/calmodulin-dependent phosphatase, and overexpression of CHP1 decreases CaN activity to 50% (Lin et al., 1999), we hypothesized that CHP1 may be involved in endocytosis via modulation of CaN activity.

To ascertain the impact of *Chp1* knockdown (KD) on endocytosis and its effect on this process in *Smn*-depleted cells, we conducted knockdown studies followed by endocytosis assays. Specific endocytosis assay for fluid phase (FITC-dextran uptake) and clathrin-dependent receptor-mediated endocytosis (Transferrin uptake) (Le Roy and Wrana, 2005) were carried out. Western blot analysis confirmed knockdown *of Smn* and *Chp1* after 48h of transfection (Figure 4A). *Smn*-depleted NSC34 cells showed a significantly decreased FITC-dextran uptake, whereas *Chp1* KD results in a 2.5 fold increase of FITC-dextran endocytosis compared to cells treated with control siRNA. Most interestingly, combined KD *of Smn* and *Chp1* restored the impaired dextran endocytic uptake in *Smn*-depleted cells (Figure 4B). In contrast, *Smn*-depleted cells showed no difference in clathrin-mediated endocytosis compared to control cells (Figure 4C). It is remarkable that upon *Chp1* KD the velocity of Transferrin uptake is significantly decreased, whereas the total Transferrin endocytosis after 15 min is not different in comparison to control cells (Figure 4C). This phenomenon might be explained by a compensatory mechanism counteracting the high fluid phase endocytosis. Taken together, in *Smn*-depleted cells the clathrin independent fluid phase endocytosis is most prominently impaired and can be restored by *Chp1* KD. However, we could not observe any major impairment of the clathrin-dependent endocytosis in *Smn*-depleted cells.

In summary, we show that CHP1 downregulation dramatically enhances fluid phase endocytosis as well as rescues the impaired fluid phase endocytosis in SMA.

### CHP1 influences endocytosis by regulating CaN activity

In order to elucidate the mechanism behind the increased endocytosis upon *Chp1* KD, we treated CHP1-depleted cells with inhibitors for major players involved in different types of endocytosis. When treating cells with Dynasore (Abcam) - a dynamin inhibitor - we observed a marked reduction of endocytosis in control cells as well as in *Chp1* KD cells (Figure 5A); suggesting that the increased endocytosis in *Chp1*-depleted cells is dynamin dependent. Since CHP1 was reported to regulate CaN and CaN has a well characterized role in endocytosis (Baumgartel and Mansuy, 2012), we also investigated if the impact of CHP1 on endocytosis is dependent on CaN activity modulation. Therefore, we treated cells with the CaN inhibitor Cyclosporin A (Liu et al., 1991; Liu, 2003), to analyse if this treatment inhibits the increased endocytosis in *Chp1* KD cells. Indeed, we observed a diminished increase in endocytosis upon *Chp1* KD when treating the cells with Cyclosporin A (Figure 5B). We therefore speculated that CHP1 might act upstream of CaN. To further prove this hypothesis, we performed a specific cellular CaN phosphatase activity assay in *Chp1* KD cells. Hereby, the phosphorylation of the RII phosphopeptide - the most specific substrate of CaN activity- is detected (Roberts et al., 2008). Importantly, *Chp1* KD led to increased free phosphate, corroborating increased CaN phosphatase activity (Figure 5C). In line with this observation, we detected decreased levels of phospho-dynamin 1 P-DMN1 in *Chp1* depleted cells (Figure 5D), proving that CHP1 downregulation results in increased CaN phosphatase activity. Dynamin 1 (DNM1) is specifically dephosphorylated by CaN (Liu et al., 1994) and can therefore be used as an indicator for CaN activity. Moreover, we showed a robust increase of DMN1 phosphorylation in MN derived from E13.5 SMA embryos (Figure 6A). DNM1 hyper-phosphorylation can be explained by increased cyclin dependent kinase 5 (CDK5) activity in SMA (Miller et al., 2015). In congruence with our observations in NSC34 cells (Figure 5D), CHP1 reduction in HET*-Chp1*^{*vac*/*wt*} MN results in decreased phospho-DNM1 (Figure 6B). Additionally, downregulation of CHP1 rescued DMN1 hyper-phosphorylation in SMA MN (Figure 6B).

Taken together, these results indicate that the increased endocytosis in *Chp1* KD cells underlies increased CaN phosphatase activity and is dynamin-dependent. Moreover, we showed increased dynamin phosphorylation in SMA MN, which is significantly reduced upon CHP1 downregulation.

### CHP1 reduction improves axonal outgrowth in cell culture and in zebrafish and mouse SMA models

The SMA phenotype is mainly characterized by the degeneration of *α*-motor neurons in the anterior horns of the spinal cord, leading to progressive denervation and atrophy of the skeletal muscles. In previous studies in NSC34 motor neuron-like cells a reduced neurite length was reported upon *Smn* depletion (Nolle et al., 2011). Here, we wanted to investigate if *Chp1* KD can improve the aberrant neurite outgrowth in *Smn*-depleted cells. Therefore, we treated NSC34 cells for 72h with retinoic acid (RA) to induce differentiation and measured the neurite length for the different KD conditions. Western blot analysis confirmed a significant reduction *of Smn* and *Chp1* after 72 h (Figure 7A). Consistent with previous results (Nolle et al., 2011), a significant decreased neurite length in *Smn*-depleted cells was observed (Figure 7B). Interestingly, *Chp1* KD in *Smn* depleted cells rescued the decreased neurite length to normal length (Figure 7B), whereas, *Chp1* KD alone had no impact on neurite length.

Next, we wanted to investigate the modifying effect of CHP1 suppression *in vivo.* CHP1 is a highly conserved protein, whereby the human CHP1 and its orthologue in zebrafish are 90% identical. Hence, we performed a morpholino (MO)-mediated knockdown in zebrafish of either *smn* and *chpl,* or both together. Knockdown efficiency was confirmed by Western blot quantification (Figure 8A and B). In line with earlier studies, *smn* depletion resulted in motor-axon outgrowth defects and altered axon morphology (Figure 8D and E) (Oprea et al, 2008). Importantly, combined KD of *smn* and *chp1* not only significantly improved the altered motor axon phenotype (Figure 8D), but also reduced the number of atrophic motor axons (Figure 8E).

Finally, we wanted to investigate if CHP1 reduction does also rescue the decreased motor neuron length in motor neurons derived from SMA mice. Therefore, we crossbred the so called *vacillator* mouse model (which carries a missense mutation in the murine *Chp1* gene, creating a novel splice donor site which results in aberrant splicing and consequently in less CHP1 protein), with the Taiwanese SMA mouse strain (Hsieh-Li et al., 2000; Riessland et al., 2010). Primary motor neurons were isolated from E13.5 HET (*Smn*^{*ko*/}*^{wt}; SMN2*^{*tg*/*0*}), SMA *(Smn*^{*ko*/}*^{ko}; SMN2*^{*tg*/}*⁰),* HET*-Chp1*^{*vac*/*wt*} and SMA-*Chp1*^{*vac*/*wt*} embryos and cultured for 5 days. As previously shown, primary MN derived from SMA animal were significantly shorter than MN derived from HET embryos (Figure 9). Most importantly, downregulation of CHP1 restored the reduced motor axon length in SMA-*Chp1*^{*vac*/*wt*} MN compared to SMA MN (Figure 9). Consistent with our previous findings in NSC34 cells (Figure 7B), CHP1 reduction in MN from HET-*Chp1*^{*vac*/*wt*} embryos did not result in longer motor axons (Figure 9).

Collectively, these results show that *Chp1* KD rescues the impaired motor axon outgrowth in SMA cells, fish and mice, further suggesting that CHP1 is a cross-species SMA modifier.

### Chp1 reduction improves survival in an intermediate SMA mouse model

Our previous studies have shown that genetic protective modifiers (PLS3 and NCALD) are unable to rescue a severe SMA-phenotype in mice, however it can be rescued in an intermediate or mild SMA-phenotype (Ackermann et al., 2013; Hosseinibarkooie et al., 2016; Riessland et al., 2017). This is in line with the asymptomatic *SMN1*-deleted individuals, who carry 3-4 *SMN2* copies, a genotype otherwise associated with an intermediate or mild SMA phenotype. This suggests that the modifier acts positively only when a certain SMN amount is available, but fails if SMN falls under a certain threshold (Wirth et al., 2015). Consequently, we generated an intermediate SMA mouse model, which resembles SMA type II patients, by systemic subcutaneous injection of SMA, SMA-*Chp1*^{*vac*/*wt*} as well as HET, HET-*Chp1*^{*vac*/*wt*} with low dose (30 µg) of SMN-ASO (Spinraza^{™}) at P2 and P3 (Hosseinibarkooie et al., 2016). SMN-ASO treatment increased the amount of full-length functional SMN from the human SMN2 transgene in a dose-dependent manner (Hua et al., 2011). Remarkably, we observed an increased survival rate of SMA mice carrying a heterozygous mutation in the *Chp1* gene (*Chp1*^{*vac*/*wt*}) compared to SMA mice. Survival was extended from 28 ± 9 days in SMA mice to 36 ± 13 days in SMA-*Chp1*^{*vac*/*wt*} (Figure 10A). However, we could not observe any gross differences between the weight progression of SMA and SMA*-Chp1*^{*vac*/}*^{wt} mice* (Figure 10B).

Our results demonstrate that reduction of CHP1 in an intermediate SMA mouse model extends survival. Thereby, we conclude that CHP1 downregulation beneficially ameliorates the SMA phenotype.

### DISCUSSION

In summary our main findings are the following: (1) CHP1 is a direct interacting partner of the SMA modifier PLS3; (2) We confirmed the ubiquitous expression of CHP1, and its particular enrichment in neuronal tissues (reviewed in(Di Sole et al., 2012)). Importantly, CHP1 is highly expressed at SMA relevant sites including the presynaptic site at NMJs as well as MN soma and growth cone; (3) CHP1 reduction rescues the impaired bulk-endocytosis (clathrin-independent) in SMN deficient NSC34 cells; (4) Increased endocytosis upon CHP1-depletion is DNM1-dependent and CHP1 reduction results in increased CaN phosphatase activity and restored DNM1 hyper-phosphorylation in SMA (5) CHP1 deletion rescues reduced axonal outgrowth in diverse SMA models including SMN deficient NSC34 cells, zebrafish *smn* morphants and MNs derived from SMA embryos and (6) finally, we show that CHP1 downregulation extends the survival span of an SMN-ASO induced intermediate SMA mouse model, strengthening the view that a combinatorial use of moderate SMN upregulation - as happens in type I SMA patients - plus CHP1 downregulation ameliorates SMA symptoms.

In December 2016 the FDA-approved SPINRAZA^{™} - an ASO correcting *SMN2* splicing - as the first treatment for SMA. However, for type I SMA-affected individuals, carrying only two SMN2 copies, this pharmacological approach might not be sufficient to increase the SMN amount to adequate levels in order to suppress SMA symptoms (Riessland et al., 2017). Therefore, combinatorial treatment targeting SMN-dependent as well as SMN-independent pathways might be more effective (Wirth et al., 2015). Importantly, PLS3 and NCALD have been identified as the first SMN-independent modifiers of SMA (Oprea et al., 2008; Riessland et al., 2017). Recent studies demonstrate that stable overexpression of PLS3 as well as adeno-associated virus-mediated (AAV-mediated) PLS3 delivery extend survival span in an SMN-ASO induced intermediate SMA mouse model (Hosseinibarkooie et al., 2016; Kaifer et al., 2017). These studies highlight the potential utility of combinatorial therapeutics in SMA that target both SMN-dependent and SMN-independent pathways.

To elucidate the molecular mechanism behind the rescuing effect of PLS3 as well as to find new potential modifiers which might be utilized as therapeutic targets, we screened for PLS3 interaction partners. Hereby, we found CHP1 as a promising candidate that directly interacts with PLS3. Similar to NCALD (Riessland et al., 2017), suppression of CHP1 rescued the impaired endocytosis in SMN depleted cells. Since CHP1 was reported to be an endogenous inhibitor of CaN (Lin et al., 1999), we investigated the impact of CHP1 reduction on CaN activity. Indeed, a cellular CaN activity assay proved an increased CaN phosphatase activity in CHP1 depleted cells, which might be due to diminished CaN inhibition. In line with the latter assumption, we found decreased phospho-DNM1 - a high-affinity substrate for CaN phosphatase activity (Liu et al., 1994) - in CHP1 depleted cells compared to control treated cells, suggesting a higher CaN enzymatic activity. Furthermore, inhibition of CaN activity by Cyclosporin A diminished the effect of CHP1 reduction on endocytosis, thus corroborating that CHP1 acts by modulating CaN activity. Nevertheless, the precise mechanism by which CHP1 inhibits CaN remains to be elucidated (Liu, 2003). In other words, increased CaN activity underlies the gross endocytosis increase upon CHP1 downregulation.

CaN is a Ca²⁺-calmodulin dependent phosphatase involved in vesicle endocytosis in neuronal and non-neuronal secretory cells (Wu et al., 2014). During elevated neuronal activity, CaN is activated by Ca²⁺ influx and dephosphorylates dephosphins including DNM1 (Cousin and Robinson, 2001). DNM1 dephosphorization stimulates a specific DNM1-syndapin 1 interaction, which triggers dependent bulk endocytosis (ADBE) to massively increase synaptic vesicle endocytosis (Clayton et al., 2009). Finally, after scission of bulk endosomes DNM1 is re-phosphorylated by cyclin dependent kinase 5 (CDK5) (Evans and Cousin, 2007) and glycogen synthase kinase 3 (GSK3) (Smillie and Cousin, 2012). Interestingly, increased CDK5 activity was reported in both SMA mouse models and human SMA induced pluripotent stem cell-derived motor neurons (Miller et al., 2015). Thus, the aberrant endocytosis in SMA might not only be explained by decreased Ca²⁺ influx and disturbed F-actin levels (Hosseinibarkooie et al., 2016; Riessland et al., 2017), but also by increased CDK5 activity leading to DNM1 hyper-phosphorylation and inhibition of ADBE. Importantly, we demonstrated that CHP1 reduction in SMA MN rescues DNM1 hyper-phosphorylation. Additionally, the reduced Ca²⁺ influx in SMA, could also prevent a complete CaN activation and thereby decrease DNA1 dephosphorylation and ADBE in SMA.

Moreover, we found that CHP1 knockdown rescues reduced axonal outgrowth in diverse SMA models including SMN-deficient NSC34 cells, zebrafish *smn* morphants and MNs derived from SMA embryos. Interestingly, dephosphorylation of DNM1 by CaN was linked to neurotrophin receptor endocytosis promoting axonal growth (Bodmer et al., 2011). Together, our results indicate that CHP1 reduction and the subsequent increase of CaN-dependent dephosphorylation of DNM1 stimulates growth factor endocytosis and thereby axonal growth.

Finally, survival analysis using an SMN-ASO induced intermediate SMA mouse model expressing reduced levels of CHP1 shows extension in life span compared to mice expressing normal levels of CHP1. Interestingly, NCALD reduction, although beneficial, did not show such a favourable effect on SMA. This difference might be due to the rather ubiquitous expression of CHP1, which guarantees a systemic endocytosis restoration, while NCALD expression is restricted to neuronal tissues (Riessland et al., 2017). Additionally, CHP1 downregulation has a better effect on endocytosis than NCALD downregulation. Although, CHP1 reduction does not show such a gross effect on survival extension like PLS3, it is important to consider that PLS3 is involved in additional pathways disrupted in SMA such as actin-dynamics (Hosseinibarkooie et al., 2016) and in consequence can exert a general and stronger effect on the SMA phenotype.

Taken together, we show that CHP1 is a novel modifier of SMA, which restores processes impaired in SMA including endocytosis and axonal outgrowth defects. Furthermore, we demonstrate that CHP1 downregulation acts over CaN to promote neurite outgrowth and increase fluid phase endocytosis (Figure 11). Most importantly, CHP1 downregulation extends the lifespan of an intermediate SMA mouse model, resembling type II-III SMA patients. Although further studies are required to elucidate the mechanism behind the interaction of PLS3 and CHP1 and its influence in SMA, here we provide enough evidence to postulate CHP1 downregulation as a promising therapeutic target to counteract the pathogenic SMA phenotype and other MN or neurodegenerative diseases such as Parkinson disease, amyotrophic lateral sclerosis (ALS), and hereditary spastic paraplegias also characterized by endocytosis and Ca²⁺ misregulation (Schreij et al., 2016).

Since disturbed CaN activity or CaN-related pathways, like NGF signalling, have been further implicated in Down's syndrome and Alzheimer's disease neurodegeneration (Cooper et al., 2001; Ermak et al., 2001; Ladner et al., 1996), the activation of CaN activity by downregulation of its endogenous inhibitor CHP1 might be used as cross-disease therapy targeting a common neuropathogenic pathway.

### References

Ackermann, B., S. Krober, L. Torres-Benito, A. Borgmann, M. Peters, S.M. Hosseini Barkooie, R. Tejero, M. Jakubik, J. Schreml, J. Milbradt, T.F. Wunderlich, M. Riessland, L. Tabares, and B. Wirth. 2013. Plastin 3 ameliorates spinal muscular atrophy via delayed axon pruning and improves neuromuscular junction functionality. Hum Mol Genet. 22:1328-1347.
Andrade, J., H. Zhao, B. Titus, S. Timm Pearce, and M. Barroso. 2004. The EF-hand Ca2+-binding protein p22 plays a role in microtubule and endoplasmic reticulum organization and dynamics with distinct Ca2+-binding requirements. Mol Biol Cell. 15:481-496.
Barroso, M.R., K.K. Bernd, N.D. DeWitt, A. Chang, K. Mills, and E.S. Sztul. 1996. A novel Ca2+-binding protein, p22, is required for constitutive membrane traffic. J Biol Chem. 271:10183-10187.
Baumgartel, K., and I.M. Mansuy. 2012. Neural functions of calcineurin in synaptic plasticity and memory. Learn Mem. 19:375-384.
Bodmer, D., M. Ascano, and R. Kuruvilla. 2011. Isoform-specific dephosphorylation of dynaminl by calcineurin couples neurotrophin receptor endocytosis to axonal growth. Neuron. 70:1085-1099.
Clayton, E.L., V. Anggono, K.J. Smillie, N. Chau, P.J. Robinson, and M.A. Cousin. 2009. The phospho-dependent dynamin-syndapin interaction triggers activity-dependent bulk endocytosis of synaptic vesicles. J Neurosci. 29:7706-7717.
Cooper, J.D., A. Salehi, J.D. Delcroix, C.L. Howe, P.V. Belichenko, J. Chua-Couzens, J.F. Kilbridge, E.J. Carlson, C.J. Epstein, and W.C. Mobley. 2001. Failed retrograde transport of NGF in a mouse model of Down's syndrome: reversal of cholinergic neurodegenerative phenotypes following NGF infusion. Proceedings of the National Academy of Sciences of the United States of America. 98:10439-10444.
Cousin, M.A., and P.J. Robinson. 2001. The dephosphins: dephosphorylation by calcineurin triggers synaptic vesicle endocytosis. Trends in neurosciences. 24:659-665.
Crowe, J., B.S. Masone, and J. Ribbe. 1995. One-step purification of recombinant proteins with the 6xHis tag and Ni-NTA resin. Molecular biotechnology. 4:247-258.
d'Ydewalle, C., D.M. Ramos, N.J. Pyles, S.Y. Ng, M. Gorz, C.M. Pilato, K. Ling, L. Kong, A.J. Ward, L.L. Rubin, F. Rigo, C.F. Bennett, and C.J. Sumner. 2017. The Antisense Transcript SMN-AS1 Regulates SMN Expression and Is a Novel Therapeutic Target for Spinal Muscular Atrophy. Neuron. 93:66-79.
Di Sole, F., K. Vadnagara, O.W. Moe, and V. Babich. 2012. Calcineurin homologous protein: a multifunctional Ca2+-binding protein family. Am J Physiol Renal Physiol. 303:F165-179.
Dimitriadi, M., J.N. Sleigh, A. Walker, H.C. Chang, A. Sen, G. Kalloo, J. Harris, T. Barsby, M.B. Walsh, J.S. Satterlee, C. Li, D. Van Vactor, S. Artavanis-Tsakonas, and A.C. Hart. 2010. Conserved genes act as modifiers of invertebrate SMN loss of function defects. PLoS Genet. 6:e1001172.
Ermak, G., T.E. Morgan, and K.J. Davies. 2001. Chronic overexpression of the calcineurin inhibitory gene DSCR1 (Adapt78) is associated with Alzheimer's disease. J Biol Chem. 276:38787-38794.
Evans, G.J., and M.A. Cousin. 2007. Activity-dependent control of slow synaptic vesicle endocytosis by cyclin-dependent kinase 5. J Neurosci. 27:401-411.
Feldkotter, M., V. Schwarzer, R. Wirth, T.F. Wienker, and B. Wirth. 2002. Quantitative analyses of SMN1 and SMN2 based on real-time lightCycler PCR: fast and highly reliable carrier testing and prediction of severity of spinal muscular atrophy. American journal of human genetics. 70:358-368.
Flanagan-Steet, H., M.A. Fox, D. Meyer, and J.R. Sanes. 2005. Neuromuscular synapses can form in vivo by incorporation of initially aneural postsynaptic specializations. Development. 132:4471-4481.
Hosseinibarkooie, S., M. Peters, L. Torres-Benito, R.H. Rastetter, K. Hupperich, A. Hoffmann, N. Mendoza-Ferreira, A. Kaczmarek, E. Janzen, J. Milbradt, T. Lamkemeyer, F. Rigo, C.F. Bennett, C. Guschlbauer, A. Buschges, M. Hammerschmidt, M. Riessland, M.J. Kye, C.S. Clemen, and B. Wirth. 2016. The Power of Human Protective Modifiers: PLS3 and CORO1C Unravel Impaired Endocytosis in Spinal Muscular Atrophy and Rescue SMA Phenotype. American journal of human genetics. 99:647-665.
Hsieh-Li, H.M., J.G. Chang, Y.J. Jong, M.H. Wu, N.M. Wang, C.H. Tsai, and H. Li. 2000. A mouse model for spinal muscular atrophy. Nat Genet. 24:66-70.
Hua, Y., K. Sahashi, F. Rigo, G. Hung, G. Horev, C.F. Bennett, and A.R. Krainer. 2011. Peripheral SMN restoration is essential for long-term rescue of a severe spinal muscular atrophy mouse model. Nature. 478:123-126.
Jimenez-Vidal, M., J. Srivastava, L.K. Putney, and D.L. Barber. 2010. Nuclear-localized calcineurin homologous protein CHP1 interacts with upstream binding factor and inhibits ribosomal RNA synthesis. J Biol Chem. 285:36260-36266.
Kaifer, K.A., E. Villalon, E.Y. Osman, J.J. Glascock, L.L. Arnold, D.D. Cornelison, and C.L. Lorson. 2017. Plastin-3 extends survival and reduces severity in mouse models of spinal muscular atrophy. JCI insight. 2:e89970.
Kubler, E., and H. Riezman. 1993. Actin and fimbrin are required for the internalization step of endocytosis in yeast. Embo J. 12:2855-2862.
Kuwahara, H., J. Kamei, N. Nakamura, M. Matsumoto, H. Inoue, and H. Kanazawa. 2003. The apoptosis-inducing protein kinase DRAK2 is inhibited in a calcium-dependent manner by the calcium-binding protein CHP. J Biochem. 134:245-250.
Ladner, C.J., J. Czech, J. Maurice, S.A. Lorens, and J.M. Lee. 1996. Reduction of calcineurin enzymatic activity in Alzheimer's disease: correlation with neuropathologic changes. Journal of neuropathology and experimental neurology. 55:924-931.
Le Roy, C., and J.L. Wrana. 2005. Clathrin- and non-clathrin-mediated endocytic regulation of cell signalling. Nat Rev Mol Cell Biol. 6:112-126.
Lefebvre, S., L. Burglen, S. Reboullet, O. Clermont, P. Burlet, L. Viollet, B. Benichou, C. Cruaud, P. Millasseau, M. Zeviani, and et al. 1995. Identification and characterization of a spinal muscular atrophy-determining gene. Cell. 80:155-165.
Lin, X., R.A. Sikkink, F. Rusnak, and D.L. Barber. 1999. Inhibition of calcineurin phosphatase activity by a calcineurin B homologous protein. J Biol Chem. 274:36125-36131.
Liu, J., J.D. Farmer, Jr., W.S. Lane, J. Friedman, I. Weissman, and S.L. Schreiber. 1991. Calcineurin is a common target of cyclophilin-cyclosporin A and FKBP-FK506 complexes. Cell. 66:807-815.
Liu, J.O. 2003. Endogenous protein inhibitors of calcineurin. Biochemical and biophysical research communications. 311:1103-1109.
Liu, J.P., A.T. Sim, and P.J. Robinson. 1994. Calcineurin inhibition of dynamin I GTPase activity coupled to nerve terminal depolarization. Science. 265:970-973.
Liu, Y., H.C. Zaun, J. Orlowski, and S.L. Ackerman. 2013. CHP1-mediated NHE1 biosynthetic maturation is required for Purkinje cell axon homeostasis. J Neurosci. 33:12656-12669.
Miller, N., Z. Feng, B.M. Edens, B. Yang, H. Shi, C.C. Sze, B.T. Hong, S.C. Su, J.A. Cantu, J. Topczewski, T.O. Crawford, C.P. Ko, C.J. Sumner, L. Ma, and Y.C. Ma. 2015. Non-aggregating tau phosphorylation by cyclin-dependent kinase 5 contributes to motor neuron degeneration in spinal muscular atrophy. J Neurosci. 35:6038-6050.
Murray, L.M., L.H. Comley, D. Thomson, N. Parkinson, K. Talbot, and T.H. Gillingwater. 2008. Selective vulnerability of motor neurons and dissociation of pre- and postsynaptic pathology at the neuromuscular junction in mouse models of spinal muscular atrophy. Hum Mol Genet. 17:949-962.
Naryshkin, N.A., M. Weetall, A. Dakka, J. Narasimhan, X. Zhao, Z. Feng, K.K. Ling, G.M. Karp, H. Qi, M.G. Woll, G. Chen, N. Zhang, V. Gabbeta, P. Vazirani, A. Bhattacharyya, B. Furia, N. Risher, J. Sheedy, R. Kong, J. Ma, A. Turpoff, C.S. Lee, X. Zhang, Y.C. Moon, P. Trifillis, E.M. Welch, J.M. Colacino, J. Babiak, N.G. Almstead, S.W. Peltz, L.A. Eng, K.S. Chen, J.L. Mull, M.S. Lynes, L.L. Rubin, P. Fontoura, L. Santarelli, D. Haehnke, K.D. McCarthy, R. Schmucki, M. Ebeling, M. Sivaramakrishnan, C.P. Ko, S.V. Paushkin, H. Ratni, I. Gerlach, A. Ghosh, and F. Metzger. 2014. Motor neuron disease. SMN2 splicing modifiers improve motor function and longevity in mice with spinal muscular atrophy. Science. 345:688-693.
Nolle, A., A. Zeug, J. van Bergeijk, L. Tonges, R. Gerhard, H. Brinkmann, S. A1 Rayes, N. Hensel, Y. Schill, D. Apkhazava, S. Jablonka, J. O'Mer, R.K. Srivastav, A. Baasner, P. Lingor, B. Wirth, E. Ponimaskin, R. Niedenthal, C. Grothe, and P. Claus. 2011. The spinal muscular atrophy disease protein SMN is linked to the Rho-kinase pathway via profilin. Hum Mol Genet. 20:4865-4878.
Oprea, G.E., S. Krober, M.L. McWhorter, W. Rossoll, S. Muller, M. Krawczak, G.J. Bassell, C.E. Beattie, and B. Wirth. 2008. Plastin 3 is a protective modifier of autosomal recessive spinal muscular atrophy. Science. 320:524-527.
Palacino, J., S.E. Swalley, C. Song, A.K. Cheung, L. Shu, X. Zhang, M. Van Hoosear, Y. Shin, D.N. Chin, C.G. Keller, M. Beibel, N.A. Renaud, T.M. Smith, M. Salcius, X. Shi, M. Hild, R. Servais, M. Jain, L. Deng, C. Bullock, M. McLellan, S. Schuierer, L. Murphy, M.J. Blommers, C. Blaustein, F. Berenshteyn, A. Lacoste, J.R. Thomas, G. Roma, G.A. Michaud, B.S. Tseng, J.A. Porter, V.E. Myer, J.A. Tallarico, L.G. Hamann, D. Curtis, M.C. Fishman, W.F. Dietrich, N.A. Dales, and R. Sivasankaran. 2015. SMN2 splice modulators enhance U1-pre-mRNA association and rescue SMA mice. Nature chemical biology. 11:511-517.
Pinard, E., L. Green, M. Reutlinger, M. Weetall, N.A. Naryshkin, J. Baird, K.S. Chen, S.V. Paushkin, F. Metzger, and H. Ratni. 2017. Discovery of a Novel Class of Survival Motor Neuron 2 Splicing Modifiers for the Treatment of Spinal Muscular Atrophy. Journal of medicinal chemistry*.*
Riessland, M., B. Ackermann, A. Forster, M. Jakubik, J. Hauke, L. Garbes, I. Fritzsche, Y. Mende, I. Blumcke, E. Hahnen, and B. Wirth. 2010. SAHA ameliorates the SMA phenotype in two mouse models for spinal muscular atrophy. Hum Mol Genet. 19:1492-1506.
Riessland, M., A. Kaczmarek, S. Schneider, K.J. Swoboda, H. Lohr, C. Bradler, V. Grysko, M. Dimitriadi, S. Hosseinibarkooie, L. Torres-Benito, M. Peters, A. Upadhyay, N. Biglari, S. Krober, I. Holker, L. Garbes, C. Gilissen, A. Hoischen, G. Nurnberg, P. Nurnberg, M. Walter, F. Rigo, C.F. Bennett, M.J. Kye, A.C. Hart, M. Hammerschmidt, P. Kloppenburg, and B. Wirth. 2017. Neurocalcin Delta Suppression Protects against Spinal Muscular Atrophy in Humans and across Species by Restoring Impaired Endocytosis. American journal of human genetics. 100:297-315.
Roberts, B., J. Pohl, and J.L. Gooch. 2008. A fluorimetric method for determination of calcineurin activity. Cell Calcium. 43:515-519.
Schreij, A.M., E.A. Fon, and P.S. McPherson. 2016. Endocytic membrane trafficking and neurodegenerative disease. Cellular and molecular life sciences : CMLS. 73:1529-1545.
Smillie, K.J., and M.A. Cousin. 2012. Akt/PKB controls the activity-dependent bulk endocytosis of synaptic vesicles. Traffic. 13:1004-1011.
Smith, D.B., and K.S. Johnson. 1988. Single-step purification ofpolypeptides expressed in Escherichia coli as fusions with glutathione S-transferase. Gene. 67:31-40.
Timm, S., B. Titus, K. Bernd, and M. Barroso. 1999. The EF-hand Ca(2+)-binding protein p22 associates with microtubules in an N-myristoylation-dependent manner. Mol Biol Cell. 10:3473-3488.
Wirth, B., M. Barkats, C. Martinat, M. Sendtner, and T.H. Gillingwater. 2015. Moving towards treatments for spinal muscular atrophy: hopes and limits. Expert opinion on emerging drugs. 20:353-356.
Woo, C.J., V.K. Maier, R. Davey, J. Brennan, G. Li, J. Brothers, 2nd, B. Schwartz, S. Gordo, A. Kasper, T.R. Okamoto, H.E. Johansson, B. Mandefro, D. Sareen, P. Bialek, B.N. Chau, B. Bhat, D. Bullough, and J. Barsoum. 2017. Gene activation of SMN by selective disruption of IncRNA-mediated recruitment of PRC2 for the treatment of spinal muscular atrophy. Proceedings of the National Academy of Sciences of the United States of America. 114:E1509-E1518.
Wu, X.S., Z. Zhang, W.D. Zhao, D. Wang, F. Luo, and L.G. Wu. 2014. Calcineurin is universally involved in vesicle endocytosis at neuronal and nonneuronal secretory cells. Cell reports. 7:982-988.

## Claims

1. An inhibitor of Calcineurin B Homologous Protein 1 (CHP1) for use in a method for the treatment or prevention of a patient suffering from or being at risk of developing a disorder associated with a pathological calcium homeostasis, impaired vesicular trafficking and/or disturbed neuronal and/or neuromuscular synaptic function.

2. The inhibitor for use according to claim 1, wherein said inhibitor reduces the expression rate of CHP1, preferably wherein said inhibitor knocks down the CHP1 expression, in particular wherein knocking down is reducing the transcription rate of the CHP1 gene, reducing the translation rate of the CHP1 messenger ribonucleic acid (mRNA), reducing the transcript level of CHP1 and/or reducing the CHP1 function.

3. The inhibitor for use according to claim 1 or 2, wherein said inhibitor is an oligonucleotide or an oligonucleotide analogue, in particular an oligonucleotide or an oligonucleotide analogue selected from the group consisting of:
(a) an antisense oligonucleotide, in particular an antisense deoxyribonucleic acid (asDNA), an antisense ribonucleic acid (asRNA);
(b) an antisense oligonucleotide analogue, in particular an antisense 2'-O-methoxyethyl (2'MOE) oligonucleotide, an antisense morpholino, an antisense peptide nucleic acid (PNA), an antisense glycol nucleic acid (GNA), an antisense locked nucleic acid (LNA) or an antisense threose nucleic acid (TNA);
(c) an interfering oligonucleotide, more preferably small interfering ribonucleic acid (siRNA), short hairpin ribonucleic acid (shRNA) or micro ribonucleic acid (microRNA), in particular siRNA of from 18 to 24 bases in length;
(d) an oligonucleotide modifying the splicing of pre-mRNA, in particular wherein said oligonucleotide is single stranded deoxyribonucleic acid (ssDNA) or single stranded ribonucleic acid (ssRNA);
(e) an oligonucleotide analogue modifying the splicing of pre-mRNA, in particular wherein said oligonucleotide is a 2'MOE, morpholino, PNA, GNA, LNA or TNA; and
(f) an oligonucleotide encoding for one or more of the aforementioned (a)-(e), optionally wherein said oligonucleotide is embedded in a vector or virus in particular a self-complementary adeno associated viruses (scAAV).

4. The inhibitor for use according to any of claims 1 to 3, wherein said inhibitor is an oligonucleotide having a sequence homology of at least 80 % to any of SEQ ID NOs: 1-8, in particular wherein said oligonucleotide has a sequence of any of SEQ ID NOs: 1-8.

5. The inhibitor for use according to any of claims 1 to 4, wherein said inhibitor is
(a) covalently and/or non-covalently bound to at least one cell-penetrating peptide and/or at least one membrane disrupting peptide;
(b) included in or covalently and/or non-covalently bound to a liposome;
(c) included in or covalently and/or non-covalently bound to a micelle;
(d) included in or covalently and/or non-covalently bound to a polymersome;
(e) included in an episome;
(f) covalently and/or non-covalently bound to or included in a microbead and/or nanobead; and/or
(g) covalently and/or non-covalently bound to a non-toxic polymer, preferably, wherein said polymer is a water soluble polymer, in particular wherein said polymer is polyethylene glycol (PEG), polyethylene imine (PEI), polylactic acid (PLA), polyglycolic acid (PGA), poly(lactic-co-glycolic acid) (PLGA), hydroxypropyl methacrylate copolymer (HPMA) or a copolymer or block polymer of two or more thereof.

6. The inhibitor for use according to any one of claims 1 to 5, wherein the disorder is a neuronal disorder, preferably a motoneuron disease, in particular a disorder selected from the group consisting of spinal muscular atrophies (SMA), amyotrophic lateral sclerosis (ALS), hereditary motor neuropathies (HMN) Parkinson's disease, Frontotemporal Dementia, Alzheimer's disease, Morbus Huntington, and polyglutamin expansion diseases.

7. The inhibitor for use according to any of claims 1 to 6, wherein the patient is further administered with an agent increasing survival motor neuron (SMN) activity, preferably wherein said agent is selected from the group consisting of:
(a) an agent increasing the expression rate of SMN, more preferably wherein said agent is an antisense oligonucleotide or an oligonucleotide analogue blocking a silencer or enhancing an enhancer of exon 7 inclusion of pre-mRNA of survival motor neuron 2 (SMN2), in particular wherein said agent is an oligonucleotide having a sequence homology of at least 80 % to any of SEQ ID NOs: 9-16, preferably of at least 90 % to any of SEQ ID NOs: 9-16, more preferably of at least 95 % to any of SEQ ID NOs: 9-16, more in particular wherein the oligonucleotide has a sequence of any of SEQ ID NOs: 9-16;
(b) an agent capable of inhibiting non coding RNAs which are inhibitors of SMN2,
(c) an agent increasing the rate of functional SMN, in particular wherein said agent is an oligonucleotide or an oligonucleotide analogue modifying pre-mRNA splicing;
(d) an agent comprising genetic material encoding for functional SMN, optionally wherein said genetic material is embedded in a vector;
(e) an agent stabilizing the SMN; or inhibiting the proteasomal degradation of SMN;
(f) an agent that is increasing the activity of the SMN;
(g) replacement by gene therapy expressing SMN1 in an self-complementary adenovirus vector,
(h) an agent increasing PLS3 activity or decreasing NCALD, and
(i) an chemical molecule which increases the inclusion of exon 7 by enhancing U1-pre-mRNA association.

8. The inhibitor for use according to any of claims 1 to 6, wherein the impaired vesicular trafficking is an impaired endocytosis.

9. A pharmaceutical composition comprising an inhibitor according to any one of claims 1 to 8 and a pharmaceutically acceptable carrier.


SEQUENCE LISTING

<110>  Universität zu Köln

<120>  Calcineurin B Homologous Protein 1 Inhibitors and Therapeutic and
        Non-Therapeutic Uses Thereof

<130>  P70864EP

<160>  22

<170>  PatentIn version 3.5

<210>  1
<211>  21
<212>  DNA
<213>  Artificial Sequence

<220>
<223>  Mm Chp_1

<400>  1
ttcttgcttt ctagtattta a                                                21


<210>  2
<211>  21
<212>  DNA
<213>  Artificial Sequence

<220>
<223>  Mm Chp_2

<400>  2
cacgttattg cgggacgaag a                                                21


<210>  3
<211>  21
<212>  DNA
<213>  Artificial Sequence

<220>
<223>  Mm Chp_3

<400>  3
caataaatca attgttgtta a                                                21


<210>  4
<211>  21
<212>  DNA
<213>  Artificial Sequence

<220>
<223>  Mm Chp_4

<400>  4
agagctcgag gagatcaaga a                                                21


<210>  5
<211>  21

<212>  DNA
<213>  Artificial Sequence

<220>
<223>  Hs CHP_5

<400>  5
acggaagtgc ctaatatccc a                                                21


<210>  6
<211>  21
<212>  DNA
<213>  Artificial Sequence

<220>
<223>  Hs CHP_6

<400>  6
ttggcaaatg ttaatgacgg a                                                21


<210>  7
<211>  21
<212>  DNA
<213>  Artificial Sequence

<220>
<223>  Hs CHP_7

<400>  7
ttggttgact ttctgactca t                                                21


<210>  8
<211>  21
<212>  DNA
<213>  Artificial Sequence

<220>
<223>  Hs CHP_8

<400>  8
cagcagtatg cttacaggtt t                                                21


<210>  9
<211>  20
<212>  DNA
<213>  Artificial Sequence

<220>
<223>  SMN2 human ISS blocking morpholino

<400>  9
attcactttc ataatgctgg                                                  20


<210>  10
<211>  18
<212>  DNA
<213>  Artificial Sequence

<220>
<223>  SMN2 human ISS blocking ASO 10-27 2′-O-methoxyethyl-modified
      oligonucleotide with phosphorothioate backbone

<400>  10
tcactttcat aatgctgg                                                    18


<210>  11
<211>  15
<212>  DNA
<213>  Artificial Sequence

<220>
<223>  SMN2 human ISS blocking ASO 09-23 2′-O-methoxyethyl-modified
      oligonucleotide with phosphorothioate backbone

<400>  11
tttcataatg ctggc                                                       15


<210>  12
<211>  20
<212>  DNA
<213>  Artificial Sequence

<220>
<223>  SMN2 human ISS blocking phosporodiaminidate morpholino
      oligonucleotide (PMO) SMN2E7 (-10-29)

<400>  12
attcactttc ataatgctgg                                                  20


<210>  13
<211>  25
<212>  DNA
<213>  Artificial Sequence

<220>
<223>  SMN2 human ISS blocking phosporodiaminidate morpholino
      oligonucleotide (PMO) SMN2E7 (-10-34)

<400>  13
gtaagattca ctttcataat gctgg                                            25


<210>  14
<211>  22
<212>  DNA
<213>  Artificial Sequence

<220>
<223>  SMN2 human ISS blocking phosporodiaminidate morpholino
      oligonucleotide (PMO) SMN2E7 (-10-31)

<400>  14
agattcactt tcataatgct gg                                               22


<210>  15
<211>  21
<212>  DNA

<213>  Artificial Sequence

<220>
<223>  SMN silencer, preferably, SMN-siRNA

<400>  15
aagaaggaaa gtgctcacat a                                                21


<210>  16
<211>  22
<212>  DNA
<213>  Artificial Sequence

<220>
<223>  morpholino knocking down SMN

<400>  16
cgacatcttc tgcaccattg gc                                               22


<210>  17
<211>  20
<212>  DNA
<213>  Artificial Sequence

<220>
<223>  SMN-AS1 ASO-A

<400>  17
cagagtcctc ggtagaactt                                                  20


<210>  18
<211>  20
<212>  DNA
<213>  Artificial Sequence

<220>
<223>  SMN-AS1 ASO-B

<400>  18
gaaatctaat ttttcagttt                                                  20


<210>  19
<211>  15
<212>  DNA
<213>  Artificial Sequence

<220>
<223>  Sequence SMN1/2-AS1 lncRNA (NC_000005.10) RN-0005

<400>  19
cataguggaa cagat                                                       15


<210>  20
<211>  53452
<212>  DNA
<213>  Artificial Sequence

<220>

<223>  15 dna:chromosome chromosome:GRCh38:15:41229639:41283090:1

<400>  20
atgcctgtaa ccccagctac tagggagtct gaggcaggag aatcgcttga acccgggagg      60

cggaagtttc ggtgagctga gatggcgcct ttgcacttca gcctgggcag aagagcaaaa     120

actccgtctc aaacaacaac aacaacaaca aacaaacaaa actctagttc ttcaaactag     180

aacctcagct tgcagggaac gtgtggggcc tcaggctaag ctgtaaagcc ttaggtaaca     240

cttaaaacta gtaacttaag ttgacctggg aaagcctgct acgacaaaaa tatcctggta     300

cttgggtact aacagcagtc agaattcggt tcttccttat cttatacgtg ttctaaccaa     360

acaggtgtga aggggtaagg caggtgaagc agtaagaact ggttcacctg agagtaggca     420

ctaatttaac gggtggcttt tacctttttt tttttttttt tttgagacgg agtcttgctc     480

tgtcacccag gctggagagc aatgccgcga tctcggctca ctgcaacctc cgcctcccgg     540

gttcaagcga ttctcctgcc tcagcctccc gagtagctgg gattacaggc acgcggcacc     600

acacccggat aattttgtat ttttattaga gatggggttt ctccatattg gtcaggctgg     660

tctcgaactc acgacctcag gtgttctgcc cacctcggcc tcccaaagtg ctgggattac     720

aggcgtgagc caccgtgcca ggcctacctt tttaatcaaa acaaacaata caccatgaga     780

ataaaatgca aaatttctca tttttaagac aaaataagga acttcaaata aatggcggac     840

cataagctag gaattcactg tcctccatcg ttagggcttt ttcctccgaa ggaagtttgg     900

gaaatctgga tcctaatttc agccaagtgg tgcgttcctc gaacttcagt ctagaactaa     960

aagaagaggg gctgcaatga aggaagaagt ctcgggacgc tccacgccac ccggcggcgg    1020

ttatcaaatc acaggctgaa aacctggaga aaggtccgcg acgccgggga cacacgccgc    1080

agaggcgacg cccgcccggc ccaacgtcca gtctcgtctg tttcccgagg agccaatcag    1140

cacgcggccc cgaggccggc tccccagcgc cccctgcggc cgggaggagg acggcagcca    1200

ggaaagatga agagcgcgtg cagtccccga agggtacacc ctagggttcc cccccacgcg    1260

aagtttctct ccgcaatccg tgccacctat cgagtaggat tcctagtgtt cgattcctgt    1320

gagaaaaggt agactacgct gcctgtcgca ggttagtttg ttgtaaaact gttggtcaat    1380

gacgttgccc ttttaattgc gtcgttagcg accgagtggc atcgcccctt taaggccggt    1440

ggcagctgcg gttccggggg cggggtccgt cttcagcagg acttgggctg gagctgggct    1500

gattcattcg gggctggaac tcgaacgctg acgttggctc ctgagccggg cgctgcctcg    1560

cgcctgattg gcgggtcctc ggggccgccc tccccagcgc accacccctg ggttccctcc    1620

cgggtccgca gtggaaacac tgccctctcc cttcttgacc cctagccctt ccttccctcc    1680

ctccttccct cctgtcgccg tctcttctgg cgccgctgct cccggaggag ctcccggcac    1740

ggcgatgggt tctcgggcct ccacgttact gcgggacgaa gagctcgagg agatcaagaa    1800

ggagaccggc tgtgagttcg ggttgggggt gggaacgccg ggcgcctcag gctggcctca 1860

caaccaaggg cggctgtcgc taaggtctgg agctcgggtg cctgtgaggt tggggggtcc 1920

tgggcagcct ccaggtttgg ggaccgagag ctggaagacc tgttcttcca gctgcagctt 1980

cctggagcgg ggctctagct actgtggggc tgtccttccc ttcaaaatcg ggactcttga 2040

tcactcgtag agcgtcgacg cccccctttt ctgcaccctg cccatcaaag tccccgaact 2100

cctttccttt ctgttgcctc gcaccaccct tccctatgaa gaggcatcct gaacagtagg 2160

taaagcccga gaagcaaatt caggaactcg agatggattc tgtagaaaat ccgtttctgg 2220

gtaaggacct aaaacctctg acaactatgg gaaattacaa acaagttctt gtcacaaatt 2280

gggaaaacca cccagaaagc aagaccgtgt agtaaggctt tgggagacat gacccgatgc 2340

ctggagacct ttaaacaatc aggggtctgg gattgtgaga cttctttcaa tctgaacttc 2400

tagagaaaaa acacggatac tgtgaccacc agttttttac ttacacgacg atacttgctg 2460

tcctaatctc atatatgtat tgatttctac tatgctggtg tactttttta ttcgctattg 2520

aaattggatg tgctccaaaa atctccgtgt gaatttgtac taggaactga tttttttttt 2580

tttttttttt gaaacggagt ctccctctgt caaccaggct ggagtgcagt ggcgtgatct 2640

cggcacactg caacttccgc ctcccaggtt caagcagttc tcctgcctca gcctcccagg 2700

tagctgggag tacaggtgcc tgccaccacg cttggctaat ttttgtattt ttggtagaga 2760

tggggtttca ctatgttggc ccggctggcc tcgaactcct gacctcaagt gatccaccca 2820

cctcggcctc ccaaagtgct gggattacag gtgtgagcca ccgtgccctg cagaactgat 2880

tttttttttt atagtccagt ctcttcctca ttagtctgaa caaaggagca aaaggactcg 2940

ggattctgaa gcgctgtaaa ataccatcat aactttgaat agtccttgat aatgcaatgt 3000

atgatacaat tgtattgatg catacaatat tttatctttt cagtaaacct ttatgattct 3060

tattgagtta atgaaggatg tagcacctga gctctgtctc ttcatcatac ttgtctttga 3120

taatgacatg accagtaact cactccggcc catatgcaga tagtactgag gagtgctatg 3180

gggaactaca gaatttttca ggcctgttta agtgctgttc tctgctttgt gacaagttca 3240

actttctgga gttttcaagt tggaagggat gttttctagc cccacccctt tctggactgg 3300

tttgcttctt tcagcctccc ctaaaatatg gcccttaagc ctctctgtaa agacatttaa 3360

tgatggtaac ctctccacct accaaggcag tccttttcac gatttgaatg acccaaagta 3420

gttagctctt tataatttgc agtacttgtt tggccactga cttactctga ccctcggagt 3480

ggagacgcaa gcagtttgga ttcaacaagc attttattac gttttgtggc cataacttct 3540

ggccacattt gtaattagta ctactttagg tctagggtga caccgaatta aagtatttgt 3600

gttaaagagt cacatggaga aataagcaga atcaaagaga aataaatata gagtcacata 3660

atgagaaagt gtaaaccata gtgaagatgc aaaaagccat ttttgcttca gaatgaaggg 3720

agcagtaatc ccattctggt ttatatacat ttgaccaaat ggaaatgacc tggatgggat 3780

atttttacca tgtggcttgt ggacaaacaa ctaagtgctc atggaaaagc tgttttcccc 3840

attgccaaag agtgagtcac ttgtgaggga gattctctcc acaaccagtg caaatgtatg 3900

tattgagcca gaatgcttag tgaaacaagt gcttactgaa tttttacagt gtctgttcag 3960

tactgtgcta ggtactcaga gcagatggtg acaaaaaacg cccccaacct ggttggggag 4020

ataagcccaa gagagttatg attagcatca tgacataaga aagctgagaa ttgtgtgtca 4080

tccaccttca gaaatcaggc aagagagaga gtccttgtgg gctgtcctca tggaggacag 4140

gaattgggcc ttgatacatg ggtggagtag atttggatag attaacctag ggcattttgg 4200

gtgacttcca ggagagatct ccccctaagc ctgctacaaa gtcattcctt tgccctggga 4260

accatcagtt tatccaaata gtgtccatca tcagagttgt tttttctttt tctttttctt 4320

tattttagag acagggtctt tgctctgtct cccaggccgg actgcagtgg cttgagcata 4380

gctcactgca agccttaaac tcctgggctt aagccatcct cccgcttcag cgtcccaagt 4440

agctaggact acaggcgcat accaccatgc ttacctgatt tttattttta tcttttgtag 4500

agatggagtc ttgttatttt gtcgaggctg gtcataaact cctggcctca agtgattctt 4560

aggccacttc aacctcccaa agcacgggga ttacaggtgt gagccactgc gccaagccca 4620

gagttgtttt ttcaaggagt aatttggtat gatgcaaaag attttgaaga ataaaaacgg 4680

atatctggag tgggtagaga ggtggaattt gtcccatttt catctgcctt ttcttctatg 4740

aagaatttgg aaaactatct atcagaagca ctttaagaac aattataaga cattttacta 4800

cttcctttgc cagtacaaaa tgtaacataa ttgaggaata ctaaatttat attcagtgca 4860

aaagctgtgc attggaattt gatgattata tttaaccata ttttttgcaa atttaaaaag 4920

attagaattt ctacatgact agattagctg gtatttgata tttaatgaat aacagtaagt 4980

gtttggaaaa gtaattctgg ataagaatta cctgtgatcc tcagtttcat catctgtaaa 5040

attagaataa taatagtgcc tactttatag gattcttacg acaattacat gagatgaatt 5100

atgcaaacta cttagaatag tgtctggtat aataaacttc atgtgagtgt tattattttg 5160

aaccatagtt aacaatcgtc ttattgggaa aataccattt gcatgtacta gttataaaaa 5220

ctatgtgtag ggctatatca ttggggaggt gacagcattt tatacttctt tttgtagtgg 5280

acagttgatt aggaattaag tacagatgtt ttgccaatta taaggaaata aaatttttaa 5340

aggagttttt aacagtttat gaaactgtta tgatgtttta acaggaattg tctaagaaat 5400

gctttctatt ggcatatttg ctaaattatc tttgttctac taccttcttt cagacttctg 5460

cattttcata aagcttattt gaaaaaaagg tgtaatcggg gataaagcca caaatgtctt 5520

aggagtttct gtaatcatca cccttaattc acttaattgt agcaataagc tggctgtgaa 5580

ttacttaaaa ctttatgagg ccaggcgcag tggctcacac ctgtagttcc agcactttgg 5640

gaggccaagg tggcagatcg cttgagccca ggagttcaag accagcctgg gcaacatagg 5700

gaaaccccat ctctacaaaa aaataaattt aaaaaaaatt agctggacat ggtgaagcgt 5760

gcctgcagtc ccagctactc aggaggctga ggtgggagga ttgcttaggc gggaggtcaa 5820

ggctgcagtg agccatgttc gtgccactgc actctggcct gggagaaaca gaccctgtct 5880

caaaaaaaaa caaaaacaaa caacaaccac aaaaacacct tgataaacaa ttcatgccat 5940

tcaagtagta atgaatgcag attattttta gtctctacct tttggtatat gtcatgtggt 6000

acgggaaggg tgttgaacag gtttaggaag taagttgcac aggttggtaa ggttttgttt 6060

gtttgaaaga gtgtagtaat gatagagttc taacagagta caagcctggc aaaggcaatt 6120

gcttgagaac aatatcatgg ctagaatgta agtagaaatt caacttcagg tttctcgtgc 6180

ctccaaatgt aggacccttc acagccctca ccagcctgaa ggttctgtca cttcagaaaa 6240

ccaggctgtc ttctgcctcc tattgaaatg gaaatgcatg gataactaac acctttaact 6300

ggttttgggt taagtgattt gtaaagctct gtactttttg agttctagtg caaagtttta 6360

ttctcatttg ttgccataaa ggagtaagaa gcaactattt gtcaattaga gagggcattg 6420

tgggaaagag accctaagaa cttttttttt ttttttttga gacagggtct cactctgttg 6480

cccaggctgg agtgcagtgg tgcaatcata gctcattgca gcctcaaact cctggcctca 6540

aatgatcctt ccaccccaga ctttcaaagt actgggatta tagatgtgag ccactgtgcc 6600

tggcaactac ctgattttta atttttattt atttattttt tcagacaggg tcttgctctg 6660

tcatccaggc tggagtgcaa tggcatgatc acggctcact gcagcctcaa gcttctgggc 6720

tcaagcaatc ctcccagctc agcctcctga gtagctggga ctacaggcac atgccaccac 6780

gcctggctaa ttttttaatt ttttgtagag atagggtttc accatgttgc ccaggctggt 6840

cttgaactcc tgagttcagg tggtccgctc atcccagtcc cccaaagtgc tgcgattaca 6900

gaggtgagcc actggccagg aacttttttg cgggggcaag gaggggatgg tggtccagac 6960

cagagagtag ttggaggttt attcattcag aagataattg accccagcag cagctgcgaa 7020

gatataagct ttatttttgt aaatttctgg ataaaccatt tgaaagttgc aggtattgga 7080

actctttacc ccttcatact tctgcatgta tcccttaagt attgggatgg tttttacgta 7140

acaacaatac tattatcaca cctaagaaag ttgataatac ttcttaatac tatctttttt 7200

ttttttttga gacagagtct tgctcttgtt gcccaggctg gagtgcagtg actcgatctc 7260

agctcactgc aacctctgcc tcctgggttc aagccattct cttgcctcag cttcccaagt 7320

agctgggatt acaggtgcct gccaccaggc ctggctaatt ttttgtattt ttagtagaga 7380

tggggtttcg ccacgttggc caggctggtc tcgaactcct gacctcaggt gatccaccca 7440

cttcagcctc ccaaagtgct gggattacag gcgtgagcca ccgcacctgg cccctttttt 7500

tctttttctt tttttttttt taattgagat ggagtttcac ttttgtcgcc caggctggag 7560

tgcagtggcg cgatctcagc tcactgcaac ctccgcctcc tggcttcaaa tgattctcct 7620

gcctcagcct cccaagtagc tgggattaca ggcatgtgcc accacgcctg gctaattgtt 7680

tttattttta gtagatacga ggttttacca tgttggccag gctggtctcg aactcctaac 7740

ctcagatgat ctgcctgcct cagcctccca aagggctagg attatagacg tgagccacct 7800

taatattatc tatcattcac ttcctattca aatttctcca attataagaa gatagatttt 7860

gtttgtttta tataatgttt taaaatctct cttaatccct aagagactag tccagggctt 7920

gaagtcttca tgtcaggggg aatgttaagg tgtagtctat tccgggaagc gcaggcttca 7980

actttccttt atctagagct acctttagct gtacttgtaa tctttacata ggttatcagt 8040

ctgttcttct gcattaagat ttggaataaa ggcaaggaca aaataacttt ttcttttctt 8100

tttttttgag atggagtctc gttctgtcac caggctggag tgcagtggtg caatcccgcc 8160

tcccgggttc aagggattct cccgcctcag cctcctgagt agctgggact ataggcacgt 8220

gccaccatgc ccggctaatt tttgtatttt tagtagagat ggggtttccc catgttggcc 8280

aggatgatct tgatctcttg acctcgtaat ccacccacct cccaaaagtg ctaggattac 8340

aggcatgagc caccatgcct ggccctccaa aataactttt tcttactccc tttttgtttt 8400

tattctttcc tctgccactt ctgcttcgca cttctgtgaa atgccttcct cctctttgca 8460

caatgcagtg atgatagaaa gatttcagca acgttaacag ggtctttttt tgtttttttg 8520

gcgtgtgttt tttttttgag acagggtctc actctgtcac ccaggttgga gtggaggggt 8580

gtgatcatag ctcactacaa ccttgaactc ctgggctgaa acagtccttc tgcctctgcc 8640

tcctaaatag ctcagcgtac aggaatgcac cacgatgccc agctaatgat tgattgattg 8700

attatagaga tgaagtcttg ctttgttgcc caggctagtt ttgaactggc ctttcgtcaa 8760

gggatcctcc cactttagcc tcccaaagtg ctaggattac aggcatgagc cactgccctc 8820

agccaaacag ggtcttttaa ataatctttt aacaatttaa aatgattata tacccatacc 8880

agtggagaaa aaatacagct tcattcaaga catgtttata gcagggcacg gtggctcatg 8940

cctgtaatcc cagcacttta gggcggctga ggcgggtgga tcacgaggtc gggagatcga 9000

gatcatcctg gctaacacgg tgaaacctgg tctctactaa aaatacaaaa aaaaaaaaaa 9060

attagccagg catggtggtg gcaggtacct gtagtcgcag ctactcagga ggctgaggca 9120

ggagaatggc ctgaacctgg gaggcagagt ttgcagtgag ccaagatcgc gccactgcac 9180

tccatcctgg gtgacaaagc aagcctccat ctcaaaaaaa gaaaaaagac acgtttatat 9240

gcacggcctt ctctgagagt aactgtcaac tcctgttatt tcctttgggt taccaagaat 9300

tgtgcaggtt ttgggcacct atctgactag cttagagcct tctggggaaa atacttcact 9360

ggaaacatga tctgccttca ttcacaccag ttccagtacc tttctacaca tggatggtgt 9420

agctccttta taggtagatt gggattgttc tgagattgct ttatattctt tgtcacagtg 9480

aatttagtac ctaaattaaa gccttctctc cttctttttg ttaacaaaat tacttgtaac 9540

catatcgtta tggttctaaa atttgttcta aaattttgga gataagttta gatatgacca 9600

tattgattag tattaaaagt tgacatttgg ttgtatgttt ctccactgac tcccatataa 9660

cccagttcgt cctggaagat agtttttttt tttttaatgt tttggctact tatttacatt 9720

atttattttt aacatattag tacattattt tttgttatcg tttgttttgt tttgttttgt 9780

tttgaggcgg agtctcactc tgtcgcccag gctggagtgc agtggtgaga tttcagctca 9840

ctgcaacctc catctcccag gatcaagcga ttctcctgcc tcagcctccc aagtagctgg 9900

gattatacca ccacacctgg ctaatttttg tatttttagt agagacaggg ttttaccatg 9960

ttggccaggc tggtcttgaa ctcttggcca caggtgatct gcccgccctc agccccccaa 10020

agtgctggta ttgcaggctt gagccaccag gccttgccac attttgaatt taagcagcca 10080

taaactggtc ttaacatact ataggaggtt tcagtgccca gttattttat ttctttttta 10140

tttatttatt ttttcgagac agagtctcct ctctgtcacc caggctggag tgcagtggcg 10200

caatctcagc tcactgcaag ctccgcctcc caggttcaaa ccattctcct gcctcagcct 10260

cccgagtagc tgggactaca ggtgcctgcc accacgcccg tctaattttt tgtatttgta 10320

gtagagacag ggtttcaccg tgttagccag gatggtctca atctcctgac ctcgtgatcc 10380

gcccgcctcg gcctcccaaa gtgctgggat tacagctggg attacaggtg tgagccacca 10440

cgcccagcct cagtgcccag ttattttaga tgatatagat tagaaagatg tttaatggct 10500

catttgtgga tttttttggt ttaaaataat tttatttttt atttttattt ttattttgtt 10560

ccccagcctg gtcttgaact cctggcctca aatcattgtc ctgcttctgc ctctggaagt 10620

gctgggatta taggcgccag ccatcatgcc tggctctcat ttgttcattg aaggggtcgg 10680

attagataac atttctacca attttaaaat tatataaatt ggataaagaa ggactccaaa 10740

gcaatataga aaaagtatga attactaaat gaaaagcaag ctattacaac tttgtaaagt 10800

acacgagtaa ctgatattag atgatatatg aagagctggg cgcggtggct catgcctgta 10860

atcccagcac tttgggaggc cgaggtgagt ggatcacccg aggttgggag tttaagacca 10920

gcctgaccaa catggagaaa ccctgtctct actaaaaata caaaattagc tgggtgtggt 10980

ggcgcatgcc tataattcca gctacttggg aggctgaggc tggagaatca cgggaaccca 11040

gaaggcggag gttgtggtga gccaagattg tgccattgcg ctccagcctg ggcaagaaga 11100

gcgaaactct atctcaaaaa aaaaaaaaaa aaaaagatct atgaagaaat gaaaatggca 11160

gtgttgggat tgtgaacaaa ttttgttttt ttcaaaaaat cttttgggaa acttagtaat 11220

tctttttttt tttgaaacag agtcttgttc tgtcaccagt ctggagtgca gtgacgtgat 11280

ctccgctcac tgcaaccttc gcctccctgg ttcaagtgat tctcctgcct cagcttccca 11340

agtagctggg actagaggca gacgccacca cacccagcag atttttgtat ttttagtaga 11400

gacgaggttt cgccatgttg gccaggatgg tctcgatctc ttttcctcag catccgcccg 11460

ccttggcctc ccaaagtgct gagattacag gcgtgagcca ctgcgcccag cctgaaattt 11520

agtaattctt taactgactt atttttgtta aactaaaggg aactttagta caagaatagc 11580

ctttgaggcc tgaggccaga actgggatag actggtcaag ttcttaaaga aaacggttgt 11640

aactggcaaa gtcaggaggg aaatggagag taattagtgc agcagtaata cttgtgcctt 11700

caggctcagg atgtaagcat gatgctgata atagctaatc tggagtgctt gccatgtgcc 11760

tggcactttg ccaagcattt tcctttcatt gactggtgag ttcgctctac aatcccattc 11820

acttacctct ctctggtaag ccatggtgct agcaatggaa tcagaatctg tctgcctaac 11880

cctcacaagt gtgtacactt tacagtgagc catttatacc tgtccggctg gcaaaacagt 11940

ccacaactgt ctccttagtg gctggatccc tgctggctct gccccgatgg aaatgctttt 12000

gccttctctg ctcagtgatc actttagagc actaggacag aacagagccc agcttcgagg 12060

gtgaaacctg atgctgaaga attcacagcc acacatagtg tgtcctctta tcccagctct 12120

gggccactca ctacctgctt gtccctgttc actttgtccc attgttccca acactacaag 12180

tacatttttg tttcatgagc tgagggagga ggacacctta tctgaatgac tttggccacc 12240

cctttaaacc tctgaaggta cagcctcgga caaagtccac acttgtcaca ccctttttgg 12300

ctgttgtctt ttctcctctg tttgtttccc ctctcttact tccaatctat caggagacta 12360

aactatttcc cagaaaggcc tttttgagtt tttagtatta ttatgtacct gccattattg 12420

cctcatttta attgtcaact tgtatcttga gtttatggac taaatccaaa atgaggagca 12480

aaatagcttt ctggtagatg tcatctgatg atagcatgac cattttacga atttgcctaa 12540

tttgcttcct tggagccagt tcccttcctg ggaactcagt gaagctcatg ccatcacaga 12600

aaagaggttg atctttatgt tttccaaggg aaaagataaa gcctttaatg cttggtgtag 12660

gagtaaacca gagttttaaa atagaatctg tttgatgtac cccaatcaaa tattgtacac 12720

ccaacaccta cctagccgta cacatcacct acctagccgt acacctacct agcagtacag 12780

cctgaaacag caaactgatc atggatattt tttcaatcaa aacatgcatt ttttagctgg 12840

aactgtggct catgcctgta atcccagcta cttgggagac tgaggcacga gaatcgcttg 12900

aacccaggag gtggaggtta cagtgagctg agattgtacc actgcactcc agcctgggcg 12960

acaaagttga ggctctgtcc cccaaaaata aaataaaata aaatttaaaa aataagcaaa 13020

ataggccggg catggtggct catgcctgta atcccagcac tttgggaggc tgaggcaggt 13080

ggatcacctg aggtcaggag ttcaagacca tcctggtcaa catggcaaaa ccctgtctct 13140

actaaaaata caaaattagc tgggcatggt ggtgggcgcc tgtaatccca gctacttggg 13200

aggctgaggc aggagaatcg cttgaacacg agaggcagag gttgcagtga gccaagattg 13260

tgccattgca ctccagcctg ggcaacgaga gtgaaacccc atctcagaaa aaaagaaaaa 13320

agtaaaaaaa taaatacagt aaaaacatac attttcttag cctctaagtc agggcattga 13380

aacttcaaag tttcacagct tccttttaaa ctaaatttgg gctgggcatg gtggctcaca 13440

cctataatcc cagcagtttg ggaggctgag gcgagtggat cacctgaggt caggagttca 13500

agaccagcct agccaacatg gcgaaacccc gtctctacta aaaatacaaa aaattagctg 13560

ggtgtggtgg cggaagcctg taatcccagc tactcgggag gctgaggcag gagagttgct 13620

tgaacccggg aggcagaggt ggcagtgagt gagatcgtgc cactgcactc cagcctgggt 13680

gacaagagca aaactttgtc tcagtaaaat aggatgggat aggataggac aggacaggac 13740

aggacaaaat ttgcctttgt tttgttaaat cttaaccatc taatattcat cacagtctta 13800

attaagccta gacttcattc ctgggattga attttgattg cccttgattt agtttattct 13860

ctaactcctt tcattcttta gcttttaaac tttctgcttt aacattctag agataagagt 13920

ttcgacatcc cagtcctact gaattttgaa gcatttgaca gcgaggggtg ggttcagtgt 13980

gaatgagggc tacttccccc gagctgggac taattttgtg ctctttagtt tcccacagtc 14040

aaatcactcg cctctacagc cggttcacca gcctggacaa aggagagaat gggactctca 14100

ggtaggcagt gtttttcttt attttcctca cagaaaccag aaaccctctg gcagtgtctg 14160

aatagctgcc tttatcccta gggtagacat gatagacatc cagcatgcag atagcaagga 14220

ccatttctct actggtcttt gagcatcatt gatccacaag agaattaaat cagcagaaca 14280

ggctgggcgc ggtggctcac gccttaatcc cagcactttg ggaggtcaag gcgggcggat 14340

catgaggcca ggaggtccag accagcctga ccaacatggt gaaaccccgt ctctactaaa 14400

aatacaaaaa ttagctcagt gtggtggtag gcgcctgtaa tcccagctac tcaggaggct 14460

gaggtaggag aattgcttga acccgggagg cagaggtcgc agtgagcaaa gatcacgcca 14520

ctgcactcca gcctcggcaa cagagcaaga ctccatctaa aaaaaaaaaa aaaaacagcg 14580

gaactatgac aattaaatgc attaacagat gtgtgttgct ttattccaac tccccttaat 14640

ttaggtggcc attgttctga ttctaggaat gtatttttaa gatttcagct ctctctgctt 14700

acattttccc ataatccttt cccaaacatt ggcttttagg aagttgaccc atagcgatcc 14760

caggtggtct aggttgtttt gtcttctttt atttaccaac ccataaccct gaggtacatc 14820

actttatgta tattaatgtt ggacaaaatg ttcagtatgt tagtctagag cagtggttct 14880

catctgaggg ccattttgcc acctggggca aaatgtctgg aaacattttt aattgccatg 14940

actggagaat gctactagtg cctagtgggt tgtggccaga gatgctgcta aacatcctac 15000

agtgcacagg acagccccca tgacaaataa ttattctggt ctagatgtcg gtagtgccaa 15060

aattgagaaa ctctggtcta ggtaagtata tgatatcaga gtgatgtaat gctatattgt 15120

tttatgtttg taattgtctt accataatca aatgaaatat tctttaccat tatctcccac 15180

actaactctc agtagcaacc tttcttatgc tgtggttctt catctttgat tggcatgtaa 15240

tgaagctgtt gcccttcctt ttggctggca tgaagcagct taggacttct ttagaaagtt 15300

tctagcacag gtgttggatg tagcataaag ggggaaggaa tacagttgct aagcctttgg 15360

acgtaatcat caccccaaat ggaaacccca tactcattaa gcatggggtt aagcatacct 15420

attaagcata cgtatttccc ccttctctca tcccctggca accataaatc taccttcttt 15480

ttctctctgg acaattcata taaatggaat tatatggccc tctcataact gacttcttct 15540

ggctggacac ggtggctcat gcctgtaatt ctagcacttt gggaggccaa ggcgggcgga 15600

tcacctgaga tcaggagttc gagaccaggc tggccaacat ggggaaaccc cgtctctatt 15660

aaaaatacaa aaattagcgt ggcgtggtgg tgggcgcctg tgcgtggtgg tgggcgcctg 15720

taatccaagc tactcaggag gctgaggcag gagaattgct tgaacttggg aggtggaggt 15780

tgcagtgagc ccagattgtg ctgctgtatt ccagcctggg cgacagagag agactttgtc 15840

tcaaacaaac aaactgactt cttctactta acatgatgtt tttaaggttc atccatgatg 15900

tagcatatac cagtacttca ttccttttta tggctgaata aatagtctgt catatggata 15960

tgccacattt gtgtatccat tcagcagtta gtaaacattt gagtttacac cttttgagta 16020

ttgcaaatag tgttgcttta aacatttttg ggtgcacata tgttttaatt tctcttggac 16080

gtatacctag gagtggaatg gtgggtcata tgagttgtct tttcactctc tttgtagtgt 16140

tctttaaagc acaaaagttt ttaattttga tgaagtccag tttctttaat atttctttca 16200

gtgcttgtgc ttttggtatc atatataaga aaccattgcc aaatccagtg tcccaaaggc 16260

ttatcctata tcttcttgta agggctttat ggttttagtt cttatattaa ggtcttttga 16320

tttattttga gttggttttg tatatggtgt cagagaagag tccaacatca tttttttttg 16380

catgtggata tccagttgta ccagcaccat tcgttgaaga cgctattaaa actatgtgaa 16440

tttacacttg aaaaataact agactagaaa tccctatttg taattttagc tccaatactt 16500

aaaaattcct ttgagtttat acttgcctca taaattcaga cttaaccacc cagtaaagac 16560

ttaaaattta tattctgagt tcttttcttc tttgtagaaa gtgggtggtt tctaaatgtg 16620

tgtgttttta tcatgtttgc tgtgtgctgg aaaataatcg aaaagttttt tttttaatga 16680

gctgtttctt ttttttttaa gatggagttt cactcttgtt ggccaggctg gagtgcaatg 16740

gtgtgatctc ggctcactgc aacctctgcc tcccgggttc aagtgattct cctgcctcag 16800

cttcctgagt agctaggatt acaggcgcat gacaccacgc cctgctaatt tctgtatttt 16860

tagtagaaac ggggtttcac catcttggcc aggctggtct caaactcctg acctcgtgat 16920

ccacccgcct tggcctccca aagtgctggg attaaaggtg cgagccaccg tgcctggcca 16980

aaaagctttt tttttttttt tttttttttt ttgagagaga gtctcgctct gtagcccatg 17040

gtctcgatat cctgacctcg tgatgcacct gcctggctaa cgcagtgaaa ccccgtctct 17100

attaaaaata caaaaaatta gctgggcgtg gtggcaggcg cctgtagtcc cagctactcg 17160

agaggctgag gtaggagaat cacttgaacc tgggaggcga gatcacgcta ctgcactcca 17220

gcctgggaga cacagctaga ctccatctca aaaaattaaa aaaaagcccg ggtgtggtgg 17280

ctcacacctg taatcccagc actttgggag gctgagacgg gcggatcaca aggtcaggag 17340

gtcgagacca tgggctacag agcgagactc tgtctcaaaa aaaaaaaaaa aatgttgaaa 17400

actttccagg cgtcagtgta gcctgctcct taaaatctaa gtaagggcca agcgcagtgg 17460

cttacacctg gctgtaatcc agcactttgg gaggccaagg caggtggatc acttgaggcc 17520

aggagtttga gatcggcctg gccgacatgg tgaaacacca tctttacgaa aaatacaaaa 17580

attagctggg tgtggtggcg catgcctgga attccaggta cttgggaggt tgaggcagga 17640

aaattgcttg aacccgggag gcggaggttg tagtgaacca agattgcagc actgccctgc 17700

accctgggca acacagtgag atcctgcctt taaaaaaaaa aaaaattctg gccggttgtg 17760

gtggctcact cttgtaatcc cagcactttg ggaggccgag atgggtggat cagctgaggt 17820

caggagttcg agaccaggct ggccaacatg gtaaaacccc gtctctacta aaaatacaaa 17880

aaaattaacc aggtgtggtg gcgcacgcct gtaatcccag ctacttggga ggctgaggca 17940

ggagaatcac ttgaacccgg gaggtggagg ttgcagtgag ccgagatcgt gccactgcac 18000

tccagcctgg gcagcagagc aagactccat cttaaaataa ataaataaat agggccgggc 18060

gcggtggttc acgcctgtaa tcccagcact ttgggaggcc gaggcaggca gatcatgggg 18120

tcaagagaag accaacctgg ctaacacagt gaaaccccgt ctctactaaa aatacaaaaa 18180

attagctggg agtggtggtg ggtacctgta gtcccagcta ctcgggaggc tgaggcagga 18240

gaatggtgtg aacccgggag gcggagcttt cagtgagccg agatcacacc accatactcc 18300

agcctgggcg acagagcaag actccgtctc aaataaaaaa ataaataaat aaaataaata 18360

aataaataaa taagtatcta agtaagattt gctcaatctg taagttacca tttcacagcc 18420

tgtggaaaac cagcagacta gctcactcat ttggtaggta tttactgact atctatgatg 18480

tgccagacac tgttctccat gctggcaata tttttttttt ttttgtaaga gacagggtcg 18540

cactctgtct cctaggctgg agtgtaatgg caccatcaca gctcactgca acctcaacct 18600

cctgggctca gacagttctc ccacctcagc tgcccaagta gctaggacta caggcatgca 18660

tcactatgcc tggctaattt tttttctttt ttacttttag caaaaaagca aaaaagcgca 18720

cattatgtgg tacaatggaa tagtttcttc taggtattca aggtagcttg gtgcttctcc 18780

tgttattttt attttatttt cattttttgt ggagacacag tctcaccttg ttgcccatgc 18840

tggtctcaaa ctcctgggct ccatcagtcc tcccgccatg gcctcccaaa gtactgggat 18900

tataggcata agccactgta cctggcctga aagctctagg tcttaaacct gttctttgat 18960

ctctatcatg gtgtggattg aaatataaga aaggttgggc gaggagactc atgcctgtaa 19020

tcccagcact ttgggaggct gaggtgagag gattccttga ggcccggagt ctcaagacca 19080

gcctgagcaa catagtgaaa ccccgtctct acaaaaaata aaaataaatg agagaagcac 19140

caagctacct tgaataccta gaagaaatta gccaattgta ccacataatg tgccacatag 19200

taaacgagta atatatttta tgtaaatcgt tttgtcagac ccacccacta ctgggatttc 19260

actctggctg tgcctcctga caattatctt cttagcctgt ggccatccga ttaaattgtc 19320

ttccccttct ctcagccctc tcctcttcta ccccctctag ccttagaaat gagttggcct 19380

aactgggaac tcttctctag tagaatgatc cagttctctc cagcctttgt tgccaaactc 19440

agcttaaggc ctgtcttgat gtttccagga cccatcagag tgctcatccg aatacaagtg 19500

gcttccaatc ttttgttacc tggaacagct tccaccttct ttttttggtt tttgtttttg 19560

agacagggtc tgactctatt gcccaggctt gattgcagtg gcaccatcac ggctcactgt 19620

agcctcccac ctcagcctcc tgagtagctg ggactacagg cacgtgtcac tgcctggcta 19680

acttttgtat tttttgtaga gaggaggtct cgccatgttc cccatgactg gtctcgaact 19740

cctgaactca aaccatctgc ctgcctcagt ctcccaaagt gctgggacta cagatgtgtg 19800

ccaccatgcc tggccttcac cttctttttt tttttttttt tttttttttt ttgagatgga 19860

gtctcgctct gtcgtccagg ctggagtgca gtggcacgat ctcggctcac tgcaacctcc 19920

gcctcccggg ttcatgccat tctcatgcct cagccaaccg agtagctggg actacaggtg 19980

cccgccacca cacccagcta attttttgta tttttagtag agacggggtt tcactgtgtt 20040

agccaggatg gttttgatct cctgaccttg tgatccacct gccttggcct cccaaagtgc 20100

taagattaca ggcgtaagcc actgcgcccg gcctcgcctt caccttaaga aggcagttgt 20160

agttattagg agaaagattt tcacttcaac aatttttttt tttttttttt actttcctct 20220

ttcaaggtgc tccatgtctg ggcctgaagc caaattctgc agagtgagag gctacagtgt 20280

acagtctggg gcattctgca ttcctttgta gtgatcagct ccagtggaac ataaggcaga 20340

actgtggaag gctttggtta gaatggagct tcttcagagt gctttcagag actcactccg 20400

aagcatgctt tatagaacag tcattccttg gtctatcaat aggtgttatg ttaaaaaaaa 20460

aaaaaaaaag caaaaagtta ttgactaaaa tttgtgaaaa ccttggtcaa caaggttaaa 20520

taggctacct aattactaga tttcaccaca tctttaattt acctgggtga attgggaaca 20580

ttcaagggag aagtgtttgc caaattgatt tgcaatttcc ctgtctgcct ttttacatgg 20640

accatctctt cagattgata tttcacggaa tataaatata cactgtaacc ttataaacct 20700

tactttcttc agatgccatt attttgggta agatatttct accattgcca tagagattgt 20760
tttttctaaa aaggcagctt aggccaggcg cagtggctca tgcctgtaat cccagcactt 20820
tgggaggccg aggcagggga atcgcttgaa cccgggaggc ggaggttgca gtgagccaag 20880
atcatgccat tgcactccag cctgggcagt aagagtgaaa ttccatctca aaaataaata 20940
aataataaat aaataaataa taaagaggag gagaagaaat aatttttaaa ataggaacaa 21000
aatccaggtg aggtttctta ccaaaaaaaa aaaaaaggaa cagaagcaaa cttcccagct 21060
aaagggccca tttttagtct aagaaggttc acctaatgct gaacaatgaa taataaagaa 21120
tacttcacaa taagatatat atagatgaaa tttcagaata tcaagaggag atatttaaga 21180
ctttctttct ttctttcttt tttttttttt gagacagagt ctcgctctgt caccaggctg 21240
gagtgcagag gcacaatctc ggctcactgc agccaagttc accgcttccg ggttcaagtg 21300
attctcctgc ctcagcctcc agagtagctg caattatagg cgtgcactac cacgcccagt 21360
gaattttttt atttttagta gagacgggtt tcaccatgtt ggccaggatg gtctcgatct 21420
cttgaccttg tgacccaccc acctcagcct cccaaagtgc taggattaca ggtgtgagcc 21480
actgtgcctg gcccagagga tgtttttaaa gcaaacttta ttgaagtata acatacagaa 21540
aagtgcacaa atcaaaagca ttggacatga taaattttca ccaagggaag actatccagc 21600
ctccagatga agaaactgat tattaccaat acccagaagc ttcccttatg ctccttttgt 21660
ctttacctgc tcccctcccc aaagggtaat cattatcctg ccttgtaact ccatagatta 21720
catcagaaca aagaatattt aaagaagaga aaccatcttt gtgttcagat actgaccagg 21780
ctcttacctt aacctgacct gcaaaagacc actttaggac ccctggggtc cccaacctct 21840
gggttgcaga ccagtaccag tctgtggcca gttaggaacc gggctacaca gcaggaggtg 21900
aacagaatgc aagtgagcat tatcacctga gcaccgcctc cggtcagatc agtggcggca 21960
ctagattctc ataggaacag aaaccctatt gtgaactgcc catgtgaggg atctagactg 22020
agtgctcctt atgagaatct aatgcctgct gatctgaggt tgaacagtgt catcctgaaa 22080
ctatcctgtc tacccccagg agaaaaatta ttttccataa aactggtccc tggtcccaaa 22140
aaggctgggg atcgctgcca gctgcctttt tttttttttt tttaattaga gacagggttt 22200
ccctatgttg cccaggctgg tctcggactc ctgggttggg attacaggca catgccacta 22260
tgcctggctt attttgtatt tttaatagag acagggtttc tccatattgg tcaggctggt 22320
ctcaaactcc tgacctcagg cgatctgccc accttggcct cccaaagtgc tgggattata 22380
ggcgtcaacc accacgcctg gccatcttat ccatttaatt taaaaatact gattaagtgc 22440
tttagctaat ggaaattttt gtcatttcag tgttaataaa tatgttgtac catatgctta 22500
acctctcata ctacttaaaa tggaaagctg tattttcttt tctttttttt ttttgagaca 22560
gagtctcgct ctgttgccca ggctggagtg gtacaatctt ggctgactgc aacctccgcc 22620

tcccgggttc aagcagttct ctgcctcagc ctcccaagta gctgggatta caggcacccg 22680

ccaccacgcc cgtctgattt ttgtattttt agtagagacg gggttttacc atcttggcca 22740

ggctcgtgtc taactcctga cctcatgatc cacccacctc ggcctcccaa agtgctggaa 22800

ttacaggagt gagccaccac gcccggcctg gaaagctgtg ttttctaatt gaatgtgtgt 22860

tccagtgggt atgtcctatt aacagatttt aatctcctca ttatgtacaa gtcaactaaa 22920

gcaacaacct gtgggtgctt gagatagaag ttacctgtga ggctgaatct ctctgtaaga 22980

gaagggactt gcacagtgaa ggtgagcagt atagagggat ggcatgtctg gttactaaca 23040

gtctaaaaac cctggttttt ctaagcatcc ctgtgccctt gaatgtttgg aaaaccactc 23100

cccattggaa atttcttggc cacaaagaat gccatcgtac atggctctgt tctcagaatt 23160

gactactacc agtgatactg tctttttcta aaaaaatctt caaggaaggt tttcctaggt 23220

ataccatgag gcagaacata atgttggtat gcttttcagt gaatccacac aaatctgtat 23280

ttcacatggt tttttttttt tttttttttt tttttttttg agatggagtc tcactctgtc 23340

accaggctgg agtgcagtgg cgcaatcttg gctcactgca acctccacct cccaggttca 23400

agtgattctc ctttctcagc ctccagagta gctgggacta caggctcgcg ccaccatgcc 23460

cagctaattt ttgtgttttt agtagagacg gggtttcacc atgttggcca ggatggtctt 23520

gatctcttga cctcgtgatc cacctgcctc agcctcccaa agtgctggga ttacaggcgt 23580

gagccactgt gcccggcctt cggatggatc ttatgcttgt ggtgtttagg ccactttgga 23640

gtatttattt ttttctatta ggtttatgtt gatgggtaaa tttgagaatc taagttttag 23700

agaaactgcc cagatctaca ttctactata aagcaaccag taataataac ttgttcgtat 23760

attatttatt taacagttga caaagtattt atttatttat ttatttattt atttatttat 23820

ttatttattt attttgagac ggagtcttgc tctgtcacca ggctggagtg cagtggcgca 23880

atcttggctc actgcaacct ccacctccca ggttcatgcc attctcctgc ctcagtctcc 23940

cgagtagctg ggactacagg tgcccgtcac cacgcctagc taattttttg tttttttagt 24000

agagacgggg tttcaccgtg ttagccaggt tggtcttcat ctcttgacct catgatcctc 24060

ccgcctcggc ctcccaaagt gctgggatta caggtgtgag ccactgcgcc cggctgacaa 24120

attatttttt atactctttc tgtttgtttt gttttttctg ttttgaaaca gggtctcact 24180

ctgtcaccca ggctcgaggg cagtgatgtt accatggctc cttgcagcct tgaccttcta 24240

ggctcaaaca atcctcccgc ttcagcttcc caagtagctg ggactacagg cacgtgccac 24300

catgcctggc taattttttt ttttttttta aactttttag agatgtggtc ttgctctgtt 24360

gcccaggctg gtctcgaact cctagcctga agcagttctt ccaccttggc ctccaaaagt 24420

gctgggatta taggctgagc tgccataccc agccctcttt ctttttaact ttaatacttt 24480

tagcaatact gtccttgatc tgggtgactg gaactacatt atatatacaa tctaaattct 24540

aagagtagag ttagaaagta tcaaaggaca ggtccatcgg aaggccaagt gtacacctcc 24600

ttattgagac tgaagttcag gctaggttgt gcatcaccac ttgatactag acttggtatt 24660

taaactgcct tttctcagct aaagtttctt aagcttgtta gacattaaac tgaagtatgt 24720

agccatgcaa ttcaaatcag ccttagtctt aatttaaaag tgagtagtta ttgtttcttg 24780

acctctgtca gacaggagga gctacatttt gatgatagtg tagactttgt attacagaac 24840

aaattatgta ataaaagctt agtacatgtt tgttgaatta aataatcagg acctcggtaa 24900

ttttctcttt catcatctta agcaatccag ttatcttatg aatgacttct tctggttcat 24960

gcattgatat aaaattatta cactaaatgg tcaagaagga ctagaatcca ggatgattgt 25020

tccagagctt tgtgttcagt ccactaggtc agtttctacc tgtgtcacca aatgtggtgg 25080

agaagagctt gctgagttaa ttaagcacca cgtaaacgtt aaaccacaag aggaaaagag 25140

cgtgaagtct cctgggtaca gtaggagtat tttattatct tccgtctttg ctagcatata 25200

ttacaaagag ggtggaggta tacattttat ctgtctgtaa gtaaacttct tgaaggcagg 25260

agtaaaatct agtttataat ctttttttta actttttatc tggaaataat tttaaacttg 25320

aagttacaag aacaatacaa gataactttt attctgatac ccagattcac caattgttaa 25380

cattttgtca catacttcac tgtttatttc ctatagaaag gacattctca gatcaaaatc 25440

aagaaagttg cattttgaaa cagtaccatt atgtaatatt ccatatttta tatggtccat 25500

gcttcatttt cacatattat ccaataatgt ccttatagca gtttttttcc tagtccggga 25560

tcatgtgttg ggttttgttg tgtgtctctt tagccccccc ttgatctgga acaatcctct 25620

gcttttcttt gtcctccatg acattgatat ttttgaagac tgcagcacag ataagttgta 25680

gacagctttg tattcttggc ccaggaacca acaaaagaaa gtttttaaaa aatacttatt 25740

gaaaaattgg gggactgggt gcggtggctc acgcctgtaa tcccaggact ttgggaggct 25800

gaggtgggtg gatcacctga ggtcaggagt tcaagaccag cctggccaat gtggtaaaac 25860

cccacctcta ctaaaaatac aaaaattggc cgggcatggt agcacccgct tgtaatgcta 25920

gctacttggg agactgaagc agtagaatcg cttgaacccg ggaggaggag gttgcagtga 25980

acccagagat caagccacta cactccagcc tgggagcgaa actccatctc aaaaaaataa 26040

aaataaagaa tttgggggag agtaactttc attcaaaagg ttcaatatta gggctgggca 26100

ccgtggctca ctcctgtaat cccacccagc actttgggag gccgaggccg gcagatcacc 26160

tgaggtcagg agttcaagac cagcctggcc aacatggtga aactccgtct ctactaaatg 26220

tacaaaaaac tagccaggcg tggtggagag tgcctgtaat cccagctact tgggaggctg 26280

aggcaggaga atcgcttgaa cctgggaagt ggaggtggca gtgagccaag accatgccat 26340

tgcattccag cctgggtgac tgagcaagac tctgtctcaa aaaaaagttc aatataggaa 26400

agaaatatgg tttttagaaa aagaacacat actttgactt acagacttag atttaaatca 26460

cagttctgct acttaggagt agtgtggctt tgggcaagtt actttatctt cctgactctc 26520

aatttcctca gctctaaaag ggtatacata aaacattggc gtgaagaaat gagaataatg 26580

tatgtaaaca gagcagcaca tgatatatat gtgatgaatg gtaactgaca atattcgctt 26640

tggtataacc tcgcctaagt catgtgagga tgactgctct tgtcatgttt gagggttaaa 26700

tacttctgta gccctagtcc acagaacatg gaaatggaga aaaagccaga gtcagaacaa 26760

ggaaatcaat tggttgtcac attatttcct tgcatgtaaa tagcacctaa gaattccttg 26820

cattttgtaa gtgcctaaaa atcttaacaa ggatatggag acccctctaa gtgtttgtcc 26880

ccatattcaa gttctgcagt atttgatact ttccatttgc tgttcacttt ccaaacttaa 26940

taactaggta cgcaatgcta atgttcaaga aatcatttac taagggacag cttagtattt 27000

gtccatcagt tggcttattg acgtaacctg tgtgctaaat tcactggaat gtggcgtaat 27060

caaatctggc tccttccact tctgagttgg tgcaagaaac acttggcagc tattaaagtg 27120

ctattctttg cagttatttg gtataatttg ccacagaagt aaattccagg aggtaatatt 27180

cttgctaggt tacaccctgt tacctcctga cttaaggagt tcatgctaag ggcaatgatc 27240

tctatctaac tcaaggtgat tctcttggca gccgggaaga tttccagagg attccagaac 27300

ttgccatcaa cccactgggg gaccggatca tcaatgcctt ctttccagag gggtgagtca 27360

gtacagttgt tggacttcct tcctgaggct atcacaacct aaattatttg ctaatctgga 27420

ataatgatat tggttgtgcc atctcagact gtgatacccc ctgattatat tgagcaattc 27480

taattttgtc atttctagaa tatctcttct gttctaccct cctgtgtgtt ttaaacttca 27540

aatagatata tgagcctatc ttcaaaattc ttatgtttat cattgatgta aaactagact 27600

tttaaaaaat gtttatttgg aaataatttc aaatttacaa aaaagttgca aaagttaaaa 27660

gtaattcgga gaatacttgt atatttattg ccctgatgca tctactgtta tattgttaac 27720

attttgcctc atttgtgtgc tctcttctta gtagtagcca ataaaaatat aatgcaagcc 27780

acatacgtaa ttttacattt tctagtaacc acatttaaaa agtaaaaata agttaacttt 27840

ggtaatatat attttattta atatatttaa aatattattt ccatatataa tcaatattaa 27900

aaataactga gatccttttt tcgtttgttt gtttgtttta agacagagtc ttgctcggtc 27960

gctcaggctg gagtacagtg gtgccatctc ggctcactgc aacctctgcc tcccgggttc 28020

aagcgattct cctgcctcag cctccgagat agttgggatt acagccacat gccaccatac 28080

ctggctaatt tttgtatttt tagtagagac agggtttcac catgttggcc aggctggtct 28140

tgagctcctg acctcaggtg atccgcctgc ctcagcctcc caaagtactg ggattactgg 28200

cgtgagccac catgcccagc ctacattttt tcataataag tccttgaaat ccagtgtgta 28260

atttacactt aaacacatat caagtcagac tgaccatgtt ttcagtgcac ataaccagat 28320

gtgacaagtg actgcagtat tgaacagcgc agcttgtaca cccgtgctta ctctacatac 28380

cctctcttgc agacactttt tccctctctc tttcccctct ctctacctac acatactcta 28440

cgtgtgtgtg tatatacata catacatgcc tacatgtacg tacatacata cacatacata 28500

catgtatgta tatacacata cttatgtaat ttttttgtgt atgtgtgttc acataattgt 28560

atttctgaac tatttcaggg taagttgcat agtaatgacc ctttacctct gaatgcacca 28620

gtgtgtgttt gctaagaata gggacattct tttacatatc catagtatag ttatctactt 28680

cagtaaactt aacattgata caatcctctt atctaatcta ttactctgtt ttattaacat 28740

attatccagt ttattctaat ttatattcat attccaattt tgtcatccaa taatctgttt 28800

tatagcactt ccccgcctcc agtacaggat ctagtctggg tcagttgtca tgtctcttta 28860

gcctccttta atctggaaca tttccccagc ctttatttta ctttcgtgat attgaataaa 28920

catagttctc accacatatg ctttgtcaag tagattgttc ttaatgtggg tttttctaac 28980

atgaaactag tcttttgata tgctttacta cctaatacac tgccttcaca gtagtgagta 29040

aatatttact gatttgatta ggtacctttt caacaataaa acttacatat aagcttgttt 29100

taagtaattt cctaaacaat ctattcctgg tttatatgga tagtctatga cccacaggac 29160

ttcaaaatat tatatgggtg ccagttttta ataatgagac tgtcaatgag gacactgtca 29220

gataaaatgc ctggagagga aattttagaa ctctaaattg agagcaaagg aatttgctag 29280

ttgagagggt tgggcattca ggaaatgtga gtgaaagtca gaaggggaat taggtcgggc 29340

agttagttca tgtaaagatg ggaatcaaat cacgttgact atgaggaatc ttatgccctc 29400

tttcattttt tcctttctct ctccttgaac tgccctgttt gtttcaggtg tggtttttat 29460

gctgaattct tccttttttc taattcctct tttacccctg caaagcattg aaccctatcc 29520

agagataact ttcacagcta tgctttgtat ccaaaccttt tgcaatgaaa agtaaaccca 29580

ggagagtcac tttgccaact ttttttcaga attccttagt caccattgat tacttgaaga 29640

agcagcttgt gggaagctgt tttgtaagac taaactacat ttgctgtgac tacaatatca 29700

cctgtcattc tgtccaccaa tctactgcta aggaaatggg acagagtgca tatgaggaag 29760

tggagggctc tactgggctg cactgcttag ctcttagcaa gctgcgactc tgcttcttgc 29820

tctaatcaga tagatatatt tggcttctgg gtttttcttt cttccctagg catatgcaca 29880

ttggggcttt tttttttttt tttttaaaga atgagatttg aaagtggacc atggttattc 29940

aattcccttt tacccagaaa ctttatgcta gcaataatca ttaatgtgtt ttttctagca 30000

tatgttttaa actttactcc acattacttg attcctcaga tctgacattt ttgaccagat 30060

actggtccat tagcagtata gatacatcga agaactaaaa ggtcattctt ttgttttgtt 30120

ttttgttttt ttgagacgga gtctccctct gtctcccagg ctggcatgca gtggtgtgat 30180

cttggctcac cgcaacctct gcctcctggg ttcgagtgat tctcctgcct cagcctcccc 30240

aagtagctgg gactacaggc tcacgccacc acgcctggct aaggtttcac cacgttggcc 30300

aggctggttt tgaactcttg acctcaggtg atccgcccgc cccggcctcc cagagtgctg 30360

ggattatggg cgtgagccac tgcacccagc caaggtcatt cttttgagta cagatggctc 30420

acaggctagc ctttgaactc tgcctttaga agggctatga actgcatgtc tatagagcac 30480

agaagagaac ctacatacct cagctattcg gtggggcaca gaaaggctgg ggtttttttt 30540

ttttttaggt aaccacagga accaggccat ctgccttaga taaataaatt atattatgtt 30600

acatggggca ttctttttta atattaaaga acattcttca ttcaagtttt gagaattatt 30660

tcttacaggg ttattgatta gcctgtggca caatgaaaag attttctcca agcttaagta 30720

ccaaaaaggc tttatacaca agaggtcttt caaaactttt tttttttttt ttttgagatg 30780

gagtctccct ctgtcaccca ggctggagtg cagtggtgca gtctcgactc attgcaccct 30840

ccatctttgg ggttcaagtg attctcctgc ctcaggctct caagtagctg ggattacagg 30900

catgcaccac caggcccagc taatttttgt atttttagta gagacggggt ttcaccatgt 30960

tggccaggct ggtcttgaac tcctgacctc aggtgatcca cccaccttgg cctcttgaag 31020

tgctgggatt atagacgtga gccactgtgc ctggcctaaa agattattta gagaaaaatt 31080

atccaattat gagggaataa taaaatcatg atgtgtccac agaattatgc atatgtttta 31140

aatgtttcaa agtatttata ttgattggag aaatgcttaa aagttaaaaa aaaaggcagg 31200

atactaatac agttatctga aaagcataga aaagttacta gaagaaaatg cattcatata 31260

tatatgaggg agtcttgcac cgtcacctgg gctggagtgc agtggcgcag tctcggctca 31320

cgcaacttct gcctcctgtg tgttcaagtg attctcccac ctcagcctcc caaataactg 31380

gcagtataag cgtgagccac catgcctggc tgattgattg attgattaat tgattgattc 31440

cttccttcct tcctttcctc cctcccttcc ttccttttcc tttccttttc cttccttttc 31500

ctttcctttc cctttccttt cctttctttt tttttgaca tagtcttgct gtgtcaccca 31560

ggctggagtg cagtggtgca atctaggctc actgcagcct ctgcctccca gtttcaagcg 31620

attctcctgc ctcaccctcc cgagtagctg ggattacagg tgcccactac cacgtccagc 31680

taattttttt gtatttttag tagagacagg gttttaccat gttggtcagg ctggtctcaa 31740

ctcctggcct caagtgatct gcctgcctca gcctcccaaa gtattaggat gatgggcatg 31800

gaccaccgtg cccagccaga aaatatattt atattaactg tggttgtaat tggtagaact 31860

cggatcagta tttttctatc tacttttctt ttttccacat tttcaaattt atgtttgctt 31920

ttataattaa ataaacttgg ctgggcatgg tggctcatgc ctgtaatccc agcactttgg 31980

gaggccgagg tgggcagatc atgaggtcag gagattgaga cgatcctagc taacacggtg 32040

aaaccctgtc tgtactaaaa atacaaaaaa ttaggctggg tgcagcagct cacacctgta 32100

atcccaccac ttgggaggct gaggcgggca gatcacctga ggtcgggagt tcgagactag 32160

cctgaccaac atggagaaac cccgtctcta ctaaaaatat aaaattagct gagcgtggtg 32220

gcgcatgcct gtaatcccag ctactccaga ggctggggca ggagaatcgc ttgaaccagg 32280

gaggcggagg ttgcagtgag ctgagatcgc gccattgcat tccagcctgg gcaacaagag 32340

caaaactcca tctcaaaaaa aaaaaaaaaa aatacaaaaa attagccggg catggtggcg 32400

ggcgtctgta gttccagcta gtcaggaggc taaggcagga gaattgcttg aacccgggag 32460

gcggaggttg cagtgagccg aggtagtgcc actgcattcc agcctggatg caagactctg 32520

tctcaaaata aataaataaa gcaagacttt atttatttat aagcagagca agactctgtc 32580

tcaaaataaa taaataaata agaaaaatta aaaattaaat aaactttatt ttaaaagcgg 32640

cctttccata atgtgcctta attttaaaat atcccttggt atacattact gcaggggaga 32700

tgggggagat gtgggaggtg gctgaggagt cattgggctg cctgtggctc gctgcagagt 32760

tcaaagcaat gagttcaagt tctggctgtc ttgtatagtg ctggcactac tctacattca 32820

ttttttggaa aacagatgca agtaaattca atagataact ttatccctgg taagtcctgt 32880

tacctaaatc tggttaaatc atttataaag tgggagtaat agcacctgcc ttacaaggtt 32940

ggggtgaggc ttaaatgata tggtactgtg agtcccaaag taccatgcgt atggaaagaa 33000

acctcattaa atgacctttc aggctaccgt agctaaagct gttgccagta tatttaattc 33060

ataagaaata atcaccgtga ttgacatggt gttgtgtttg ttatatttca caatcagaga 33120

ggaccaggta aacttccgtg gattcatgcg aactttggct catttccgcc ccattgagga 33180

taatgaaaag agcaaagatg tgaatggacc cgaaccactc aacagccgaa gcaacaaact 33240

gcactgtaag gagctaaagt cttctggctt aaaatacttg cttttcattt cttaaaagtc 33300

atgtatttac tcactcatta tttgaacaag catctacaag agcatactgg ttaagacctc 33360

agactctgga gctaaactgg ccaagattaa atactagtct accctttttt ggtcgtgtga 33420

ctttgggcaa ggtacttaac ctctttgggc ttcagttttc tcttctgtaa aataggatac 33480

taaggggttc cacattggat tgttgtgagg attaaatgag ttaataaatg tcaagtgctt 33540

cacatagtgc ctggcatata ctaggtactt aataagtgtt acctagtatc attataattc 33600

attgagcacc taccgtgtgc caggcactgt gttaagtgct agcatggcaa tagtgaacaa 33660

gacccaggcc ccacctttaa ggaataatgg caatcctcag ctcatcttag aatttgatag 33720

acactggatg tgacttaagt aatgggggac agctggatac agtgcaagat ttaattttta 33780

tacccgtgtc tcatgcctct tttcctttaa tacttgtttg ccttcccttc tgcccttatt 33840

ggttcatgta caatattgcc caatccctta caacacttct ccaggaaatt gaaattaaat 33900

ttatcttttt attcagtaca agttgaaatt acagttgtat taaaactgac tgaaggctgg 33960

gcgcagtggc tcatgcctgt aatcccagca ctttgggagg ccaaggtagg tagatcactt 34020

gagcccagga gtttgagacc agcctgggca acatggtgaa acattgtctc taataaaaat 34080

acaaaaatta gccaggtgtg gtggcaggca cctgtgctcc caggtacttg ggaggctgag 34140

gtgggagaat tgcttgagcc tgagaggttg aggctgcaat gaaccttgat ggcaccactg 34200

cacttcagcc caggtgacag aaactctgtc tcaacaaaga aaagaaaaca aaacaaaatc 34260

ttcgaaatag cactagtgga tttgggcgtt acatggtgtc ttaggatacc tttagctgac 34320

tcagctggct taaacaatag ggaactatat tagctcactt tacaagtcag attaggccgg 34380

caggagggtt gattctgttt caagaactag ggatttttct ctttactctg ccatcctcat 34440

agtctgtttt atcctaaagt attatggtca catggcagct ctcagttgtt aacagggctt 34500

ttttttttct tgttcacatc tggcagtaga ggaagagaga tatatataga gagagatcga 34560

gactgaaatt gagcaacact cctcccaagc atggaatgta agtccttccc ctcactttga 34620

taagtcagtt caggtcagga gagtgcgagt gccgaggaga ttaggctaat gagattcccc 34680

tcctgatgct gtgattgggg ttctgctaag gaaaaaggtt ggaatgccac agaaatggca 34740

gacttcagga tgagatagaa taataacaaa gtgctatgca ggttatgctt ggttttattt 34800

tgtttttgct taattttttg agatagggtc tcactctgtc acccaggctg agtgcagtgg 34860

cgtgatcaca gctcactatg gccttgactt tcctggctca agtgatcttt ttgcctcagc 34920

ctctcaagtt gctgggacca caggcgtgtg cactacaccc agctaatttt ttattttcta 34980

attttttgta gggacagggt ctcactatgt tgcccagact ggtctcgaaa tcttggacgc 35040

aagcagtcat cccaccttgg cctcccaaag tgctgggatt acaggcatga gcctccatac 35100

ccagcctgtt tttttcatta catactttag tcttggctgt tggattaact tttataggaa 35160

gctaaaaatt aattagaatt ctgttgcagt ctatgtatag ggatcacatt catgtaatta 35220

gaccagtaaa accgaagcta ttctaatgcc aaagaaaaga ctagaagagt ccgagaagtc 35280

ctcaatttct gtctaaatgt gttgcttctc tttgaagttc ccacagggct gtataagctg 35340

tagcatggtg cctcttactg tagactgaga gcaattgaat gtcatatacc tagcaataca 35400

tttgccactg agacacattt accttagaga aaggggatgt gggccaggtg cggtggctca 35460

cgcctgtaat cccagcactt tgggaggctg aggcaggcgg atcacctgag gtcaggagtt 35520

cgagacaagt ctggccaaca tagtgaaacc ccatttctac aaaaatacaa aaaaaattag 35580

ccaggcataa tggtggatgc ctgtaatccc agctactcag gaggctgagg cgggagaatt 35640

gcttgaacct gggaggtgga ggctacagtg agccgagatc atgccattgc actccagcct 35700

gggtgataga gcgagactct gtctcaaaaa aaaaaaaaaa aaaaaaaaaa aaaaaaagga 35760

gatgtgttac ttagaaatct gggtttacag gccaggcatg atggctcacg cctgtaatcc 35820

cagcactttg ggaggccaag gcgggtggat cacttgagat caggggttca agaccagcct 35880
ggccaacatg gtgaaaccct gtctgtacta aaaatacaaa aaattagctg ggcgtggtgg 35940
cgggcgcctg taatctcagg tactcgggag gctgagacag gagaatactt tgaacctggg 36000
gggcagaggt tgcagttagc cgagattgca ccattactct ccagcctggg caacagagca 36060
aaactccatc tcaaaaaaaa aaaaaagaaa tgtgggttta cagaggagtg gaaccaaatt 36120
ttattatggg ttatactttg catagaatac tgtcttcagg aactctgaga acacactttg 36180
ttctatttct tttctaagcc acggtgagag aattttaatc acgatcagca aggctgaact 36240
atgcccatgt aggcacaggc ccaagcacac atagtgccac tctgctcttc tgtttgtctc 36300
ctggaagtgg taccttattg ctttcctctt tggattggtg aggttataat aatgctttct 36360
gtaggccagg cgcggtggct catgcctgta atcccagcac tttgggaggc cgaggtgggt 36420
ggatcacctg aggtcaggag ttcgagacca gcctgaccaa catggtgaaa ccatgtctct 36480
tctaaaaata caaaattagc caggcatggt ggtgcatgcc tgtaattcca gctactcagg 36540
agactgaggc aggagaattg cttgaacctg ggaggctgat attacagtga gccgagatca 36600
cgctattgca ctccagactg ggcaataaga gtgaaactcc atctcaaaga ataataataa 36660
taatagtaat aatagtgctt tgtgtaaaag gtacaaccct atctacaggt ggatagtgtt 36720
taaaaaaaaa aaagtgaaag gtataattca tcttaggccc agggcatggt aagtaagaga 36780
gaagtctaga ggtctacagg ttttccttcc cacattctaa ttgtcttctt aaatggttta 36840
agtttttata acacataatg atgtggaaag taaagagtct acattagaat atttttcttg 36900
ggtgttgagg agtataggcg gagtagagct tgggagagta gccaggaaga aatagcagca 36960
ttattcaaag aatccaaaag gtagaagcaa tcctagtgtc catcgacaga tgaatggata 37020
aataaaatgt tgtgtacaca tacaatgaaa tattagtctg tattaaaaaa gaaaattctg 37080
aggccgggag cggtggctca cacctgtaat cccagcattt tgggaggcca aggtgggtga 37140
atcatgagat cagaagttcg agaccagcct ggccaatgtg gtgaaatccc atctctacta 37200
aaaatacaaa aattagcctg gcatggtggt ggatacctgt aatcccagct agtcgggagg 37260
ctgaggcagg agaattgctt gaacccagga ggcggaggtt gcagtgagct gagattgcgc 37320
cactgcactc tagcttgggc gacagagcaa gactccatct cgaaaaaaaa aaggaaattc 37380
tggcaaatgg tacaacatgg atgaatcttg aagacattat gctgagtgaa ataaagtgga 37440
tataaaggac aaatattgta tgattcctct tatgtgaggt ttccagagtg gtcaaatttg 37500
tagagacaga aagtagaacg gtggttgccc agggctgggg gatggtggtg gtgatggtaa 37560
tggagttatt gttaaaaggg tacattttcc gctagtgaag atgcagaagt tatggggaga 37620
tggatggtgg tgatgcttgc acaacagtgt gaaagtactt aatgccacgg aactgtacct 37680
gtagaaaagg tttaaatgat acattttatg tatattttac cacaataaaa aaaaaagaag 37740

cagccgggca tggtggctta ttcctgtaat cccaacactt tgggaggcca aagtaggcag 37800

attgcttgag gtcaggagtt tgagaccagc ttggctaaca tggtgaaacc ctgtctctac 37860

taaaaataca aaaattagct gggtgtggtg gcacatacct gtagtcccag ctactcagga 37920

ggctgagaca ggaaaattgc ttgaacccgg gaggcagagg tttcagtgac ctgagatcgc 37980

gccactgcac tccagcctgg gtgacagagc aagactccgt ctcccaaaaa gaaaaaaaaa 38040

aaagaagcaa tagcattgat tgaagtaaag gtaaaggttt gtagcctggg caacagagcc 38100

tgggcaacag aacctgggca acagtatcta caaaaaaaaa aaaaattgag ctgggcatgg 38160

tggcacatgc ctgtattcct agctgctcag gaggctgagg caggaggatc acttgagccc 38220

agaagtttgg gtctgcagtg agccatgatt gcaccactgc actccagtga cagagtgagg 38280

ccctatctct taaaaaaaaa aaaaaaaaag cataaaaaag gaaggtaaag gtttgactga 38340

gatcattcct ttggtttttc attaaacaaa tgtttgagca cctactacat gccaggtact 38400

attttaggcc caggatacag tgatgaataa atcaggtaag gcccttgctt tcttagaact 38460

tatgatctaa taagagagac agactataga gaagacgggt gaataaacaa gatttattgt 38520

ggtgacaagg tctgtgaagg aagtgaatac atgagatgag aaaggcatag ggagatgtca 38580

ctatagaaag agtggtcagt gaaaacctct ctgaggagtt gaaatttgag ctgaaggccc 38640

aggcacagtg gcttacacct ataatcccag cactttggga ggctgaggca ggaggattgc 38700

ttgagcccag gtgttcaaga ccagctgtct gggcgcagtg gctcatgcct gtaatcccag 38760

ggctttggca ggctgaggca ggtagatcac aaggtcaaga gatcgagacc atcctggcca 38820

acatggtgaa accccatctc tactaaaaat acaaaaatta gctgggcgtg gtggcacatg 38880

cctataatcc cagctactcg ggagtctgag gcagaattgc ttgaacccgg gaggcggagg 38940

ttacagtgaa ccgagatcat gccactgcac tccagcctgg gcgacagagc aagactccat 39000

ctcaaaaaaa aaaaaaattc aggccaggcg cgatggctca cgcctgtaat cccagcactt 39060

tgggaggccg aggtggttag atcatgcggt tagatcatga ggttaggaga tcgagaccat 39120

cctggctaac acggtgaaac cctgtctgta ctaaaaacac acacacaaaa aattagccag 39180

gcgtggtggt gggcgcctgt agtcccagct actcgggagg ctgaggcagg agaatggcgt 39240

gaacccagga ggcggagctt gccgtgagct gagatcccac cactgcactc cagcctgggc 39300

aacagagcaa gactctgtct caacaacaac aacaaaaaac aatttatcta gacaaagacc 39360

ttgcaccttt caccagcctg ggcaacacag tgagacccta gctctacaaa aatttttaaa 39420

aattagccag gtgtggtgtc tgtggtccca actactctgg aggctgaggt gagaggatcc 39480

tttgagcctg ggaggttgag gctgtagtga gtcatgattg tgctactgca ctccagcctg 39540

ggagacagag caggaccctg tctgaaaaaa ataaataaat aaaataaaat aaaccaacca 39600

aattaaacag agcagaaaaa aaaatacata tatatgaagt ctctcctagc tacttccttt 39660

ctctggaatt ctgatatata ccttacaagg agtaaattag attttaaaat tacttttatg 39720

tcatcttagt ggactcacca ttttattttg gaggggagtg atatttaaac ctgtagatat 39780

taatccatgt tcttatgcct ttgtgcctaa taatcgctct gcctggaatg accacccctt 39840

gtctcatttc tgtttgtctt tcaaggcctt gttcaaatgt tacctctcct tgatatttcc 39900

ccagctgcac cagaaagcca cgtctgccat acactttgtt cccccagcat tttggatatg 39960

ttgtagtgct taggatgtac tcgagagtcg tgtgtcttgc cctcctctac tagatagtga 40020

gttcctaagg acagagtcta aatccatgga tcttaatagc tctagagcag tggattcggg 40080

ccttttagta tttgcagcac tctatttaag acccttaatc ttagtggcat tcttgaagtg 40140

attcaaaacc taaaaaccaa gctaaattca gttgtgggac aagacattca catcctcagg 40200

ctggcgattt gtcacggcac ctataaaatc acccagtgtt atcaccctat tctcttggta 40260

gcccttcagc aacatggcag aaatctaatg attttgcttt ctgattccta tctcatggac 40320

ccaagcaaag ttctcctatc tcctagtttc tagaaggctt aaagagaaag aaacatgata 40380

aagccaggag accatctgag ccatcatatt tatttgaaac tcaatttctt tacctccagg 40440

actcccaagt ggtagagaat tacttgaaat ttgagacaat tttgcccagg aggctctatt 40500

cccaaagctg tggtcagctc tcatggtgcc tgtgtggtgc ctattgttgg ttttccctga 40560

gtggtacctg gtagatttga tgtttgtgtg aggtgaccta atttacagaa ccacgtaagg 40620

agtgttttgg gggggggcgt ttaacattct aggttaagaa aaaggacatt tggctttctt 40680

tgtatgaagt tttctgattt gttggaagag ggaccatact tcatctaatc taagaactca 40740

tcaattctca attcttcagt gttacaactt tttaaaagcc ctacaagtaa tggcaattgt 40800

tcttggaaaa tcgattaatt ggctgtcagt tttctgtcta gtgtcttata tatttagttc 40860

cttgagaagg ggcaacacac tacagaattt tgactaactt tgtgtttttc ccttacagtt 40920

gcttttcgac tatatgattt ggataaagat gaaaagatct cccgtgatga gctgttacag 40980

gtatgtggag agctcctcga gaacttgtgc ttggactctg cctgcttatt agtgtgggca 41040

ggaactattc tttcctttag taagcattta tttaatacct gctatgtgca gagtcctgtc 41100

ctggttataa gacaaaggag atgacctggg aaaacttgaa atggctcatt gtgttttcat 41160

agacagatgt ctccactatt taaaagctaa ctaggctggg catggtggct cacgcctgta 41220

atcccagcat tttgggatgc caaggcaggc agatcacctg aggtcaggag ttcaagggca 41280

gcctggccaa catggtaaaa acccatttct agtaaaaata caaaaattag ccatcggccg 41340

ggcgctttgg ctcacacctg taatcccagc actttggaag gccgaggcag gtggatcacg 41400

aggtcaggag atcgagacca tcctggctaa catggtgaaa ccctgtctct actaaaaata 41460

caaaaaatta gccgggtgtg gtggcgggca cctgtagtcc cagctactcc ggagactgag 41520

gcaggagaat ggcatgaacc tgggaggcgg agcttgcagt gagcagagct cactccactg 41580

cactccagcc tgggcgacag agcaagattc cgtctcaaaa aaaaaaaaaa aaaaaaaaaa 41640

ttagccgtct gtggtggtgg gtgcctgtaa tcccagctgc tcgggaggct gaagcagtag 41700

aatcgcttga acccaggagg tggaggttgc agtgagccaa gatcgtgcca ctgcactcca 41760

gcctgagcga caagagtgaa actccatccc aaaaaagaaa aaaataagta aataaataaa 41820

agctaactaa atggctttta ggtactgtct gagatttaca tcagataatt cagttattgc 41880

caaatcagat tattggcagc caggtaattc tgatgacact tagttaaatg ctgttattta 41940

gtctgtcctt agggttgact aagagtcaag gagctgctgc tgtgctggtg taccctgagc 42000

taactgccac tgttctttgt tgctcctagg gataattccc tgttttccta ctagcagtga 42060

ctggcatttc atcctaggat gaaattactc agggcagatt caggagcaac ccaacgtatt 42120

cttacatttc tatcagcaaa aaccaaaaca gtcttcaggt cagtgttagc gtcccttgct 42180

tttgagttgg gaatttcttc agcgagaagc gtttattttc ttgaaagttg ccacatatgg 42240

gcttctgtgt tctgaagaaa aaaagacagt agcagtcact ctatagttgc atgtgtttat 42300

gctctggccc agatctgacc tcaggttatc cacccgcctc ggcctctcaa agtgttggga 42360

ttacaggcgt gagccaccac gcccggccca tgttttctta atatgattct tgtgaaagtc 42420

ctggttgaac ttttggaggt cattatcttg attgtgagga tggtgccatg ggtgggtaca 42480

tatgttaaac cttatccaat tgtacatttt atttatttat ttatttattt tgtattttta 42540

gtagagacgg ggtttcagca tattggccag gctggtctcg aactcctaac ctcaagtgat 42600

ccacccacct cggcctccca aagtgctggg agtacaagca cgagccatca tgcctggccc 42660

caactgtcca ttttaaatat gtacagctta ttgtatatta tttatacttc aataaagata 42720

gttactcttc tccccaactc attttatgag gctagcgtaa cttttgcctg agaatttttt 42780

tgttttgttt tgttttgttt tttgagagag tctcactttg tcgcctgggc tggagtgaat 42840

atcaccatct cagctcactg caccctccgc cacccaggtt caagcgattc tcgtgcctca 42900

gcctcctgag taactgggat tgcagacatg caccaccata cccagctaat ttttgtgttt 42960

ttagtagaga tggggtttcg ccatgttgac caggctggtc tcgaactctt gggctcaagc 43020

agttcgccca cttcagcctc ccaaagtgct gggattacag gcgtgagcca ccgtacccag 43080

ccacacctgt caatcttata gaagacagcg taagaaaaga aaattagggg ccaggcgcag 43140

tggctcatgc ctgtaatccc agcactttgg gaggccgagg caggtggatc ccgaggtcag 43200

gagatcgaga ccatcctggc taacatggtg aaaccccgtc tcccctgtct ctactaaaaa 43260

tacaaaaaat tagccgggca tggtggtgga cacctgtagt cccagttact cgggaggctg 43320

aggcaggaga atggtgtgaa cctaggaggc ggagcttgca gtgagccgag atcacgctac 43380

tgcactccag cctgggcaac agggcgagac tccgtctcaa aaaaaaaaga aaagaaaaga 43440

aaattatagc ccaatctctt ttactcacat agttgtaaaa atcctaaaca aaatattaac 43500

acactgaatc cagcaatata aaatataata tatatatatt taaagccaaa gatgtattaa 43560

agccaaggat gtattatagc caaggtgcaa ttatactaga aattcaaagt tggtttaaca 43620

ttagaaaatc aaccaatata tttcacccac accaacactt taaagaagaa aaactgagtg 43680

gtcttttcca tagaaaaatg taattgataa aattcaccat ccattcattt tattttattt 43740

acttatttat ctttttttct ggagacggtg tttcactctg tcgcccaggc tggagagcag 43800

tggcgtgatc tcagctcact gtaacatccg cctcccaagt tcaagcgatt ctcctgactc 43860

agcctccagt gtagctggga ttataggcat gcgccaccac acacagctaa ttttgtattt 43920

ttagtagaga cggggtttca ccatgttggt cagggtggtg tcgatctcct gacttcaggt 43980

gatccacctg cctcggcctc ccaaagtgtg tgagccagct tgcctggcct caccatccat 44040

tcatgattta aaaaaaaaaa aaagaagtct tagaaaatca gaagtagagg gaacctttct 44100

taaatcgata aagaatatat atataaaccc tcaccaaaca ttaaaaataa tagtaggctg 44160

ggtgtggtgt ttcactcctg taattccagc tactcgggag gctgaagtag gagaatcgct 44220

tgaaaccagg aggaggaggt tgcagtgagc tgagatcatg ccactgctct ccagcctggg 44280

cgacagagca aaactctgtc ttaataattc actaataaac ttcttttttt ttattttttt 44340

gagacggagt ctcgctctgt cgctcgggct ggagtgcaat ggcgcgatct caactcactg 44400

caacctccgc ctcccgggtt caagtgattc tcccgcctca gcctcctgag tagctgggac 44460

tataggcacc tgccaccacg cctggctaat ttttgtattt ttagtagaga cggggtttca 44520

ccgtattggc caggctggtc ttgaactcct gaccttgtga tcagcccacc ttggcctccc 44580

aaagtgctgg gattacaggc atgagccacc gcacctggcc taataacttt tgactggaac 44640

aataagtggc aacatggaca ggtagaggga gcactgagct gggaatggag gcctgggcag 44700

atagctcatg ccactggttc ctgggacctg agtcaagcca cctctgggct gtttcatgtc 44760

cttattaaca gatcggatga gataaccttt tttttctttt ttgagacagt ctcactacat 44820

ttcccaggct ggcttgaatg cttaggctca agtgatcctc tggccttagc cttccaagta 44880

ggtgggtcca gtagctggga ctacatgcat gtgtcactgc acctggtgaa atgatgttaa 44940

agacaccact acttttatcc cgaccctccg actcacacta tagataccca tacataatcc 45000

agttttctaa ttctacaaat aaaacacctc agaaaaattt ggtctgggat ctgaagaagc 45060

tgctgtctga ggttggattc atctgcttgg caacacacag ggaatgtggc aaatgggttg 45120

tctattgatt atctttgcta gcattgtctt tgtttttttt tttttttttt gagatggagt 45180

ctcgctctgt tgcccaggct agagtgcagt ggcatgatct cagctcactg cgacctccac 45240

ctcccgggtt caagtgactg tcctcccata gcctcctgag tagctgggat tataggcgcg 45300

cactaccatg cccggctaat ttttgtattc ttagtcgaga cgggtttcac catgttggtc 45360
aggctggtct ccaactcctg acctcgtcat ccacctgcct cagcctccca aagtcctggg 45420
attacaggcg tgagccacca catccggtgc attgtctctt tttattaatt cttctattgg 45480
ggtacatttg tacctaaaat gtacaacttc caattttctt ttcttttttt tcgtttgaga 45540
gggagtgtct ttcttgtcgc ccaggctgga atgcaatgga gtaatctcgg ctcactgcaa 45600
cctccacctt ctgggttcaa gcgattctgc ctcagcctcc caagtagctg ggattacagg 45660
tgcccgccac catgcctagc taatttttgt atttgtagta gagatggggt ttcaccatgt 45720
tggctaggct ggtgtcaaac tcctgatctc agatgatctg cctacctcgg cctcccaaag 45780
ctttgggatt acaggtgtga gccaccaccc ccggccaaaa cttctgattt tcaagaattg 45840
gtttatttaa aaaataatca gtttggatac aaatcagtat ttcagctttt tttcttttta 45900
gaaaatcatg taatttacta ttgatggatt ggttgattga ctgatagggt ctccctctgc 45960
tgcctactct ggagtgcagc aactattcac aggcatgatc atagcacact gcagccttga 46020
actcctgggc tcagaccatc ctcctgcctc agccgcctga gtagctggta ctataggcat 46080
gcactaccac acctggcttg taatttcgct cttgttgccc agcctggagt gcaatggtgc 46140
aattttggct caccacaacc tctgcctccc gggttgaagc gattctcatg cctcagcctc 46200
ccgagtagct gggattacag gtatgcacca ccacgcccag ctaattttgt atttttagta 46260
gagatggggt ttctccacca tgcccggccc atgaaatcga tttttaaaaa gtaaattaca 46320
ggccgggcgc agtggctcac gcctgtaatc ccagcacttt gggaggccga ggcaggcgga 46380
tcacctgagg tcgggagttc gagaccagcc tgaccaacat ggcaaaatcc cgtctctact 46440
aaaaatacaa aaattagcca ggcatgctgt tgggcacctg taatcccagc tactcgagag 46500
gctgaggcag gagaatcgcc tgaacctggg aggtagaggt tgcagtaagc caagatcgtg 46560
ccattgcatt ccagcctggg caacagagtg agactccatc tcaaaaaaaa aaaaaagaat 46620
ctatttcatg agattgtaga agggagtaaa ggagtggagg aaaggaagtc tatgagccac 46680
tgggctagag gccctttcct ttctgtggaa aagctcagtt cttgccatct gaagacagag 46740
gtggctgact gcaataataa tataaggagt cagaaaggta ccctgtctcc catttcatat 46800
tgcctaagaa ccagcctttc catattgcct gacagtcaaa ttgttaaagt tgcttatttt 46860
gtttaaggaa gcaattttca aagtatgttc acaccctaaa ggtgttaata gaattcccag 46920
gggcccaagg attggttaag tttaagacag atttggtgaa acagtgttaa attctcttac 46980
tcaattctcg cagcctttta attgtgcttg ttatttttct cagaagggga tttagcattt 47040
aactcttcaa ggatttttat gttttcttgg gccatacttt aggaatagtt ggcttaatgc 47100
agcacttcga agaggacgac attttgggtg ggatttcaga aagccccacc aaactgattt 47160

aaatcaataa ggaactagtt aggctcacgc aacaagaatg tttggcatag attgtccagg 47220

ctggtacagt acctaactga tgtccaggaa ccaggcttat tctgtgttcc tgccctggca 47280

tccatagctc atattactca cctcatggct ccagggtggc tgcagtgcca ccagacagaa 47340

tgaggtaaga agaatgagga agggcaaaat gcaaaggact ctcccttagt ctttgtcttc 47400

ttatccagtc acctagataa gttctgctta catcattgcc tagatgagaa gataaaggtt 47460

tcagcctttt agctgttact tagtcacatg atggtaaaga tggggataca ttctgagata 47520

tgcaatgtta ggcaattttg tagttgtagg aacatcatag aatgtaccta catgaaccta 47580

gatggtatag cctactacac acctaggcta tgtggtgtgg cctattgttc ctagactaca 47640

aacccgtgca gcaagttact gttctgacta ctatatgcag ttgtaacata atggtaagta 47700

tttgtgtatc taaacatatc tgtggctggg catggtggct cacgcctata atctcggcac 47760

tttgggaggt ggcagaggga gaattgcttg agcccaggag ttgaaggcct gcctgggcaa 47820

cacagtgaga cctcattcct acaaaaaaat tttgaaaaat tagccaagtg gctgggcgca 47880

gtggctcaca cttgtaatct cagcactttg ggaggcggag gcgggcagat catgaggtca 47940

ggagttcgag accagcctgg ccaacatggc gaaaccccat ctctactaaa aatacaaaaa 48000

ttagctgggt gtggtgtcac atccctgtaa tcccagctac tcgggaggcc gaggcagggg 48060

aatcgcttga actggggagg cagaggttgc agtgagccga gatcgtgcca ttgcactcca 48120

gcctgggcaa caagagtgaa actccgcctc aggaaaaaaa aaaaaaaaag gcatggttgc 48180

acacacctgt agtgtagtac cagttccttg ggggaggctg aggtgagagg atcacttgag 48240

cttcggaggt gaggctgcag tgagccacat tcgcaaccca gcactccagc ttgggcaaca 48300

gagcgagacc ctgtctcaaa aaaaaaaccc aaaaacaaat acttacatct aaacatggaa 48360

aaggtacagt aaaaatatgg tattgtaatc ttttttaaca aattttttgg ccgggcgcag 48420

tggctcatgc ctgtaatccc agcactttgg gaggccgagg tgagcggatc acgaggtcag 48480

gagatcgaga ccatcctgtc taacatgctg aaaccccatc tctactaaaa atacaaaaaa 48540

ttagcctagc cgggcacggt ggcaggcgcc tgtaatccta gctacgcagg aggctgaggc 48600

aggagaacgg cgtgaacccg ggaggcagag cttgcagtga gctgagatcg tgccactgcc 48660

ctccagcctg ggcaacagag tgagactccg tctcaaaaaa aaaaaaaaaa aaggaaaaaa 48720

aatttaactt ttaggttcag ggctacatat gcaggttagt tatgtaggta aactcttgtc 48780

atgggggttt gatgtacaga ttatttcgcc acccatgtac taagcctagt acccagtagt 48840

tattttttct gtttctctct cacctcctac tccaccctct ggtaggcccc agtgtctgtg 48900

gttccccttt ttgtgtctat gtgttctcat caaaatacag tattataatc ttatagcacc 48960

accattatat atgcagtcgg tcattaacca aaacattatt atgcagtgca tgactgtatt 49020

tgagggcatc aaaggaaaag gaggatggtg atagctttat ttttaatctt agtaaagagt 49080

ccctctgatt cccaaggccc ttgtaattcc tggctcttgg tcttccaggt gctacgcatg 49140

atggtcggag taaatatctc agatgagcag ctgggcagca tcgcagacag gaccattcag 49200

gaggctgatc aggatgggga cagtgccata tctttcacag aatttgttaa ggttggtcac 49260

ttacttcttg tttgaaaaag ttacgtttta gtggctgggc gcggtggctt acgcctgtaa 49320

tcccagcact ttaggaggcc aaggtgggcg gatcacgagg tcaggagttc aagaccagcc 49380

tggctaacat ggtgaaacct catctgtact aaaaatacaa aaattagcca ggcgtggtgg 49440

cgcgtgcctg taatcccagc tacttgggag gctgaggcag gagaatcgct tgaacccagg 49500

aggccaaggt tgcagtgagc cgagaccaca ccactgcact ccagcctggc aacagagtga 49560

gactctgcct ccaaaaaaaa aaaaaaaagt tacgttttac tgcacaggag gaagggggga 49620

aaacattact cagataagag cttgggagtg ttgtaatctt cataaccttt gtaactgtta 49680

ctggttttct cccccaggtt ttggagaagg tggatgtaga acagaaaatg agcatccgat 49740

ttcttcacta aaggagacca aactgttcct tgcggtctag tatttaagaa ctggaacttg 49800

aaagtcctcc ttctaccaac tccacctcca ccccctcatt ccccttctcc caaagtacta 49860

ctgctgttgc atgacaaccc caaatatgtt ctgtcaacac aaacctgcct ttggtgtata 49920

aacagggcat tacagaatgg tacaccctat atatttctgt tcagtatcca ttcactagtt 49980

cttcatttat aaatatcatc ttccccattc tgctgctgaa tgccacacat ccatccagtc 50040

tgagaaagtg agagaggcaa tcatgccaag aacaagccag caaagctctt tcaccagatg 50100

tagactgtag ccctgctgcc ttccctccag cgagtctgcc agcatgcttc ttcatccttt 50160

ttatatgttc tttgcttcct acttccctgt cttccaacat actgttcact tactctggca 50220

gtctttctgc ttttcattaa gcctcaaaat ctcctctgtt ctacttggca ccacaagcta 50280

tgtcctatat atgtatttct gacttggcag gatagttcag gggtctggca gtttttattt 50340

accttcatta ttaaatgggc ctctgggatg ttgcctcttc aggagctttt tggtaatcaa 50400

tacttctctc agaagtatga gaccatcctc tgcactctgc tctgtcatca aaggctgctg 50460

ggtggagata ccctttttga aaggtggcct tggtgagagg tatggagcca agtcttctag 50520

gttgcttgcc cacatcactc tatctctggc ctctgattct caactttgta cctgtgtggc 50580

tcctcttgtt agtgcaatgt tgactgttga aaaagcagca gtatgcttac aggtttgctt 50640

agtttgggga caccgttacc accagaatgg ctgctctgac aatatgccta gggactttct 50700

catggctttt atttaataag gaggctgggc accctataaa gcctcatgca ttcacacctt 50760

tgcagcatgg tttatgcctc agtgttatgt gcactggaat gttttccact tcacatttcc 50820

aagtagaaat attagtgtta cggaagtgcc taatatccca gtccaaattt tttttttttt 50880

tttttttttt ttttgagaca gagtcttgct ctgtcaccca ggctggagtg cagtggtgcg 50940

atcgctcact gcaacctcag cctcctggat ttaagtgatt ctcctgcctc agcctcccaa 51000

gtagctggga ttacaggtgt gcaccaccat gcccggctaa ttttttgtat ttttagtgga 51060

gacagggttt caccatgttg gccaggctgg tctcgaactc ctgacctcgt gatccgcctg 51120

cctcagcctc ccaaagtgct gggattacag gtgtgagcca ccacgcctgg ccccagtcca 51180

aaatatttaa agattgtttc cttagtgtct tgaagttttg cacaaaattc ttttttttga 51240

gatggagtct cactctgtca cccaggctgg agtgcagtgg cgtgatcttg gctcactgca 51300

acctctgcct cctgggttca agcaattctc ccacctcagc ctcccaagta gctgggatta 51360

cagacgtgtg ccaccatacc tgggtaattt ttgcattttt agtggagagg gagtttcacc 51420

atgttggcca ggttggtctt gaactcctga cctcaggtga tcctcctgcc tcggcctccc 51480

aaagtgctgg gattacaggc atgagccacc gtgctcagcc gcaaaattct ttatgaattt 51540

tacacttggc aaatgttaat gacggaagcc atagtctgct cctaatacat gtccaaagca 51600

ttgactgttg tgtcattagc tgcctggtta cattagctcc ctggcttctt gtttagacca 51660

ctgctaatcc cttaaaaaca agaggtctgg cactagtagc acaacctaag gtggcattac 51720

agatctttga gcgagccaca gcaacttttc tgccaagtca gcttagttta gacttcagtg 51780

aatcaggcta ttgctatcct aatgtatgtc tctatgagtg tatttagcca cacatctgcc 51840

cttggttgac tttctgactc attgcttgct tgcttgtttc cttgctttgg aaaactattg 51900

aagattgcta aaaaatacca ctgcaaagtg atggaaaagg gtggagaaca ggggagtagc 51960

caggctggat ggctcaaata taaatgaatg aggaattctt tatgaagtat cagtcagatt 52020

ttatgattaa gtgatgtaat ataggaatta tgtaaaaggg aagaatgtct gatactgatc 52080

tattagagag gtactttaga ggcttcttga ttggcataaa gttcctaagg ttatagattt 52140

tccccccttt tggctgtata gcaaagtgtt ttaatccacg gttgtgcctt attgttccat 52200

taaaattgta tcttcgatcc atcaataaat acttgtggtt gaaacaaaat aacttttgtc 52260

cattttacaa gaattttatg taacttagtc ccttcaagtt tgaaatcaca gtgtctccct 52320

tactcaagtt tatgtctgta cggtcagctg cagtctactc tgctgctgcc tggatatatg 52380

aagtggaaac attcatcgag gggccagcac ttttggcttc gacatggttt tgtcagccgt 52440

ggccacttcc cattcattcc agctaagacg caatttctat tcttgactcc ctgactcctt 52500

aaatgtagaa aaagcctaaa aatccagtat atgtccattc cttaaattgc ccacctcctc 52560

attcatgtgc agcctcagcg gtgacatact ctggttttac atggcttttt ctttgcattt 52620

cttttttgag atggagtctc actcttgccc aggctggagt gtaatggcac agtcttggct 52680

cactgcaacc tccgcctccc aggttcaagc aattctcctg cctcagcctc ctgagtagct 52740

gggattacag gcacgtgcca ccacacctgg ctaatttttg tatttttggt agagattggg 52800

gtttcaccat gttggccagg ctggtcttga actcctgacc tcagctgatc cacccgcctc 52860

agcctcccaa agtgctggga ttacagccac cgtgccaggc caggaatctg catttctaag 52920

aatccaccca gatgacagcc ctcttggtgt ttggcaagca cagtggacct acactgccta 52980

cgctgggcct cgcttgtcat aaccccgaag aggagccacc ccacgtatac cctccctgct 53040

gtcacgtgtc cgtgtgaaga gaccaccaaa caggctttgt gtgaacaaca agcctgttta 53100

tttcacctgg gtgcaggtgg gatgagtcgg aaaagagagt tcagcaaagg gtggtggatt 53160

atcattagtt cttacaggtt ttgggatagg cggtggagtt aggagcaatg ttttgtgggc 53220

agggggtgga tctcacaaag tacattctca agggtgggga gaattacaaa gaaccttctt 53280

aagggtgggg agattacaaa gtacattgat cagttagggt ggggcagaaa caaatcacaa 53340

tggtgaatgt catcagttaa ggctattttc acttctttgt ggatcttcag ttgcttcagg 53400

ccatctggat gtatacgtgc aggtcacagg ggatatgatg gcttagcttg gg 53452

<210> 21
<211> 3329
<212> DNA
<213> Artificial Sequence

<220>
<223> CHP1-001 cdna:KNOWN_protein_coding

<400> 21
cattcggggc tggaactcga acgctgacgt tggctcctga gccgggcgct gcctcgcgcc 60

tgattggcgg gtcctcgggg ccgccctccc cagcgcacca cccctgggtt ccctcccggg 120

tccgcagtgg aaacactgcc ctctcccttc ttgaccccta gcccttcctt ccctccctcc 180

ttccctcctg tcgccgtctc ttctggcgcc gctgctcccg gaggagctcc cggcacggcg 240

atgggttctc gggcctccac gttactgcgg gacgaagagc tcgaggagat caagaaggag 300

accggctttt cccacagtca aatcactcgc ctctacagcc ggttcaccag cctggacaaa 360

ggagagaatg ggactctcag ccgggaagat ttccagagga ttccagaact tgccatcaac 420

ccactggggg accggatcat caatgccttc tttccagagg gagaggacca ggtaaacttc 480

cgtggattca tgcgaacttt ggctcatttc cgccccattg aggataatga aaagagcaaa 540

gatgtgaatg gacccgaacc actcaacagc cgaagcaaca aactgcactt tgcttttcga 600

ctatatgatt tggataaaga tgaaaagatc tcccgtgatg agctgttaca ggtgctacgc 660

atgatggtcg gagtaaatat ctcagatgag cagctgggca gcatcgcaga caggaccatt 720

caggaggctg atcaggatgg ggacagtgcc atatctttca cagaatttgt taaggttttg 780

gagaaggtgg atgtagaaca gaaaatgagc atccgatttc ttcactaaag gagaccaaac 840

tgttccttgc ggtctagtat ttaagaactg gaacttgaaa gtcctccttc taccaactcc 900

acctccaccc cctcattccc cttctcccaa agtactactg ctgttgcatg acaaccccaa 960

atatgttctg tcaacacaaa cctgcctttg gtgtataaac agggcattac agaatggtac 1020

accctatata tttctgttca gtatccattc actagttctt catttataaa tatcatcttc 1080

cccattctgc tgctgaatgc cacacatcca tccagtctga gaaagtgaga gaggcaatca 1140

tgccaagaac aagccagcaa agctctttca ccagatgtag actgtagccc tgctgccttc 1200

cctccagcga gtctgccagc atgcttcttc atccttttta tatgttcttt gcttcctact 1260

tccctgtctt ccaacatact gttcacttac tctggcagtc tttctgcttt tcattaagcc 1320

tcaaaatctc ctctgttcta cttggcacca caagctatgt cctatatatg tatttctgac 1380

ttggcaggat agttcagggg tctggcagtt tttatttacc ttcattatta aatgggcctc 1440

tgggatgttg cctcttcagg agctttttgg taatcaatac ttctctcaga agtatgagac 1500

catcctctgc actctgctct gtcatcaaag gctgctgggt ggagataccc tttttgaaag 1560

gtggccttgg tgagaggtat ggagccaagt cttctaggtt gcttgcccac atcactctat 1620

ctctggcctc tgattctcaa ctttgtacct gtgtggctcc tcttgttagt gcaatgttga 1680

ctgttgaaaa agcagcagta tgcttacagg tttgcttagt ttggggacac cgttaccacc 1740

agaatggctg ctctgacaat atgcctaggg actttctcat ggcttttatt taataaggag 1800

gctgggcacc ctataaagcc tcatgcattc acacctttgc agcatggttt atgcctcagt 1860

gttatgtgca ctggaatgtt ttccacttca catttccaag tagaaatatt agtgttacgg 1920

aagtgcctaa tatcccagtc caaatttttt tttttttttt tttttttttt tgagacagag 1980

tcttgctctg tcacccaggc tggagtgcag tggtgcgatc gctcactgca acctcagcct 2040

cctggattta agtgattctc ctgcctcagc ctcccaagta gctgggatta caggtgtgca 2100

ccaccatgcc cggctaattt tttgtatttt tagtggagac agggtttcac catgttggcc 2160

aggctggtct cgaactcctg acctcgtgat ccgcctgcct cagcctccca aagtgctggg 2220

attacaggtg tgagccacca cgcctggccc cagtccaaaa tatttaaaga ttgtttcctt 2280

agtgtcttga agttttgcac aaaattcttt tttttgagat ggagtctcac tctgtcaccc 2340

aggctggagt gcagtggcgt gatcttggct cactgcaacc tctgcctcct gggttcaagc 2400

aattctccca cctcagcctc ccaagtagct gggattacag acgtgtgcca ccatacctgg 2460

gtaatttttg catttttagt ggagagggag tttcaccatg ttggccaggt tggtcttgaa 2520

ctcctgacct caggtgatcc tcctgcctcg gcctcccaaa gtgctgggat tacaggcatg 2580

agccaccgtg ctcagccgca aaattcttta tgaattttac acttggcaaa tgttaatgac 2640

ggaagccata gtctgctcct aatacatgtc caaagcattg actgttgtgt cattagctgc 2700

ctggttacat tagctccctg gcttcttgtt tagaccactg ctaatccctt aaaaacaaga 2760

ggtctggcac tagtagcaca acctaaggtg gcattacaga tctttgagcg agccacagca 2820

acttttctgc caagtcagct tagtttagac ttcagtgaat caggctattg ctatcctaat 2880

gtatgtctct atgagtgtat ttagccacac atctgccctt ggttgacttt ctgactcatt 2940

gcttgcttgc ttgtttcctt gctttggaaa actattgaag attgctaaaa aataccactg 3000

caaagtgatg gaaaagggtg gagaacaggg gagtagccag gctggatggc tcaaatataa 3060

atgaatgagg aattctttat gaagtatcag tcagatttta tgattaagtg atgtaatata 3120

ggaattatgt aaaagggaag aatgtctgat actgatctat tagagaggta ctttagaggc 3180

ttcttgattg gcataaagtt cctaaggtta tagattttcc ccccttttgg ctgtatagca 3240

aagtgtttta atccacggtt gtgccttatt gttccattaa aattgtatct tcgatccatc 3300

aataaatact tgtggttgaa acaaaataa 3329

<210> 22
<211> 195
<212> PRT
<213> Artificial Sequence

<220>
<223> CHP1-001 peptide: ENSP00000335632 pep:KNOWN_protein_coding

<400> 22

Met Gly Ser Arg Ala Ser Thr Leu Leu Arg Asp Glu Glu Leu Glu Glu
1               5                   10                  15

Ile Lys Lys Glu Thr Gly Phe Ser His Ser Gln Ile Thr Arg Leu Tyr
            20                  25                  30

Ser Arg Phe Thr Ser Leu Asp Lys Gly Glu Asn Gly Thr Leu Ser Arg
        35                  40                  45

Glu Asp Phe Gln Arg Ile Pro Glu Leu Ala Ile Asn Pro Leu Gly Asp
    50                  55                  60

Arg Ile Ile Asn Ala Phe Phe Pro Glu Gly Glu Asp Gln Val Asn Phe
65                  70                  75                  80

Arg Gly Phe Met Arg Thr Leu Ala His Phe Arg Pro Ile Glu Asp Asn
                85                  90                  95

Glu Lys Ser Lys Asp Val Asn Gly Pro Glu Pro Leu Asn Ser Arg Ser
            100                 105                 110

Asn Lys Leu His Phe Ala Phe Arg Leu Tyr Asp Leu Asp Lys Asp Glu
        115                 120                 125

Lys Ile Ser Arg Asp Glu Leu Leu Gln Val Leu Arg Met Met Val Gly
    130                 135                 140

Val Asn Ile Ser Asp Glu Gln Leu Gly Ser Ile Ala Asp Arg Thr Ile
145                 150                 155                 160

Gln Glu Ala Asp Gln Asp Gly Asp Ser Ala Ile Ser Phe Thr Glu Phe
                165                 170                 175

Val Lys Val Leu Glu Lys Val Asp Val Glu Gln Lys Met Ser Ile Arg
            180                 185                 190

Phe Leu His
        195